# EUROPEAN PATENT SPECIFICATION

(11) **EP 3 027 225 B1**
(45) Date of publication and mention of the grant of the patent: **24.03.2021**
(21) Application number: 14831230.9
(22) Date of filing: 30.07.2014
(51) Int. Cl.: A61K 48/00, C07H 21/02, A61K 38/29, C07K 14/635, C07K 16/26, C12Q 1/68, G01N 33/68, A61K 39/00, G01N 33/74, A61P 3/00

(54) **COMPOSITIONS AND METHODS FOR MODULATING THERMOGENESIS USING TRANSFORMING GROWTH FACTOR ALPHA**
ZUSAMMENSETZUNGEN UND VERFAHREN ZUR MODULIERUNG DER THERMOGENESE MIT TRANSFORMIERENDER WACHSTUMSFAKTOR ALPHA
COMPOSITIONS ET MÉTHODES DE MODULATION DE THERMOGENÈSE À L'AIDE DE FACTEUR TRANSFORMANT DE CROISSANCE ALPHA

(30) Priority: 31.07.2013 US 201361860367 P; 06.11.2013 US 201361900629 P
(43) Date of publication of application: 08.06.2016
(73) Proprietor: Dana-Farber Cancer Institute, Inc., Boston, MA 02115-5450 (US)
(72) Inventor: SPIEGELMAN, Bruce M., Waban, MA 02468 (US); KIR, Serkan, Boston, MA 02118 (US)
(74) Representative: Helbig, Christian
(86) International application number: PCT/US2014/048870
(87) International publication number: WO 2015/017529

(56) References cited:
- WO-A1-2013/090186
- WO-A2-01/55333
- WO-A2-2009/137613
- US-A1- 2004 229 787
- US-A1- 2007 037 228
- US-A1- 2013 074 199
- KATO MIWAKO ET AL: "Effect of transforming growth factor -. alpha . on inositol phospholipid metabolism in human epidermoid carcinoma cells", JOURNAL OF CELLULAR BIOCHEMISTRY, WILEY-LISS INC, US, vol. 37, no. 4, January 1988 (1988-01), pages 339-345, XP009193319, ISSN: 0730-2312
- IBBOTSON K J ET AL: "Stimulation of bone resorption in vitro by synthetic transforming growth factor-alpha.", SCIENCE (NEW YORK, N.Y.) 24 MAY 1985, vol. 228, no. 4702, 24 May 1985 (1985-05-24), pages 1007-1009, XP055340405, ISSN: 0036-8075
- ARAI MARIKO ET AL: "Effects of transforming growth factor . alpha . (TGF-.alpha.) on DNA synthesis and thyrotropin-induced iodine metabolism in cultured porcine thyroid cells", EUROPEAN JOURNAL OF ENDOCRINOLOGY, BIOSCIENTIFICA LTD, GB, vol. 132, no. 2, January 1995 (1995-01), pages 242-248, XP009193318, ISSN: 0804-4643
- ETINDI RANSOME N ET AL: "The effects of TGF-.alpha. and 17.beta.-estradiol on polyphosphoinositide metabolism in MCF-7 breast cancer cells", BREAST CANCER RESEARCH AND TREATMENT, SPRINGER , NY, US, vol. 24, no. 1, January 1992 (1992-01), pages 61-70, XP009193320, ISSN: 0167-6806

## Description

### Cross-Reference to Related Applications

This application claims the benefit of U.S. Provisional Application Nos. 61/900,629, filed on 06 November 2013, and 61/860,367, filed on 31 July 2013.

### Background of the Invention

Metabolic disorders comprise a collection of health disorders or risks that increase the risk of morbidity and loss of qualify of life. For example, diabetes, obesity, including central obesity (disproportionate fat tissue in and around the abdomen), atherogenic dyslipidemia (including a family of blood fat disorders, *e.g.,* high triglycerides, low HDL cholesterol, and high LDL cholesterol that can foster plaque buildups in the vascular system, including artery walls), high blood pressure (130/85 mmHg or higher), insulin resistance or glucose intolerance (the inability to properly use insulin or blood sugar), a chronic prothrombotic state *(e.g.,* characterized by high fibrinogen or plasminogen activator inhibitor-1 levels in the blood), and a chronic proinflammatory state *(e.g.,* characterized by higher than normal levels of high-sensitivity C-reactive protein in the blood), are all metabolic disorders collectively afflicting greater than 50 million people in the United States.

PGC1α (PPARγ coactivator-1 α) is a transcriptional coactivator that mediates many biological programs related to energy metabolism. Originally described as a coactivator of PPARγ that modulated expression of uncoupling protein 1 (UCP1) and thermogenesis in brown fat, it has also been shown to control mitochondrial biogenesis and oxidative metabolism in many cell types. PGC1α is induced in muscle by exercise and stimulates many of the known beneficial effects of exercise in muscle: mitochondrial biogenesis, angiogenesis and fiber-type switching (Handschin and Spiegelman (2008) Nature 454, 463-469). It also provides resistance to muscular dystrophy and denervation-linked muscular atrophy (Sandri et al. (2006) Proc. Natl. Acad. Sci. USA 103, 16260-16265). The healthful benefits of elevated muscle expression of PGC1α go beyond the muscle tissue itself since it has recently been determined that FNDC5 (irisin) is a secreted peptide hormone produced by muscle that can increase thermogenesis and induce brown fat development (Bostrom et al. (2012) Nature 481:463-468 and Moreno-Navarrete et al. (2013) J. Clin. Endocrinol. Metab. 98:E7679-E778).

Despite decades of scientific research, it has only very recently been determined that a secreted factor can be used to effectively treat metabolic disorders and no additional secreted factors besides irisin have been identified. Accordingly, there is a great need to identify molecular regulators of metabolic disorders *(e.g.,* secreted factors), as well as the generation of diagnostic, prognostic, and therapeutic agents to effectively control metabolic disorders in subjects.

### Summary of the Invention

The present invention is based in part on the discovery that cancer cachexia is caused at least by the secretion of PTH-related and EGF-related polypeptides that have the ability to induce significant expression of thermogenic genes and to thereby modulate body weight, blood glucose levels, cellular respiration, and other effects associated with metabolic disorders such as cancer cachexia. The invention is as defined in the claims.

In one aspect, of the disclosure (not necessarily claimed) a method of modulating a metabolic response comprising contacting a cell with an agent that modulates the expression and/or activity of one or more biomarkers listed in Table 1, or orthologs and fragments thereof, to thereby modulate the metabolic response, is provided. In one embodiment of the disclosure, the one or more biomarkers is selected from the group consisting of PTHrP, PTH, BTC, EREG, HBEGF, AREG, EGF, EPGN, and TGFA, and orthologs and fragments thereof. In another embodiment, the one or more biomarkers is selected from the group consisting of PTHrP, PTH, BTC, EREG, and HBEGF, and orthologs and fragments thereof. In still another embodiment of the disclosure, the one or more biomarkers is selected from the group consisting of human PTHrP, human PTH, a human PTHrP fragment comprising or consisting of amino acid residues 1-34, a human PTH fragment comprising or consisting of amino acid residues 1-34, and orthologs thereof. In yet another embodiment of the disclosure, the one or more biomarkers is selected from the group consisting of SEQ ID NOs:1-44 and nucleic acids and polypeptides having a sequence at least 80% identical to one or more of SEQ ID NOs: 1-44, and fragments thereof. In another embodiment of the disclosure, expression and/or activity of the one or more biomarkers is upregulated *(e.g.,* by using an agent selected from the group consisting of a nucleic acid molecule encoding the one or more biomarkers or fragment thereof, a polypeptide of the one or more biomarkers or fragment(s) thereof, and a small molecule that binds to the one or more biomarkers). In still another embodiment of the disclosure, the polypeptide used as an agent is selected from the group consisting of human PTHrP, human PTH, a human PTHrP fragment comprising or consisting of amino acid residues 1-34, a human PTH fragment comprising or consisting of amino acid residues 1-34, and fusion proteins and orthologs thereof. In yet another embodiment of the disclosure, the polypeptide can lack a signal peptide or further comprise a heterologous polypeptide *(e.g.,* a signal peptide, a peptide tag, a dimerization domain, an oligomerization domain, an agent that promotes plasma solubility, an agent that increases *in vivo* protein half-life, an antibody or fragment thereof, and an Fc domain). In another embodiment of the disclosure, expression and/or activity of the one or more biomarkers is downregulated (*e.g*., by using an agent selected from the group consisting of an antisense nucleic acid molecule, an RNA interference molecule, a blocking antibody that binds to the polypeptide of the one or more biomarkers and/or the receptor of such polypeptides, a non-activating form of the polypeptide of the one or more biomarkers or fragments thereof and/or the receptor of such polypeptides, and a small molecule that binds to the one or more biomarkers). In still another embodiment of the disclosure, the antisense, RNA interference, blocking antibodies, non-activating polypeptides, and small molecules bind to one or more biomarkers selected from the group consisting of human PTHrP, human PTH, a human PTHrP fragment comprising or consisting of amino acid residues 1-34, a human PTH fragment comprising or consisting of amino acid residues 1-34, and orthologs thereof. In yet another embodiment, the blocking antibodies and small molecules bind to the PTH1R, EGFR, or ERBB4 receptors.

The methods described herein can further be amended in a number of ways. For example, in one embodiment of the disclsoure, the methods further comprise contacting the cell with an additional agent that increases or decreases the metabolic response. In another embodiment, the step of contacting occurs *in vivo* or *in vitro.* In still another embodiment of the disclosure, the cell is selected from the group consisting of fibroblasts, myoblasts, myocytes, adipoblasts, adipocytes, hepatocytes, and neural cells. In yet another embodiment of the disclosure, the metabolic response is selected from the group consisting of: a) modified expression of one or more markers selected from the group consisting of: cidea, adiponectin, adipsin, otopetrin, type II deiodinase, cig30, ppar gamma 2, pgc1α, ucp1, elovl3, cAMP, Prdm16, cytochrome C, cox4i1, coxIII, cox5b, cox7a1, cox8b, glut4, atpase b2, cox II, atp5o, ndufb5, ap2, ndufs1, GRP109A, acylCoA-thioesterase 4, EARA1, claudin1, PEPCK, fgf21, acylCoA-thioesterase 3, and dio2; b) modified thermogenesis in adipose cells; c) modified differentiation of adipose cells; d) modified insulin sensitivity of adipose cells and/or blood glucose levels; e) modified basal respiration, uncoupled respiration, and/or total respiration; f) modified hepatosteatosis; g) modified obesity or appetite; h) modified insulin secretion of pancreatic beta cells; i) modified cardiac function; j) modified cardiac hypertrophy; and k) modified muscle hypoplasia.

Another aspect of the disclosure is a method of assessing the efficacy of an agent that modulates the expression and/or activity of one or more biomarkers listed in Table 1, or orthologs and fragments thereof, for modulating a metabolic response in a subject is provided, comprising: a) detecting in a subject sample at a first point in time, the expression and/or activity of the expression and/or activity of the one or more biomarkers; b) repeating step a) during at least one subsequent point in time after administration of the agent; and c) comparing the expression and/or activity detected in steps a) and b), wherein a significantly lower biomarker expression and/or activity in the first subject sample relative to at least one subsequent subject sample, indicates that the agent increases the metabolic response in the subject and/or wherein a significantly higher biomarker expression and/or activity in the first subject sample relative to at least one subsequent subject sample, indicates that the test agent decreases the metabolic response in the subject. In one embodiment, the one or more biomarkers is selected from the group consisting of PTHrP, PTH, BTC, EREG, HBEGF, AREG, EGF, EPGN, and TGFA, and orthologs and fragments thereof. In another embodiment of the disclosure, the one or more biomarkers is selected from the group consisting of PTHrP, PTH, BTC, EREG, and HBEGF, and orthologs and fragments thereof. In still another embodiment of the disclosure, the one or more biomarkers is selected from the group consisting of human PTHrP, human PTH, a human PTHrP fragment comprising or consisting of amino acid residues 1-34, a human PTH fragment comprising or consisting of amino acid residues 1-34, and orthologs thereof. In yet another embodiment of the disclosure, the one or more biomarkers is selected from the group consisting of SEQ ID NOs:1-44 and nucleic acids and polypeptides having a sequence at least 80% identical to one or more of SEQ ID NOs: 1-44, and fragments thereof. In another embodiment of the disclsoure, the agent is selected from the group consisting of a nucleic acid molecule encoding the one or more biomarkers or fragment thereof, a polypeptide of the one or more biomarkers or fragment(s) thereof, a small molecule that binds to the one or more biomarkers, an antisense nucleic acid molecule or an RNA interference molecule that binds to the one or more biomarkers, a blocking antibody that binds to the polypeptide of the one or more biomarkers and/or the receptor of such polypeptides, and a non-activating form of the polypeptide of the one or more biomarkers or fragments thereof and/or the receptor of such polypeptides. In still another embodiment of the disclosure, the nucleic acid, polypeptide, or biomarker against which the antisense, RNA interference, blocking antibodies, non-activating polypeptides, and small molecules bind is selected from the group consisting of human PTHrP, human PTH, a human PTHrP fragment comprising or consisting of amino acid residues 1-34, a human PTH fragment comprising or consisting of amino acid residues 1-34, and fusion proteins and orthologs thereof. In yet another embodiment, the polypeptide lacks a signal peptide and/or further comprises a heterologous polypeptide *(e.g.,* a heterologous polypeptide is selected from the group consisting of a signal peptide, a peptide tag, a dimerization domain, an oligomerization domain, an agent that promotes plasma solubility, an agent that increases *in vivo* protein half-life, an antibody or fragment thereof, and an Fc domain). In another embodiment of the disclosure, the blocking antibodies and small molecules bind to the PTH1R, EGFR, or ERBB4 receptors. In still another embodiment of the disclosure, the subject has undergone treatment for the metabolic disorder, has completed treatment for the metabolic disorder, and/or is in remission from the metabolic disorder between the first point in time and the subsequent point in time. In yet another embodiment of the disclosure, the first and/or at least one subsequent sample is selected from the group consisting of *ex vivo* and *in vivo* samples. In another embodiment of the disclosure, the first and/or at least one subsequent sample is obtained from an animal model of a metabolic disorder. In still another embodiment of the disclosure, the first and/or at least one subsequent sample is selected from the group consisting of tissue, whole blood, serum, plasma, buccal scrape, saliva, cerebrospinal fluid, urine, stool, and bone marrow. In yet another embodiment of the disclosure, the first and/or at least one subsequent sample is a portion of a single sample or pooled samples obtained from the subject. In another embodiment of the disclosure, a significantly higher expression and/or activity comprises upregulating the expression and/or activity by at least 25% relative to the second sample. In still another embodiment of the disclosure, a significantly lower expression and/or activity comprises downregulating the expression and/or activity by at least 25% relative to the second sample. In yet another embodiment of the disclosure, the amount of the marker is compared *(e.g.,* by determining the level of protein expression of the marker, such as by using a reagent which specifically binds with the protein like an antibody, an antibody derivative, or an antibody fragment). In another embodiment of the disclosure, the level of expression of the marker in the sample is assessed by detecting the presence in the sample of a transcribed polynucleotide, such as an mRNA or a cDNA, or portion thereof. In still another embodiment of the disclosure, the step of detecting further comprises amplifying the transcribed polynucleotide. In yet another embodiment of the disclosure, the level of expression of the marker in the sample is assessed by detecting the presence in the sample of a transcribed polynucleotide which anneals with the marker or anneals with a portion of a polynucleotide under stringent hybridization conditions. In another embodiment of the disclosure, the metabolic response is selected from the group consisting of: a) modified expression of one or more markers selected from the group consisting of: cidea, adiponectin, adipsin, otopetrin, type II deiodinase, cig30, ppar gamma 2, pgc1α, ucp1, elovl3, cAMP, Prdm16, cytochrome C, cox4i1, coxIII, cox5b, cox7a1, cox8b, glut4, atpase b2, cox II, atp5o, ndufb5, ap2, ndufs1, GRP109A, acylCoA-thioesterase 4, EARA1, claudin1, PEPCK, fgf21, acylCoA-thioesterase 3, and dio2; b) modified thermogenesis in adipose cells; c) modified differentiation of adipose cells; d) modified insulin sensitivity of adipose cells and/or blood glucose levels; e) modified basal respiration, uncoupled respiration, and/or total respiration; f) modified hepatosteatosis; g) modified obesity or appetite; h) modified insulin secretion of pancreatic beta cells; i) modified cardiac function; j) modified cardiac hypertrophy; and k) modified muscle hypoplasia.

In still another aspect of the disclosure, (not claimed) a method for preventing or treating a metabolic disorder in a subject comprising administering to the subject an agent that promotes expression and/or activity of one or more biomarkers listed in Table 1, or orthologs and fragments thereof, in the subject, thereby preventing or treating the metabolic disorder in the subject, is provided. In one embodiment of the disclosure, the one or more biomarkers is selected from the group consisting of PTHrP, PTH, BTC, EREG, HBEGF, AREG, EGF, EPGN, and TGFA, and orthologs and fragments thereof. In another embodiment of the disclosure, the one or more biomarkers is selected from the group consisting of PTHrP, PTH, BTC, EREG, and HBEGF, and orthologs and fragments thereof. In still another embodiment of the disclosure, the one or more biomarkers is selected from the group consisting of human PTHrP, human PTH, a human PTHrP fragment comprising or consisting of amino acid residues 1-34, a human PTH fragment comprising or consisting of amino acid residues 1-34, and orthologs thereof. In yet another embodiment of the disclosure, the one or more biomarkers is selected from the group consisting of SEQ ID NOs:1-44 and nucleic acids and polypeptides having a sequence at least 80% identical to one or more of SEQ ID NOs: 1-44, and fragments thereof. In one embodiment of the disclosure, expression and/or activity of the one or more biomarkers is upregulated using an agent selected from the group consisting of a nucleic acid molecule encoding the one or more biomarkers or fragment thereof, a polypeptide of the one or more biomarkers or fragment(s) thereof, and a small molecule that binds to the one or more biomarkers. In another embodiment, the polypeptide is selected from the group consisting of human PTHrP, human PTH, a human PTHrP fragment comprising or consisting of amino acid residues 1-34, a human PTH fragment comprising or consisting of amino acid residues 1-34, and fusion proteins and orthologs thereof. In still another embodiment of the disclosure, the polypeptide lacks a signal peptide and/or further comprises a heterologous polypeptide *(e.g.,* a the heterologous polypeptide is selected from the group consisting of a signal peptide, a peptide tag, a dimerization domain, an oligomerization domain, an agent that promotes plasma solubility, an agent that increases *in vivo* protein half-life, an antibody or fragment thereof, and an Fc domain). In yet another embodiment of the disclosure, the method further comprises contacting the cell with an additional agent that treats the metabolic disorder. In another embodiment of the disclosure, the agent is administered by intravenous or subcutaneous injection. In still another embodiment, the agent is administered in a pharmaceutically acceptable formulation. In yet another embodiment, the metabolic disorder is selected from the group consisting of obesity, insulin resistance, hyperinsulinemia, hypoinsulinemia, type II diabetes, hyperglycemia, hypertension, hyperhepatosteatosis, hyperuricemia, fatty liver, non-alcoholic fatty liver disease, polycystic ovarian syndrome, acanthosis nigricans, hyperphagia, endocrine abnormalities, triglyceride storage disease, Bardet-Biedl syndrome, Lawrence-Moon syndrome, Prader-Labhart-Willi syndrome, muscle hypoplasia, neurodegenerative diseases, and Alzheimer's disease. In another embodiment of the disclosure, the agent is administered less frequently than once per day. In still another embodiment of the disclosure, the subject is a non-human animal or a human.

In yet another embodiment of the disclosure, (not claimed) a method for preventing or treating a metabolic disorder in a subject comprising administering to the subject an agent that inhibits expression and/or activity of one or more biomarkers listed in Table 1, or orthologs and fragments thereof, in the subject, thereby preventing or treating the metabolic disorder in the subject, is provided. In one embodiment of the disclosure, the one or more biomarkers is selected from the group consisting of PTHrP, PTH, BTC, EREG, HBEGF, AREG, EGF, EPGN, and TGFA, and orthologs and fragments thereof. In another embodiment, the one or more biomarkers is selected from the group consisting of PTHrP, PTH, BTC, EREG, and HBEGF, and orthologs and fragments thereof. In still another embodiment, the one or more biomarkers is selected from the group consisting of human PTHrP, human PTH, a human PTHrP fragment comprising or consisting of amino acid residues 1-34, a human PTH fragment comprising or consisting of amino acid residues 1-34, and orthologs thereof. In yet another embodiment of the disclosure, the one or more biomarkers is selected from the group consisting of SEQ ID NOs: 1-44 and nucleic acids and polypeptides having a sequence at least 80% identical to one or more of SEQ ID NOs: 1-44, and fragments thereof. In one embodiment of the disclosure, expression and/or activity of the one or more biomarkers is downregulated using an agent selected from the group consisting of an antisense nucleic acid molecule, an RNA interference molecule, a blocking antibody that binds to the polypeptide of the one or more biomarkers and/or the receptor of such polypeptides, a non-activating form of the polypeptide of the one or more biomarkers or fragments thereof and/or the receptor of such polypeptides, and a small molecule that binds to the one or more biomarkers. In another embodiment of the disclosure, the antisense, RNA interference, blocking antibodies, non-activating polypeptides, and small molecules bind to one or more biomarkers selected from the group consisting of human PTHrP, human PTH, a human PTHrP fragment comprising or consisting of amino acid residues 1-34, a human PTH fragment comprising or consisting of amino acid residues 1-34, and orthologs thereof. In still another embodiment, the blocking antibodies and small molecules bind to the PTH1R, EGFR, or ERBB4 receptors. In yet another embodiment of the disclosure, the method further comprises contacting the cell with an additional agent that decreases the metabolic response. In another embodiment of the disclosure, the agent is administered by intravenous or subcutaneous injection. In still another embodiment of the disclosure, the agent is administered in a pharmaceutically acceptable formulation. In yet another embodiment of the disclosure, the metabolic disorder is selected from the group consisting of cachexia, hypoglycemia, obesity-associated cancer, and anorexia. In another embodiment of the disclosure, agent is administered less frequently than once per day. In still another embodiment, the subject is a non-human animal or a human.

In another aspect of the disclosure, (not claimed) a method for preventing or treating a metabolic disorder in a subject comprising administering to the subject an agent that increases expression and/or activity of one or more of PTHrP, PTH, BTC, EREG, HBEGF, AREG, EGF, EPGN, and TGFA, and orthologs and fragments thereof, in the subject, thereby preventing or treating the metabolic disorder in the subject, is provided. In one embodiment of the disclosure, the one or more biomarkers is selected from the group consisting of human PTHrP, human PTH, a human PTHrP fragment comprising or consisting of amino acid residues 1-34, a human PTH fragment comprising or consisting of amino acid residues 1-34, and orthologs thereof. In another embodiment of the disclosure, the one or more biomarkers is selected from the group consisting of SEQ ID NOs:1-44 and nucleic acids and polypeptides having a sequence at least 80% identical to one or more of SEQ ID NOs: 1-44, and fragments thereof. In still another embodiment of the disclosure, the expression and/or activity of the one or more biomarkers is upregulated using an agent selected from the group consisting of a nucleic acid molecule encoding the one or more biomarkers or fragment thereof, a polypeptide of the one or more biomarkers or fragment(s) thereof, and a small molecule that binds to the one or more biomarkers. In yet another embodiment of the disclosure, the polypeptide is selected from the group consisting of human PTHrP, human PTH, a human PTHrP fragment comprising or consisting of amino acid residues 1-34, a human PTH fragment comprising or consisting of amino acid residues 1-34, and fusion proteins and orthologs thereof. In another embodiment of the disclosure, the polypeptide lacks a signal peptide and/or further comprises a heterologous polypeptide *(e.g.,* a heterologous polypeptide is selected from the group consisting of a signal peptide, a peptide tag, a dimerization domain, an oligomerization domain, an agent that promotes plasma solubility, an agent that increases *in vivo* protein half-life, an antibody or fragment thereof, and an Fc domain). In still another embodiment of the disclosure, the metabolic disorder is selected from the group consisting of obesity, insulin resistance, hyperinsulinemia, hypoinsulinemia, diabetes *(e.g.,* type II diabetes), and hyperglycemia. In yet another embodiment of the disclosure, the method further comprises contacting the cell with an additional agent that increases the metabolic response. In another embodiment of the disclosure, the agent is administered by intravenous or subcutaneous injection. In still another embodiment of the disclosure, the agent is administered in a pharmaceutically acceptable formulation. In yet another embodiment, the agent is administered less frequently than once per day. In another embodiment of the disclosure, the subject is a non-human animal or a human.

### Brief Description of Figures

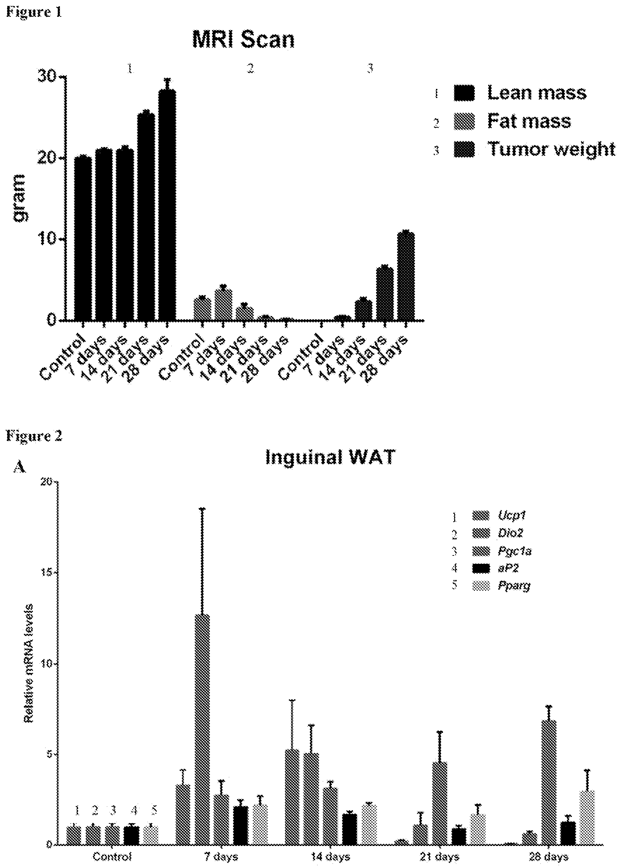

**Figure 1** shows the results of 7-9 week-old lean mice inoculated with 5 million Lewis Lung Carcinoma (LLC) cells. Tumors were allowed to grow for 1-4 weeks and tumor growth led to dramatic fat loss (cancer cachexia). For each time point, bars corresponding to weight values are presented in groupings from left to right as lean mass, fat mass, and tumor weight, according to the representative numbering.

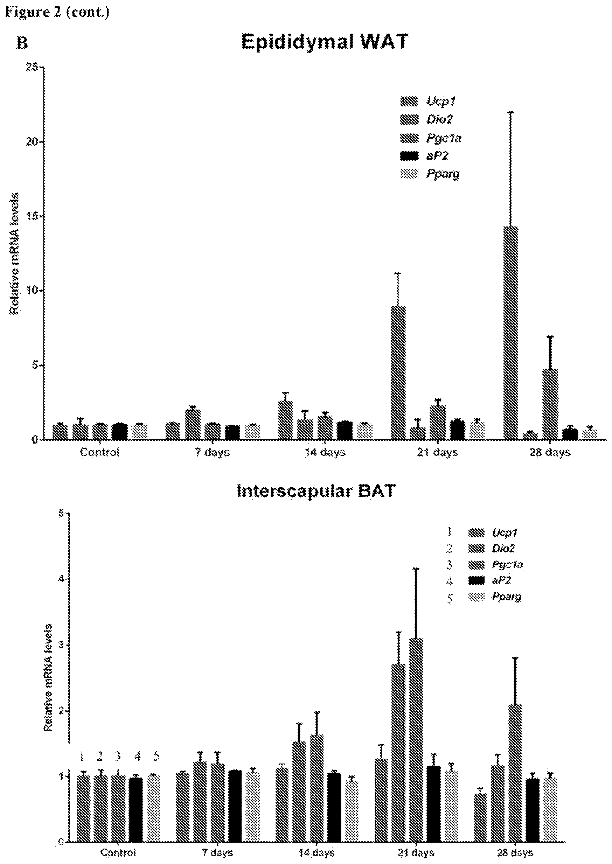

**Figures 2A-2C** show the results of thermogenic gene expression analyses in various adipose tissues of the LLC-inoculated mice described in Figure 1. Thermogenic gene expression induction in inguinal white adipose tissue (WAT) (Figure 2A), epididymal WAT (Figure 2B), and interscapular brown adipose tissue (BAT) (Figure 2C). For each time point, bars corresponding to expression of the analyzed genes are presented in order from left to right as Ucp1, Dio2, Pgc1a, aP2, and Pparg, according to the representative numbering.

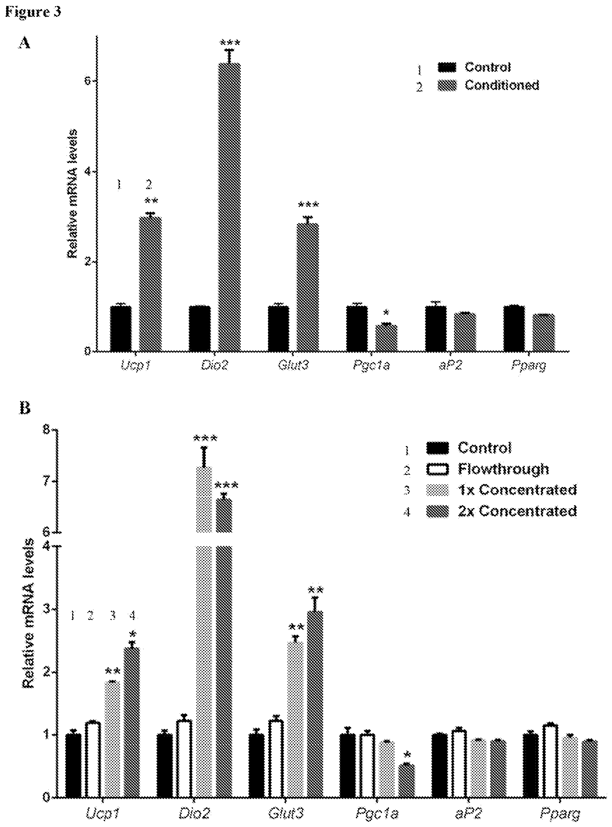

**Figures 3A-3B** show the results of primary white inguinal adipocytes treated with conditioned media of LLC cells for 24 hours. LLC cell products induced thermogenic genes, such as Ucp1 and Dio2 (Figure 3A). Figure 3B shows the results of a the same experiment shown in Figure 3A except that the LLC conditioned media was filtered using a 3 kilodalton (Kd) cut-off filter in which the flowthrough contained small molecule metabolites and the concentrated portion contained macromolecules larger than 3 Kd. Use of the filtered LLC conditioned media concentrate for a 24 hour treatment of primary adipocytes resulted in thermogenic gene induction indicating that the thermogenic induction activity from the LLC conditioned media is a protein that is bigger than 3 Kd. For each gene, the control is shown on the left and the conditioned media is shown on the right, according to the representative numbering. Values are means ± SEM. Statistics by two-tailed t test. **P* < 0.05, ***P* < 0.005, ****P* < 0.0005. LLC cells were maintained in DMEM with 10% FBS and antibiotics. For conditioned media collection, cells were plated at 1/3 confluency. The following day, medium was changed to FreeStyle expression medium (Invitrogen) which was collected 24 hours later. Adipocytes were maintained in DMEM/F12 with 10%FBS and antibiotics and conditioned media treatment experiments were performed 7 days post-differentiation. When adipocytes were treated with conditioned media for 24 hours, the treatment media were composed of 50% adipocyte culturing medium and 50% LLC condioned media. When conditioned medium was filtered and concentrated, adipocytes were exposed to 90% adipocyte culturing medium and 10% concentrated LLC conditioned media.

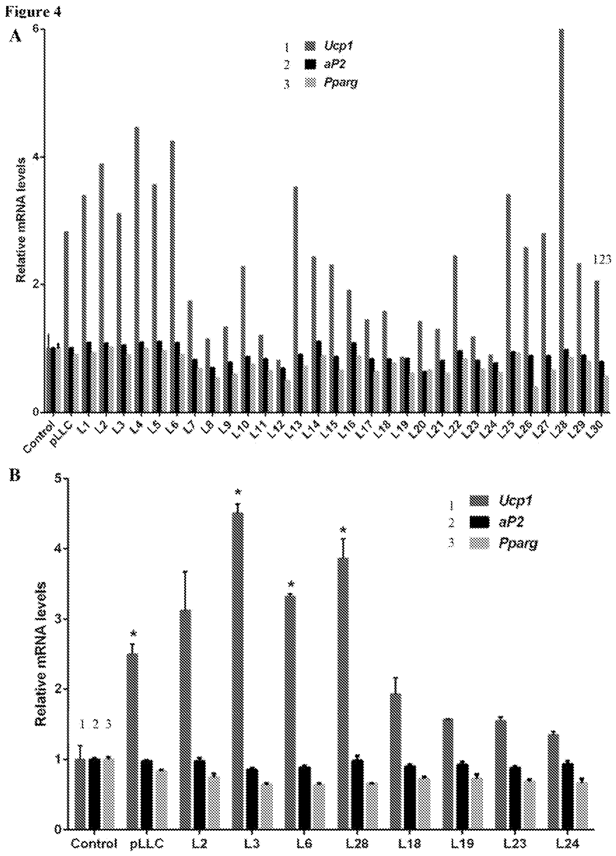

**Figure 4A-4B** show the results of primary white inguinal adipocytes treated with clonal LLC cells for 24 hours. Parental LLC cells were single cell cloned into 30 colonies and clones were determined to have varying degrees of thermogenic effects on primary adipocytes (Figure 4A). Reproducibly more thermogenic clones were clones L2, L3, L6, L28 and less thermogenic clones were clones L18, L19, L23 and L24. Clones L2, L3, L6, L28, L18, L19, L23, and L24 were chosen for further analysis to confirm their thermogenic effects on primary adipocytes (Figure 4B). For each gene, the control is shown on the left and the conditioned media is shown on the right, according to the representative numbering. Values are means ± SEM. Statistics by two-tailed t test. **P* < 0.05. Culturing conditions are the same as described for Figure 3.

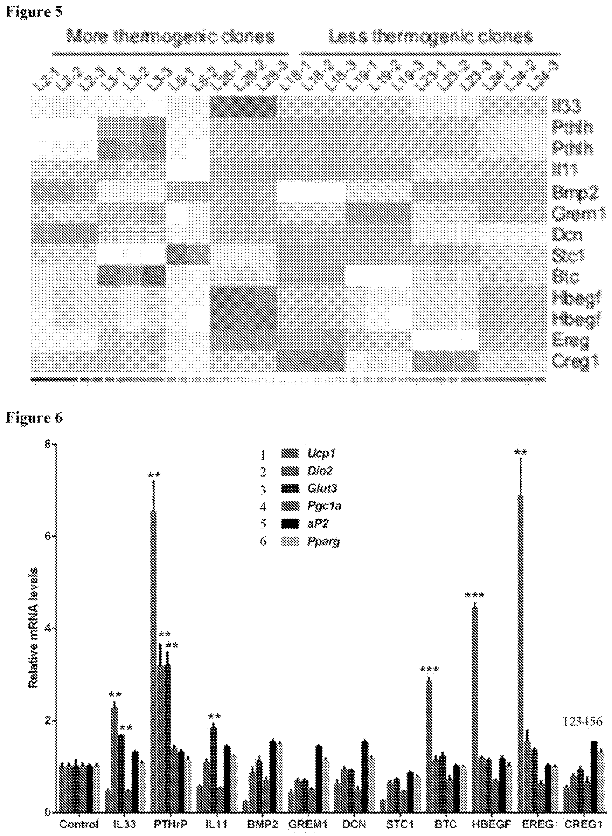

**Figure 5** shows the results of microarray analyses performed in triplicate (except for L6 which was in duplicate) using RNA obtained for each of clones L2, L3, L6, L28, L18, L19, L23, and L24. RNA samples from these cells were submitted for Affymetrix microarray analysis. Those genes that have higher expression in more thermogenic clones and are predicted to be secreted factors were determined and grouped as shown. RNA was prepared using TRIzol (Invitrogen) and Qiagen RNAeasy kit. Affymetrix Mouse Genome 430 2.0 Array was used to analyze gene expression.
**Figure 6** shows the thermogenic activity of secreted proteins (1 µg/mL) identified in the Affymetrix microarray analyses described in Figure 5 and purchased from vendors on primary white adipocytes treated for 24 hours. PTHrP (1-34) peptide along with EGF proteins, BTC, HB-EGF and EREG, are the most potent proteins to induce thermogenic gene expression, such as that of Ucp1 and Dio2. For each secreted protein tested, bars corresponding to expression of the analyzed genes are presented in order from left to right as Ucp1, Dio2, Glut3, Pgc1a, aP2, and Pparg, according to the representative numbering. Recombinant mouse IL33 (Ser109-Ile266; Catalog No. 3626-ML-010/CF), recombinant mouse IL11 (Gly23-Leu199; Catalog No. 418-ML-005/CF), recombinant human BMP2 (Gln283-Arg396; Catalog No. 355-BM-010/CF), recombinant mouse GREM1 (Lys25-Asp184; Catalog No. 956-GR-050/CF), recombinant mouse DCN (Gly17-Lys354; Catalog No. 1060-DE-100), recombinant mouse BTC (Asp32-Gln118; Catalog No. 1025-CE-025/CF), recombinant mouse EREG (Val56-Leu101; Catalog No. 1068-EP/CF), recombinant human HBEGF (Met1-Leu148; Catalog No. 259-HE-050/CF), and recombinant mouse CREG1 (Arg32-Gln220; 1697-CR/CF) were purchased from R&D Systems. Recombinant human STC1 (Thr18-Ala247; Catalog No. RD172095020) was purchased from BioVendor. Synthetic peptide PTHrP (Ala37-Ala70; Catalog No. H-6630) was purchased from Bachem. Values are means ± SEM. Statistics by two-tailed t test. ***P* < 0.005, ****P* < 0.0005. Adipocytes were cultured in DMEM/F12 with 10% FBS and antibiotics. Seven days post-differentiation, adipocytes were treated with 1 µg/mL of the indicated proteins for 24 hours.

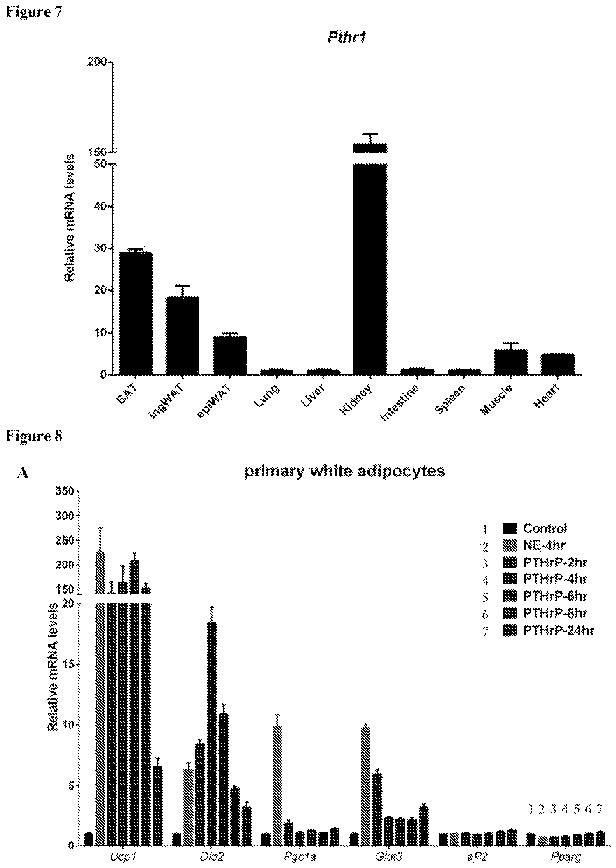

**Figure 7** shows that PTHrP/PTH receptor (PTHR1) is highly expressed in adipose tissues.

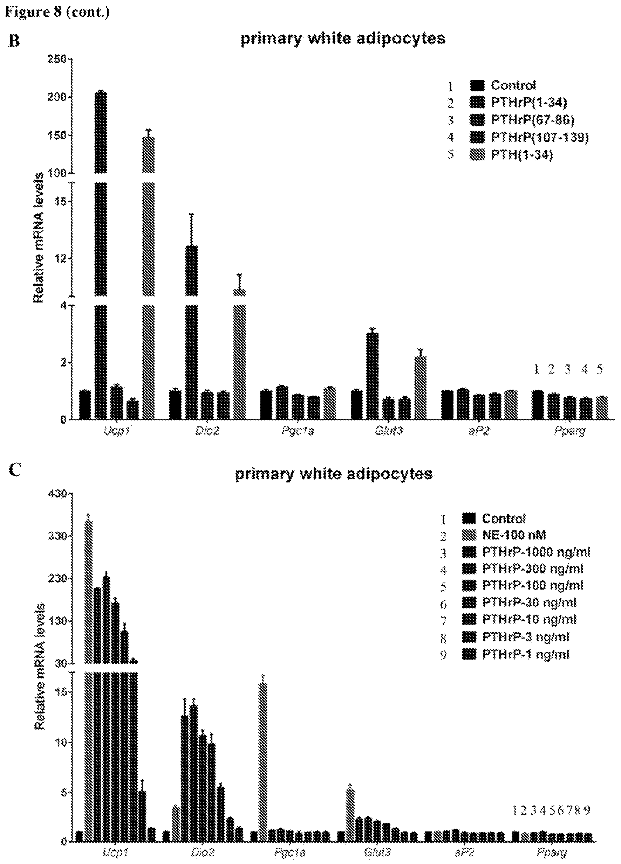

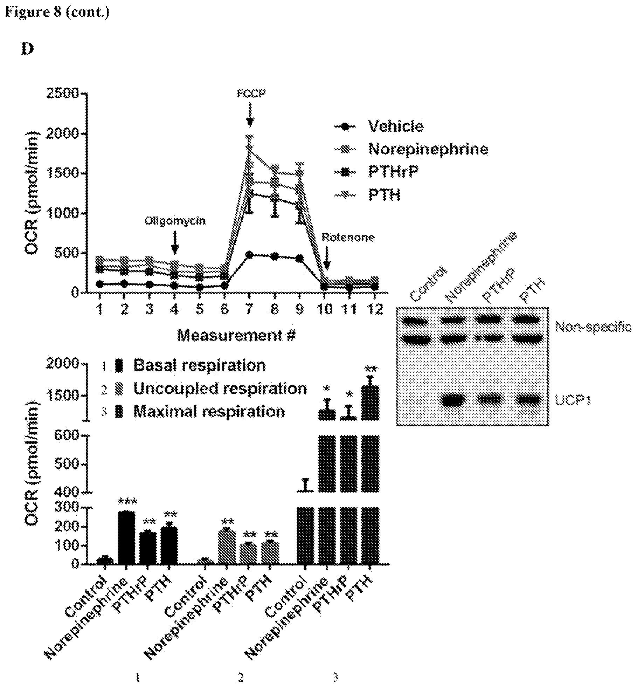

**Figures 8A-8D** show that PTHrP/PTH receptor (PTHR1) and PTH can induce thermogenic gene expression and increase cellular respiration in white adipocytes. Figure 8A shows the results of primary white adipocytes treated with 1 µg/ml PTHrP (1-34) for varying durations. PTHrP is almost as potent as Norepinephrine (NE) (100 nM) in inducing thermogenic gene expression, such as Ucp1 and Dio2. Figure 8B shows the results of primary white adipocytes treated for 4 hours with three different PTHrP peptides and PTH (1-34) (1 µg/ml), in which PTHrP (1-34) and PTH (1-34) were determined to have thermogenic effects. Figure 8C shows the results of primary white adipocytes treated for 4 hours with varying concentrations of PTHrP (1-34) or norepinephrine (100 nM). Figure 8D shows that 24 hour treatment of adipocytes with norepinephrine (100 nM), PTHrP (1-34) (100 ng/mL), and PTH (1-34) (100 ng /mL) can significantly elevate both uncoupled respiration and maximal respiration potential, as measured by SeaHorse™ assays. UCP1 protein levels were significantly increased by these treatments as shown by western blotting. For each thermogenic gene analyzed, bars corresponding to the agent applied to the primary white adipocytes are presented in order from left to right, according to the representative numbering. Synthetic peptides; human/mouse/rat PTHrP(1-34) (Ala37-Ala70; Catalog No. H-6630), human/mouse/rat PTHrP(67-86) (Tyr103-Pro122; Catalog No. H-5722), human PTHrP(107-139) (Osteostatin: Thr143-Arg175; Catalog No. H-3212) and rat PTH(1-34) (Ala32-Phe65; Catalog No. H-5460) were purchased from Bachem. Values are means ± SEM. Statistics by two-tailed t test. **P* < 0.05, ***P* < 0.005, ****P* < 0.005. Adipocytes were cultured in DMEM/F12 with 10% FBS and antibiotics and treated for 4 hours when they were 8 days post-differentiation.

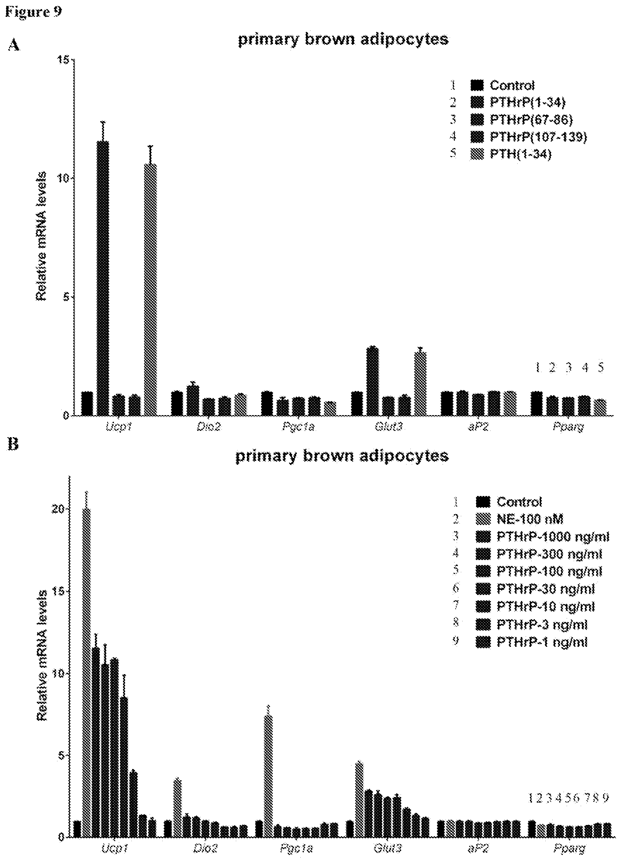

**Figures 9A-9B** show that PTHrP/PTH receptor (PTHR1) and PTH can induce thermogenic gene expression and increase cellular respiration in brown adipocytes. Figure 9A shows the results of primary brown adipocytes treated with three different PTHrP peptides and PTH (1-34), in which PTHrP (1-34) and PTH (1-34) (1 µg/ml) were determined to have thermogenic effects. Figure 9B shows the results of primary brown adipocytes treated with varying concentrations of PTHrP (1-34) or norepinephrine (100 nM). For each thermogenic gene analyzed, bars corresponding to the agent applied to the primary brown adipocytes are presented in order from left to right, according to the representative numbering. Adipocytes were cultured in DMEM/F12 with 10% FBS and antibiotics and treated for 4 hours when they were 8 days post-differentiation. The peptides used were the same as described for Figure 8.

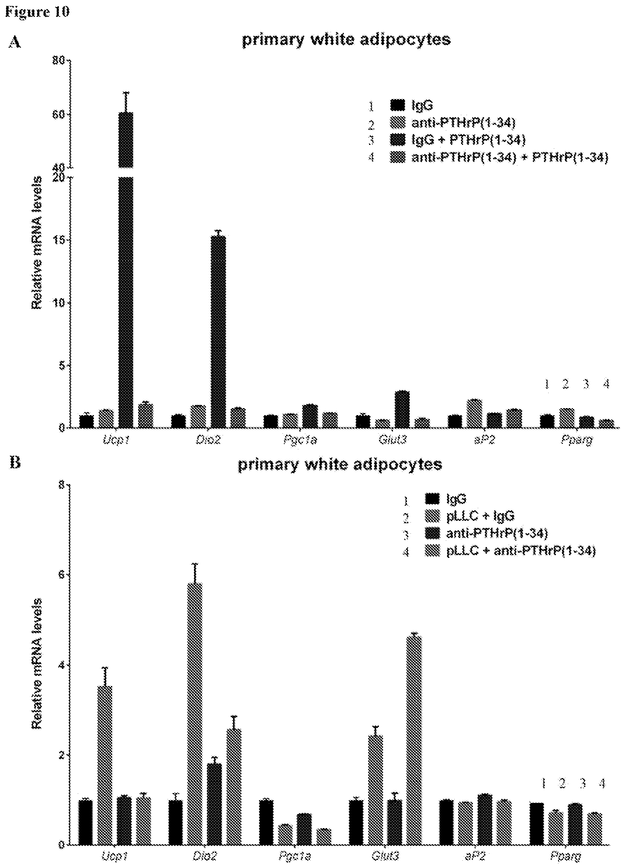

**Figures 10A-10B** show that neutralization of PTHrP blocks its thermogenic activity in primary white adipocytes. Figure 10A shows that a neutralizing antibody against PTHrP (1-34) blocks the peptide's thermogenic activity in primary white adipocytes. Adipocytes were cultured in DMEM/F12 with 10% FBS and antibiotics and treated for 4 hours when they were 8 days post-differentiation. Figure 10B shows that the anti-PTHrP (1-34) antibody block's parental LLC (pLLC) cell conditioned media-induced thermogenesis in primary white adipocytes, such as blocking upregulation of Ucp1 and Dio2 expression. LLC-media caused changes on Pgc1a and Glut3 were undisturbed, indicating that the antibody does not have general effect on the cells. For each thermogenic gene analyzed, bars corresponding to the agent applied to the primary white adipocytes are presented in order from left to right, according to the representative numbering. Adipocytes were cultured in DMEM/F12 with 10% FBS and antibiotics and treated for 24 hours when they were 7 days post-differentiation. Normal rabbit IgG (Santa Cruz Biotech; Catalog No. sc-2027) and anti-PTHrP (Bachem; Catalog No. T-4512) were used at 10 µg/ml concentration whereas PTHrP (1-34) concentration was 10 ng/ml.

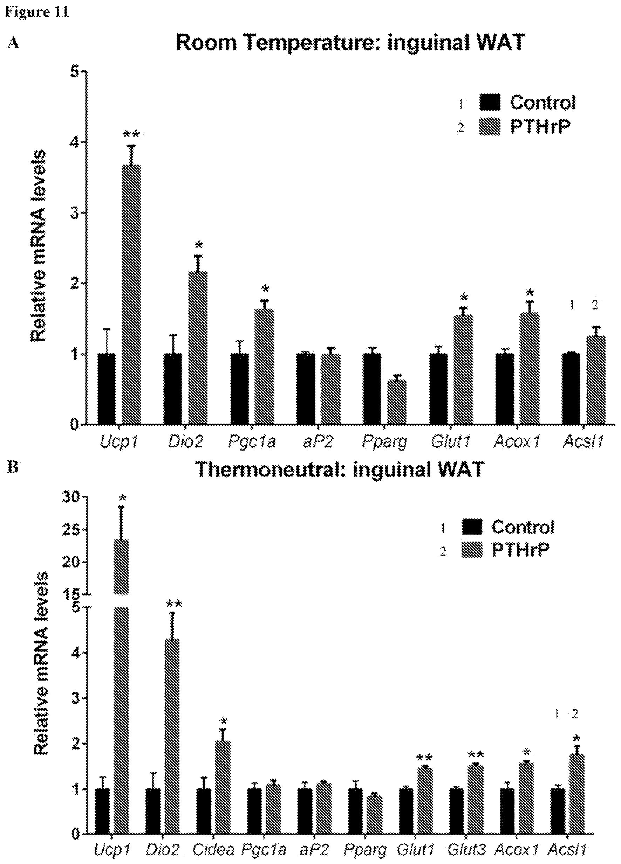

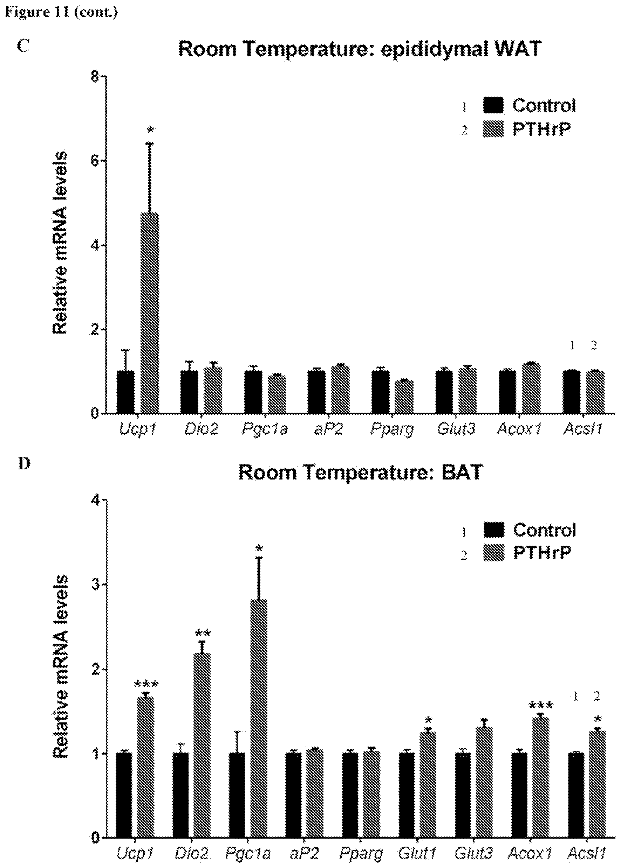

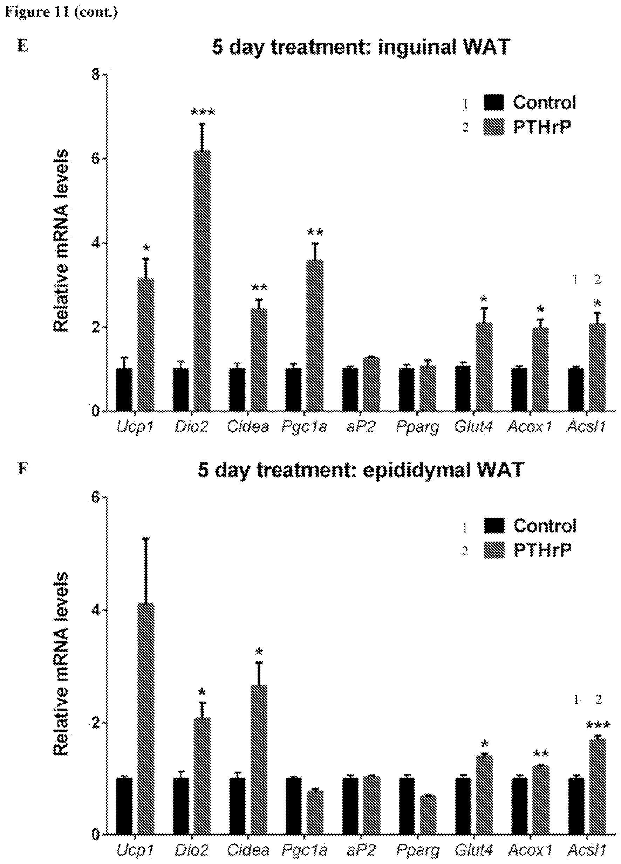

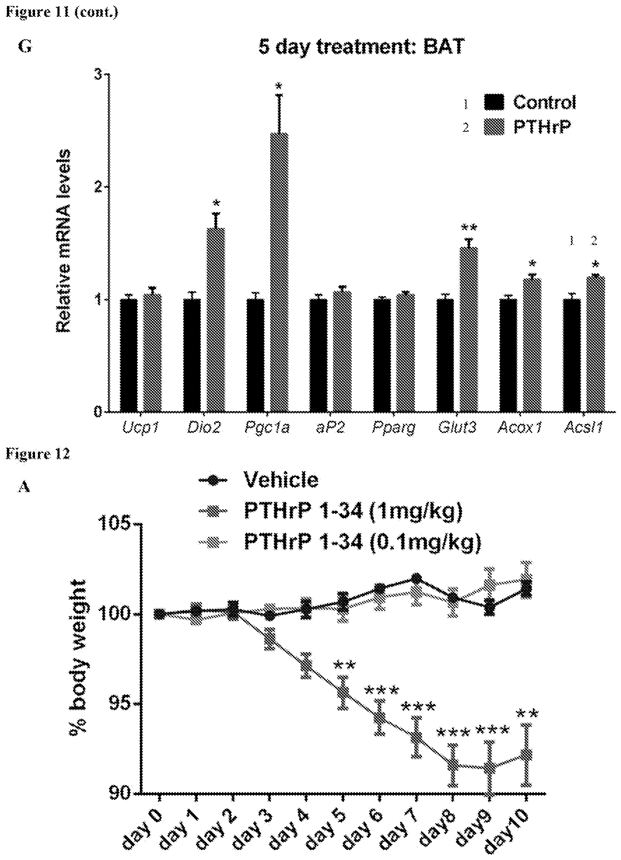

**Figures 11A-11G** show that PTHrP/PTH induce thermogenic gene expression and weight loss *in vivo.* Figure 11A shows the results of thermogenic gene expression in the inguinal fat pads of 7-9 week-old lean mice housed at room temperature and sacrificed two hours after having been injected once with 1 mg/kg PTHrP (1-34). Figure 11B shows the results of thermogenic gene expression in the inguinal fat pads of 7-9 week-old lean mice housed at thermoneutrality (30°C) and sacrificed four hours after having been injected once with 1 mg/kg PTHrP (1-34). Figure 11C shows the results of thermogenic gene expression in the epididymal fat pads of 7-9 week-old lean mice housed at room temperature and sacrificed two hours after having been injected once with 1 mg/kg PTHrP (1-34). Figure 11D shows the results of thermogenic gene expression in the brown fat pads of 7-9 week-old lean mice housed at room temperature and sacrificed two hours after having been injected once with 1 mg/kg PTHrP (1-34). Figure 11E-11G show thermogenic gene expression in inguinal white adipose tissue (Figure 11E), epididymal white adipose tissue (Figure 11F), and brown adipose tissue (Figure 11G) in 7-9 week-old lean mice injected daily with 1 mg/kg PTHrP (1-34) over a course of five days and sacrificed two hours after the last injection. For each thermogenic gene analyzed, bars corresponding to the agent applied to the primary white adipocytes are presented in order from left to right, according to the representative numbering. Values are means ± SEM. Statistics by two-tailed t test. **P* < 0.05, ***P* < 0.005, ****P* < 0.0005. All injections were subcutaneous. RNA was extracted from frozen tissues using TRIzol (Invitrogen) and Qiagen RNAeasy kit. mRNA levels were determined by RT-qPCR.

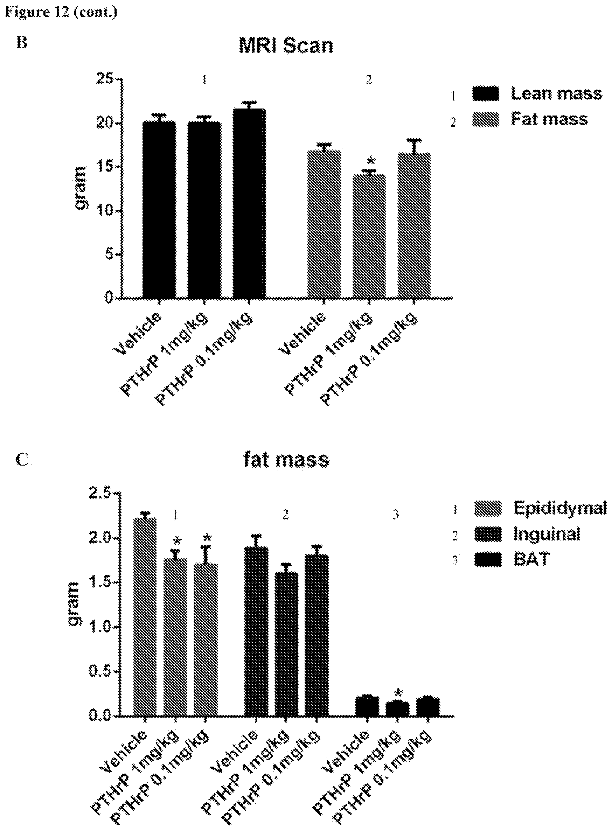

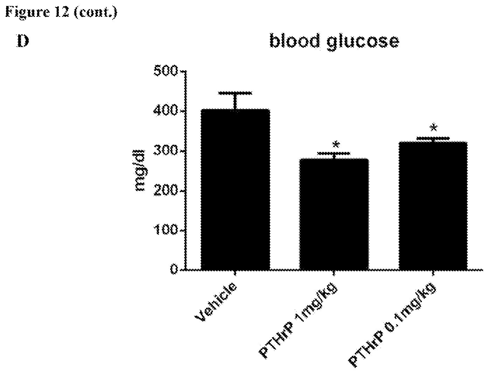

**Figure 12A-12D** show that diet-induced obese mice injected with 1 mg/kg PTHrP (1-34) daily for 10 days displayed amelioration of a number of indicators of metabolic disorders. Figure 12A shows that weight loss was observed by day 3 and the treated obese mice lost up to 10% body weight in 10 days. Figure 12B shows that the treated obese mice had about a 20% reduction in total fat mass, as determined by MRI scanning. Figure 12C shows that fat mass was reduced in three harvested and weighed fat pads from the treated obese mice. Figure 12D shows that the treated obese mice had lowered blood glucose levels and improved glucose metabolism. For each agent analyzed, bars corresponding to weight values are presented in groups order from left to right, according to the representative numbering. Values are means ± SEM. Statistics by two-tailed t test. **P* < 0.05, ***P* < 0.005, ****P* < 0.0005. All injections were subcutaneous. Echo MRI was used to measure lean and fat masses the day before mice were sacrificed. For actual fat pad mass measurements, fat pads were harvested and weighed. Blood glucose was determined using Autokit Glucose (Wako).

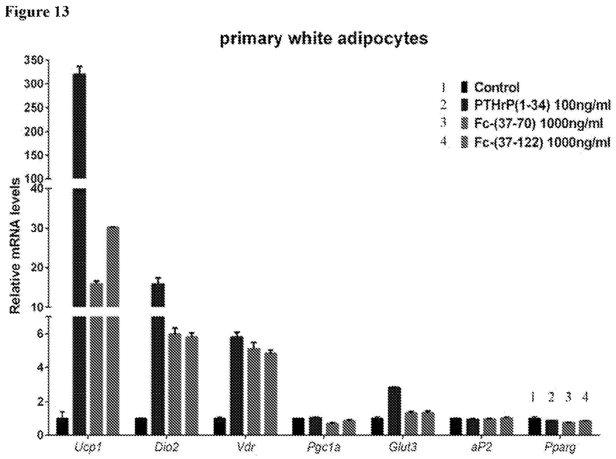

**Figure 13** shows PTHrP-Fc fusion proteins useful for increasing pharmacological half-life and/or bioavailability. The term "37-70" corresponds to amino acids residues 1-34 when the signal peptide is omitted. Amino acid residues 37-122 of PTHrP were fused to Fc protein to increase pharmacologic half-life in addition to maintaining the ability to increase thermogenic gene expression, such as that of Ucp1 and Dio2 expression. For each thermogenic gene analyzed, bars corresponding to the agent applied to the primary white adipocytes are presented in order from left to right, according to the representative numbering. Adipocytes were cultured in DMEM/F12 with 10% FBS and antibiotics and treated for 4 hours when they were 8 days post-differentiation.

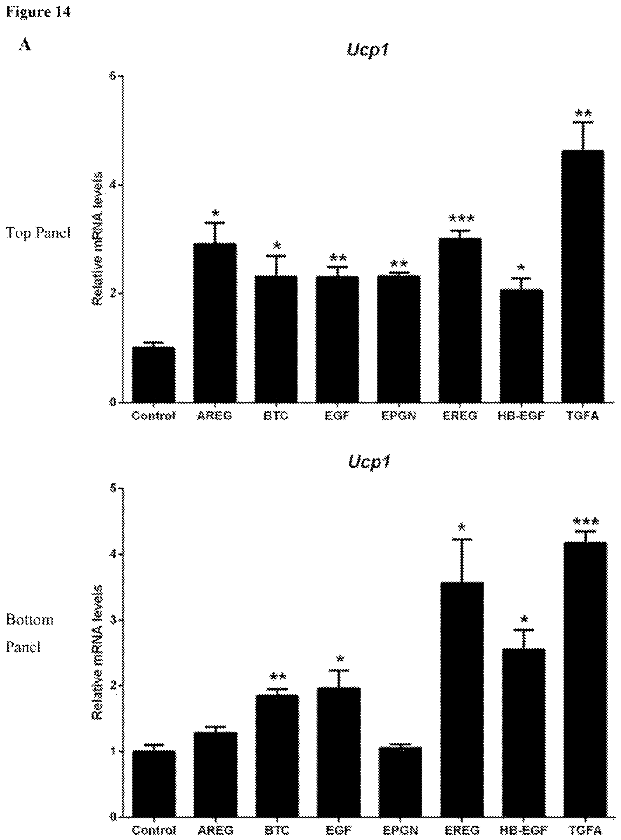

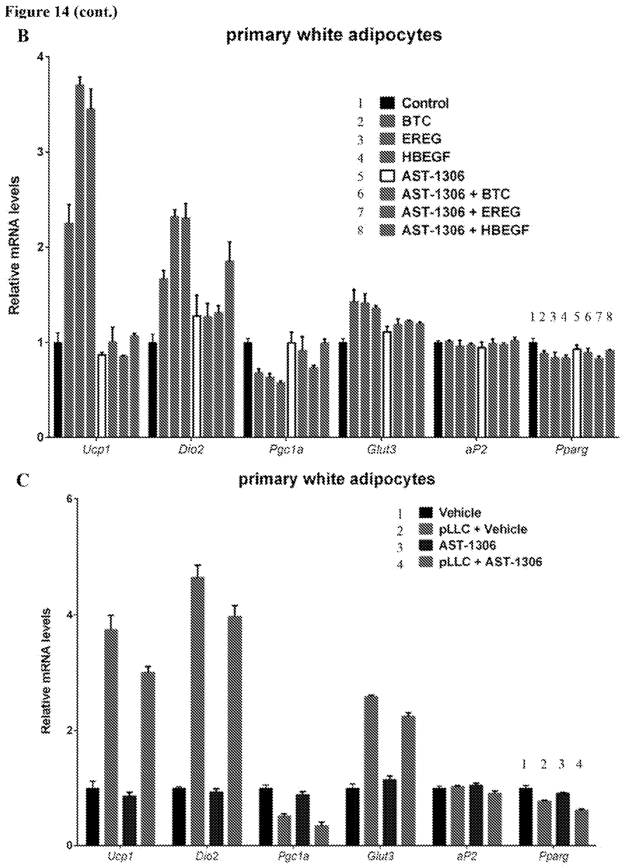

**Figures 14A-14C** show that various EGF proteins are powerful inducers of thermogenic gene expression, which effect can be blocked by inhibiting the EGF receptor. Figure 14A shows the results of treating primary white adipocytes with various EGF proteins at 1 µg /mL (top panel) or 100 ng/mL (bottom panel). EREG, HB-EGF, and TGFA were observed to have the largest effect on Ucp1 expression. Figure 14B shows the ability of EGF receptor inhibitor AST-1306 (1µM) to inhibit BTC, EREG and HBEGF (100ng/ml each) activity on primary adipocytes. Figure 14C shows that AST-1306 does not inhibit or affect parental LLC cell conditioned media activity on primary white adipocytes. For each thermogenic gene analyzed, bars corresponding to the agent applied to the primary white adipocytes are presented in order from left to right, according to the representative numbering. Values are means ± SEM. Statistics by two-tailed t test. **P* < 0.05, ***P* < 0.005, ****P* < 0.0005. Recombinant mouse AREG (Ser94-Lys191; Catalog No. 989-AR-100/CF), recombinant mouse BTC (Asp32-Gln118; Catalog No. 1025-CE-025/CF), recombinant mouse EGF (Asn977-Arg1029; Catalog No. 2028-EG-200), recombinant mouse EPGN (Leu53-Ala103; Catalog No. 1127-EP/CF), recombinant mouse EREG (Val56-Leu101; Catalog No. 1068-EP/CF), recombinant human HBEGF (Met1-Leu148; Catalog No. 259-HE-050/CF) and recombinant human TGFA (Val40-Ala89; Catalog No. 239-A-100) were purchased from R&D Systems. Adipocytes were cultured in DMEM/F12 with 10% FBS and antibiotics and treated for 24 hours when they were 7 days post-differentiation. AST-1306 was used at 1µM final concentration.

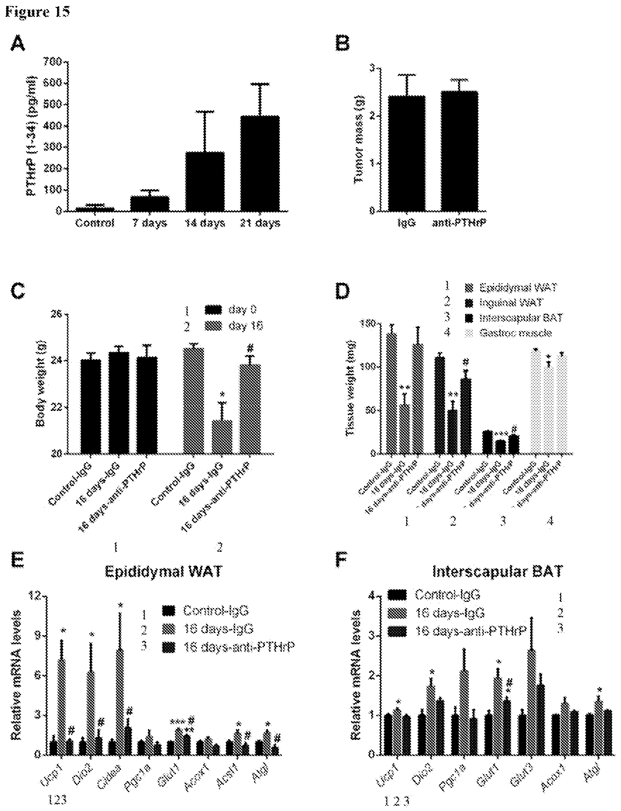

**Figures 15A-15F** show neutralization of PTHrP prevents tumor-induced adipose tissue browning and wasting of both fat and skeletal muscle tissues. Figure 15A shows results of plasma PTHrP that was measured in mice inoculated with LLC tumors for 1-3 weeks. Figures 15B-15F show the results of mice injected with LLC cells and allowed to grow tumors for 6 days. They received intraperitoneal injections of IgG or anti-PTHrP (10 mg/kg body weight) every 3 days from day 6 to day 15 and were sacrificed one day after the last injection at day 15. Body weight was measured by subtracting the tumor weight from the total weight (Figure 15C). Fat and muscle tissues (Figure 15D) and tumors (Figure 15B) were dissected and weighed. mRNA levels in epididymal WAT (Figure 15E) and interscapular BAT (Figure 15F) were measured by RT-qPCR. Values are means ± SEM. Statistics by two-tailed t test. (*) refers to differences compared to the control group. (#) refers to differences between 16 days groups. **P* < 0.05, ***P* < 0.005, ****P* < 0.0005, *#P* < 0.05.

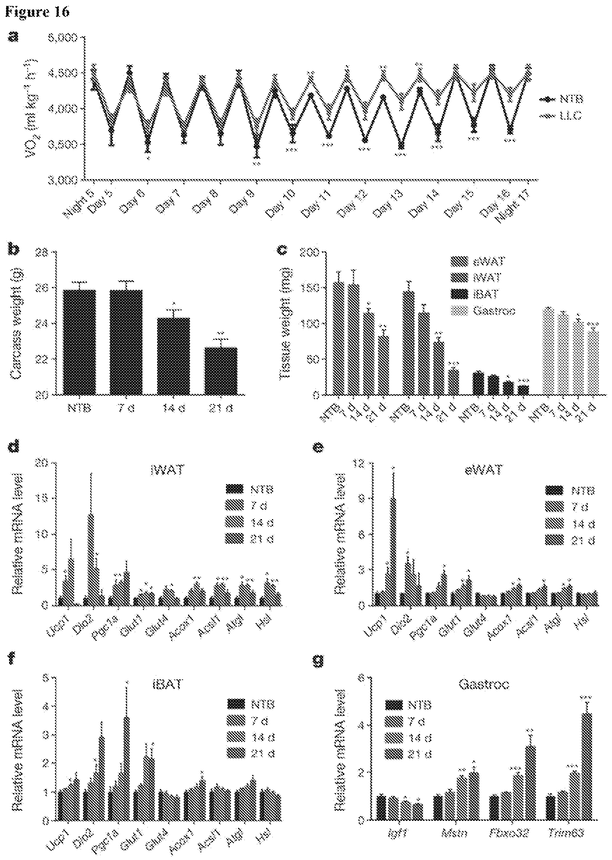

**Figures 16A-16G** show that LLC tumours cause adipose tissue browning and cachexia. Mice were inoculated with LLC cells and monitored for up to 21 days (n = 6 for each group). Four days after inoculation, a cohort of mice (n = 6 for the non-tumour-bearing (NTB) group and n = 9 for the LLC group) was placed into metabolic cages to measure oxygen consumption (VO2) (Figure 16A). Carcass weight (calculated by subtracting tumour weight from the total weight) (Figure 16B) and the weight of fat and muscle tissues (Figure 16c) were measured. The mRNA levels in iWAT (Figure 16D), eWAT (Figure 16E), iBAT (Figure 16F) and the gastrocnemius muscle (Gastroc) (Figure 16G) were determined by quantitative reverse transcription PCR (qRT-PCR). The values are mean ± s.e.m. Statistical analysis was conducted using the two-tailed t-test. *, P < 0.05; **, P < 0.005; ***, P < 0.0005 compared with the NTB group. d, day.

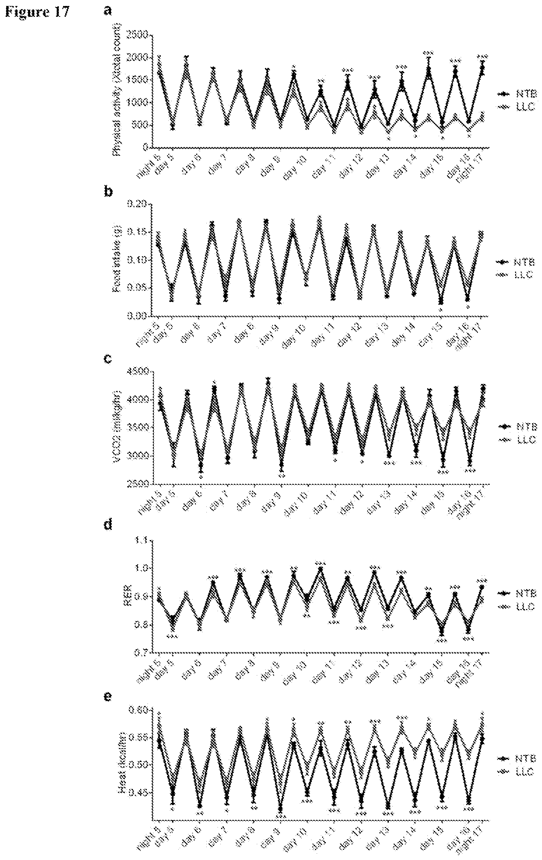

**Figures 17A-17E** show the effects of LLC tumours on whole-body metabolism, food intake and physical activity. Mice inoculated with LLC cells (n = 6 and 9 for the NTB and LLC groups, respectively) were placed into metabolic cages on day 4. Physical activity (Figure 17A), food intake (Figure 17B), carbon dioxide production (Figure 17C), respiratory exchange ratio (RER) (Figure 17D) and heat output (Figure 17E) were monitored. The values are mean ± s.e.m. Statistical analysis was conducted using the two-tailed t-test. *, P < 0.05; **, P < 0.005; ***, P < 0.0005.

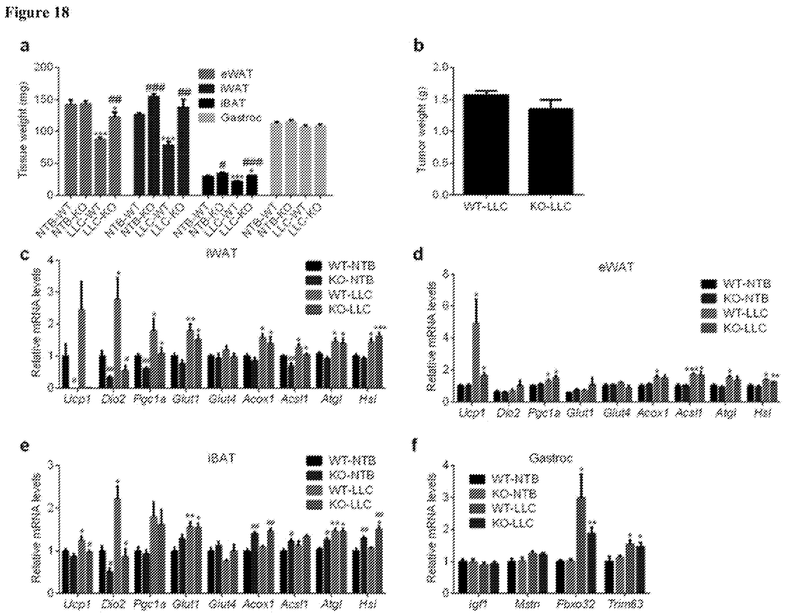

**Figures 18A-18F** show that fat-specific Prdm16-deficient mice are resistant to LLC-tumour-induced adipose tissue browning and wasting. Wild-type (WT) and Prdm16-knockout (KO) mice inoculated with LLC cells were killed on day 11 (n = 5 for the KO-LLC group and 6 for the other groups). Tissue weight (Figure 18A) and tumour weight (Figure 18B) were measured. Gene expression changes in iWAT (Figure 18C), eWAT (Figure 18D), iBAT (Figure 18E) and the gastrocnemius muscle (Figure 18F) were determined by qRT-PCR. The values are mean ± s.e.m. Statistical analysis was conducted using the two-tailed t-test. *, ** and *** denote differences between the NTB group and the LLC group. #, ## and ### denote differences between the WT group and the KO group. *, P < 0.05; **, P < 0.005; ***, P < 0.0005; #, P < 0.05; ##, P < 0.005; ###, P < 0.0005.

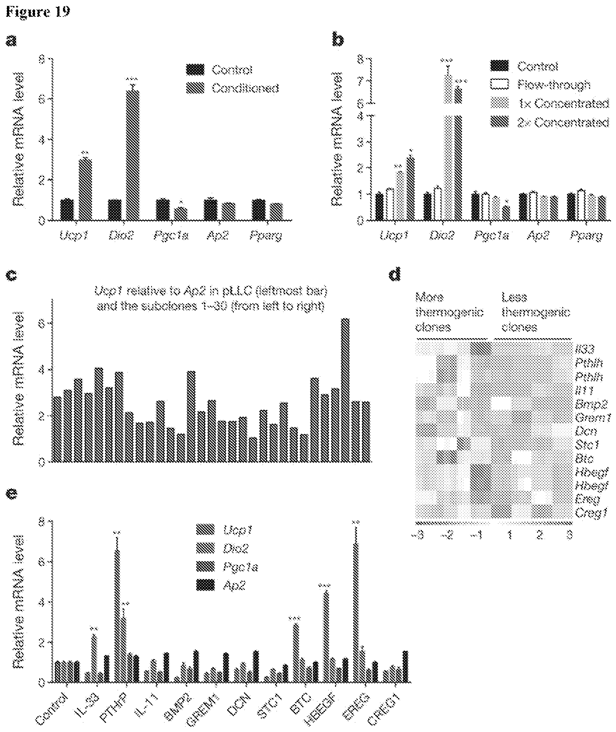

**Figures 19A-19E** show that LLC-cell-conditioned medium stimulates thermogenic gene expression in fat cells. For Figures 19A-19C, parent LLC cells or subclones were cultured in serum-free medium for 24 h. Primary adipocytes (n = 3 in Figures 19A and 19B) were treated for 24 h with LLC-cell-conditioned medium (Figures 19A and 19C) or filtered fractions (Figure 19B). Figure 19D shows a gene expression heat map of the genes encoding the secreted factors identified in the microarray analysis. Figure 19E shows the results of primary adipocytes treated with the indicated proteins (1 µg ml⁻¹) for 24 h (n = 3). The mRNA levels corresponding to the indicated genes were determined by qRT-PCR. Ap2 (also known as Fabp4) and Pparg are controls for adipocyte differentiation. The values are mean ± s.e.m. Statistical analysis was conducted using the two-tailed t-test. *, P < 0.05; **, P < 0.005; ***, P < 0.0005, compared with the control group.

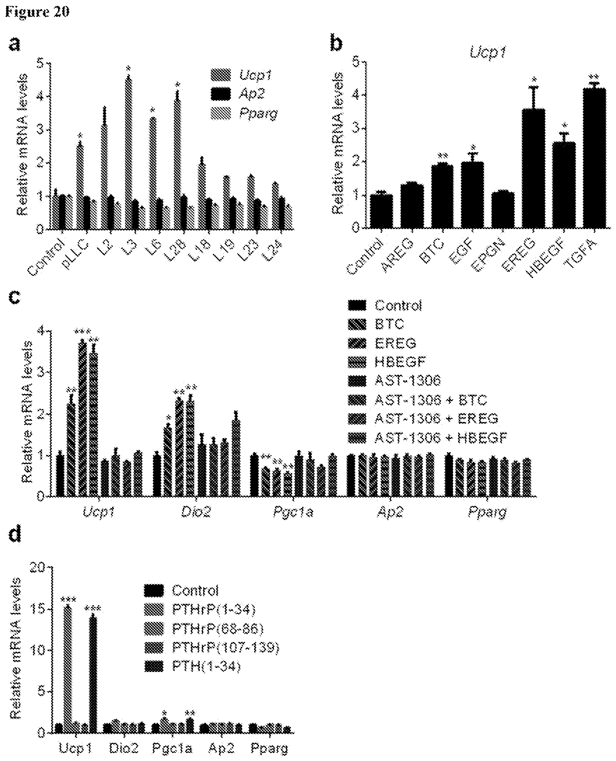

**Figures 20A-20D** show that LLC-subclone-conditioned medium, EGF proteins, and PTHrP(1-34) and PTH(1-34) peptides stimulate Ucp1 and Dio2 expression in adipocytes. Figure 20A shows the results of parent LLC (pLLC) cells or subclones cultured in serum-free medium for 24 h. Primary adipocytes were treated for 24 h with the LLC-cell-conditioned media (n = 3). Figure 20B shows the results of primary adipocytes treated with 100 ng ml⁻¹ soluble EGF domains from the indicated EGF family proteins for 24 h (n = 3). Figure 20C shows the results of primary adipocytes treated with 1 µM AST-1306 and 100 ng ml⁻¹ BTC, EREG or HBEGF for 24 h (n = 3). Figure 20D shows the results of primary brown adipocytes treated with 100 ng ml⁻¹ PTHrP or PTH peptides for 2 h (n = 3). The mRNA levels were determined by qRT-PCR. The values are mean ± s.e.m. Statistical analysis was conducted using the two-tailed t-test. *, P < 0.05; **, P < 0.005; ***,P < 0.0005, compared with the control group.

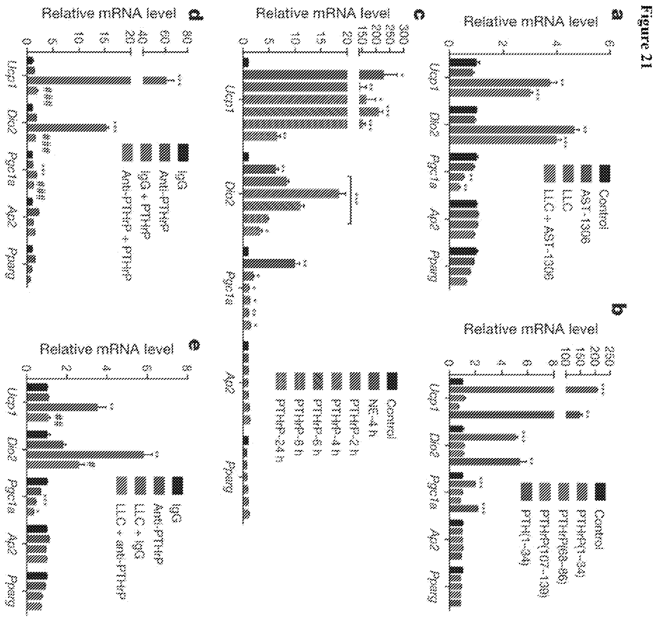

**Figures 21A-21E** show that PTHrP is responsible for most of the LLC-cell-derived browning activity. Figures 21A-21C show the results of primary adipocytes (n = 3 for each group) treated with 1 µM AST-1306 and LLC-cell-conditioned medium for 24 h (Figure 21A), with 100 ng ml⁻¹ of the indicated peptides for 2 h (Figure 21B), or with 100 ng ml⁻¹ PTHrP for 2-24 h or 100 mM noradrenaline (NE) for 4 h (Figure 21C). Figures 21D-21E show the results of primary adipocytes (n = 3 for each group) treated with 10 µg ml⁻¹ IgG or anti-PTHrP antibody along with either 10 ng ml₋₁ PTHrP for 4 h (Figure 21D) or LLC-cell conditioned medium for 24 h (Figure 21E). The mRNA levels were measured by qRT-PCR. The values are mean ± s.e.m. Statistical analysis was conducted using the two-tailed t-test. *, ** and *** denote differences from the control (IgG) group. #, ## and ### denote differences between the PTHrP groups or between the LLC groups. *, P < 0.05; **, P < 0.005; ***, P < 0.0005; #, P < 0.05; ##, P < 0.005; ###, P < 0.0005.

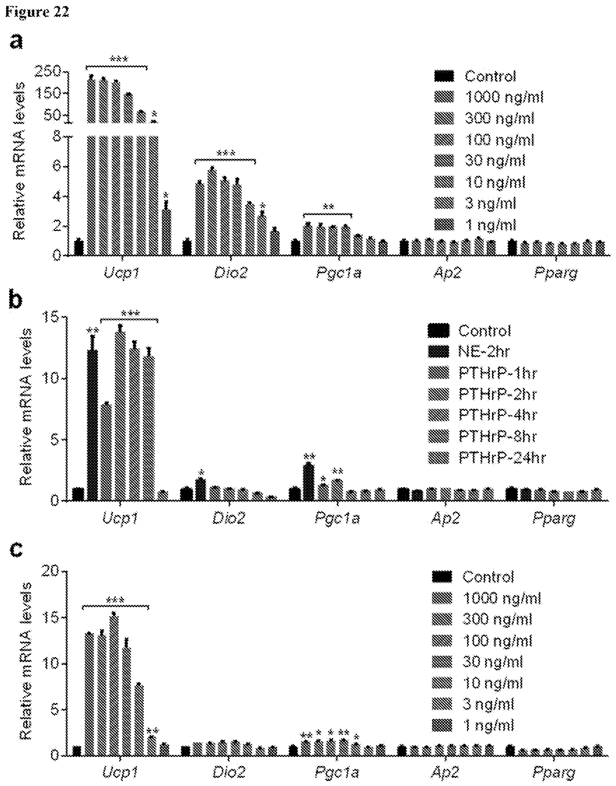

**Figures 22A-22C** show the time-course and dose-response of PTHrP treatment effects on primary white and brown adipocytes. Figure 22A shows the results of primary white adipocytes treated with PTHrP at various doses for 2 h (n = 3). Figure 22B shows the results of primary brown adipocytes were treated with 100 ng ml⁻¹ PTHrP for 2-24 h or with 100 nM noradrenaline (NE) for 2 h (n = 3). Figure 22C shows the results of primary brown adipocytes treated with PTHrP at various doses for 2 h (n = 3). The mRNA levels were measured by qRT-PCR (n = 3). The values are mean ± s.e.m. Statistical analysis was conducted using the two-tailed t-test. *, P < 0.05; **, P < 0.005; ***, P < 0.0005, compared with the control group.

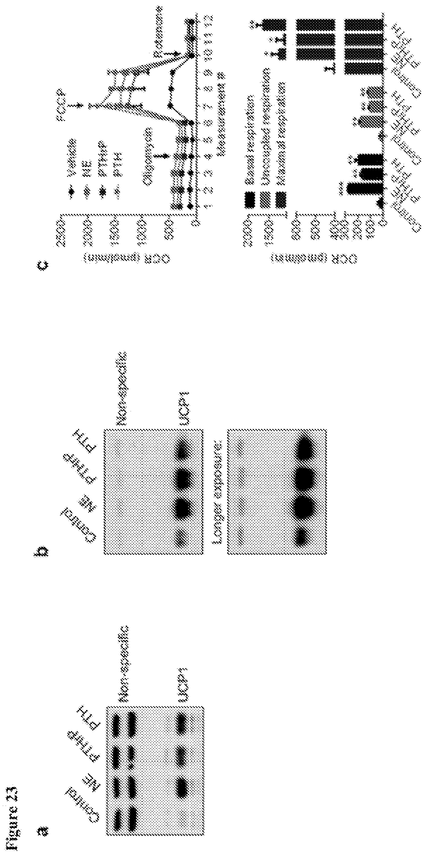

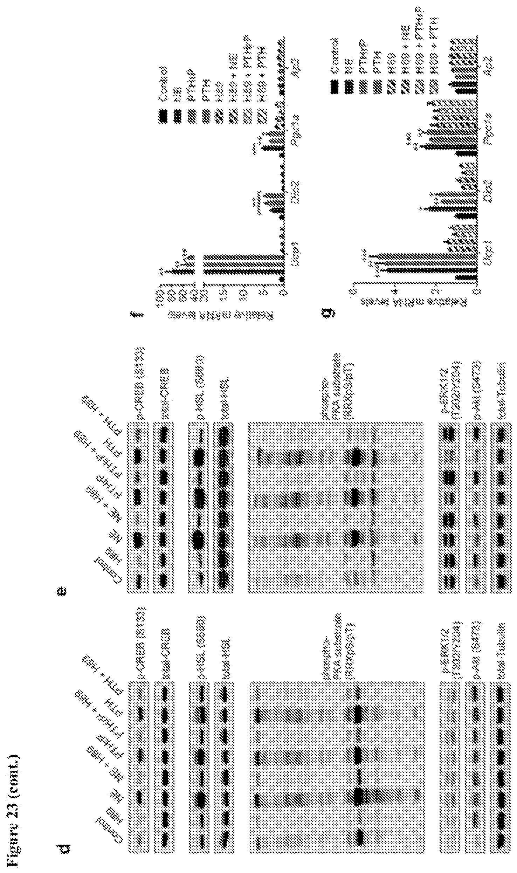

**Figures 23A-23G** show that PTHrP stimulates cellular respiration and acts via the PKA signalling pathway. Figures 23A-23B show the results of primary adipocytes treated with 100 nM NE, 100 ng ml⁻¹ PTHrP or 100 ng ml⁻¹PTH for 24 h. UCP1 protein levels were measured by western blotting in white (Figure 23A) and brown (Figure 23B) adipocytes. Figure 23C shows the oxygen consumption rate (OCR) of primary white adipocytes, including basal respiration, uncoupled respiration (by blocking ATP synthase with oligomycin), maximal respiration (by stimulating uncoupling with FCCP (carbonyl cyanide p-trifluoromethoxyphenylhydrazone)) and non-mitochondrial respiration (with rotenone), as determined using an XF24 Extracellular Flux Analyzer. Real-time triplicate readings (upper panel) and their averages (lower panel) are shown (n = 4). Figures 23D-23G show the results of primary adipocytes serum-starved for 2 h and pretreated with H89 (50 µM for white adipocytes and 30 µM for brown adipocytes) for 1 h, and then 100 nM NE, 100 ng ml⁻¹ PTHrP or 100 ng ml⁻¹ PTH was added for 30 min to analyse protein phosphorylation by western blotting in white (Figure 23D) and brown (Figure 23E) adipocytes or for 2 h to assess gene expression by qRT-PCR (n = 3) in white (Figure 23F) and brown (Figure 23G) adipocytes. The values are mean ± s.e.m. Statistical analysis was conducted using the two-tailed t-test. *, P < 0.05; **,P < 0.005; ***, P < 0.0005, compared with the control group.

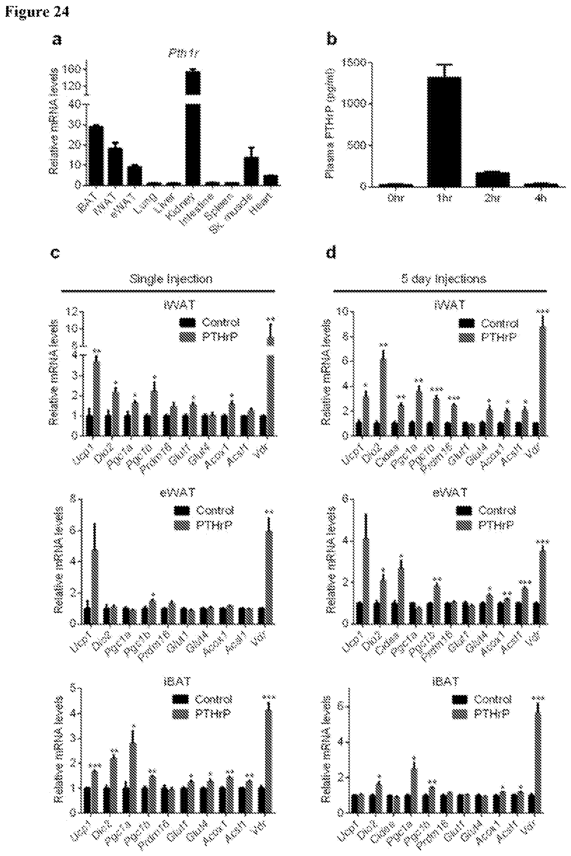

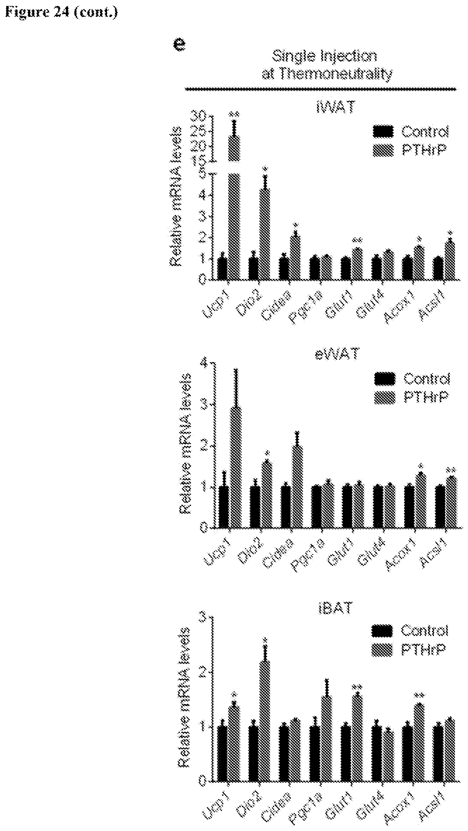

**Figures 24A-24E** show that PTHrP induces thermogenic gene expression patterns in vivo. Figure 24A shows the results of Pthlr (PTH and PTHrP receptor) expression assessed in various tissues (n = 3). Figure 24B shows the results of plasma PTHrP levels determined after subcutaneous injection of mice with 1 mg PTHrP per kg body weight (n = 4). PTHrP(1-34) has a short half-life in the blood (less than 2 h); however, its tissue retention might be different. Figures 24C-24D show the results of mice (n = 5 for the control group and 7 for the PTHrP group) having received a single dose (Figure 24C) or five daily doses (Figure 24D) of 1 mg PTHrP per kg body weight and killed 2 h after the final dose. Figure 24E shows the results of mice (n = 5 for the control group and 7 for the PTHrP group) transferred to thermoneutrality (30°C), and after two days of acclimation they received a single dose of 1 mg PTHrP per kg body weight and were killed 4 h later. More pronounced effects were observed at thermoneutrality, a condition under which basal thermogenic gene expression is diminished. Gene expression changes were measured by qRT-PCR in different fat depots. Vdr was identified as a robust mRNA target of PTHrP and was included as a positive control. The values are mean ± s.e.m. Statistical analysis was conducted using the two-tailed t-test. *, P < 0.05; **, P < 0.005; ***, P < 0.0005.

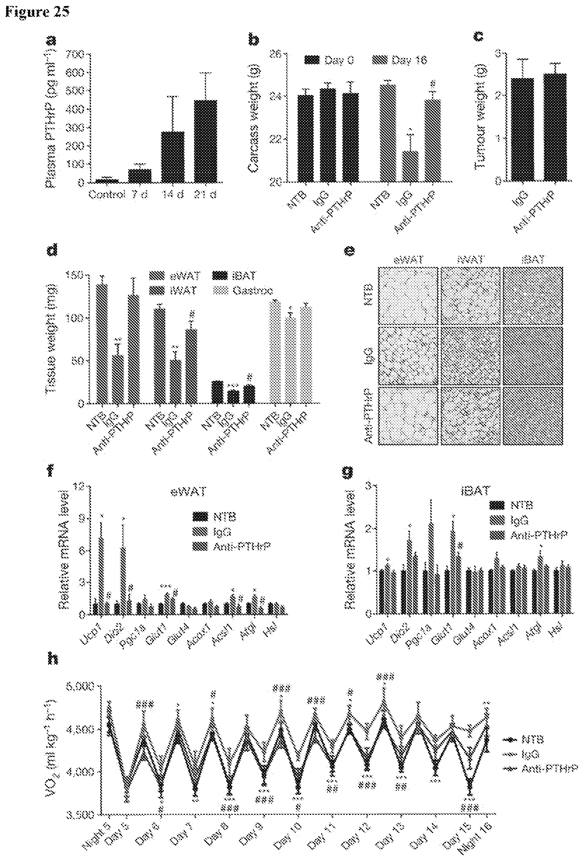

**Figures 25A-25H** show that neutralization of PTHrP prevents tumour-induced adipose tissue browning. Figure 25A shows plasma PTHrP concentrations in mice bearing LLC tumours up to 21 days (n = 6). Figures 25B-25G show the results of mice inoculated with LLC cells having received 10 mg IgG or anti-PTHrP antibody per kg body weight every 3 days from day 6 to day 15 and killed on day 16 (n = 4, 5 and 6 for the NTB, IgG and anti-PTHrP groups, respectively). Carcass weight (Figure 25B), weight of tumours (Figure 25C), weight of fat and muscle tissues (Figure 25D) and haematoxylin and eosin staining of adipose tissues (Figure 25E) are shown. The mRNA levels in eWAT (Figure 25F) and iBAT (Figure 25G) were measured by qRT-PCR. Figure 25H shows the results of mice treated similarly to those used for Figures 25B-25G except that they were placed into metabolic cages on day 4 to measure oxygen consumption (n = 6 for the NTB group and n = 5 for the other groups). The values are mean ± s.e.m. Statistical analysis was conducted using the two-tailed t-test. *, ** and *** denote differences between the NTB group and the IgG group. #, ## and ### denote differences between the anti-PTHrP group and the IgG group. *, P < 0.05; **, P < 0.005; ***, P < 0.0005; #, P < 0.05; ##, P < 0.005; ###, P < 0.0005.

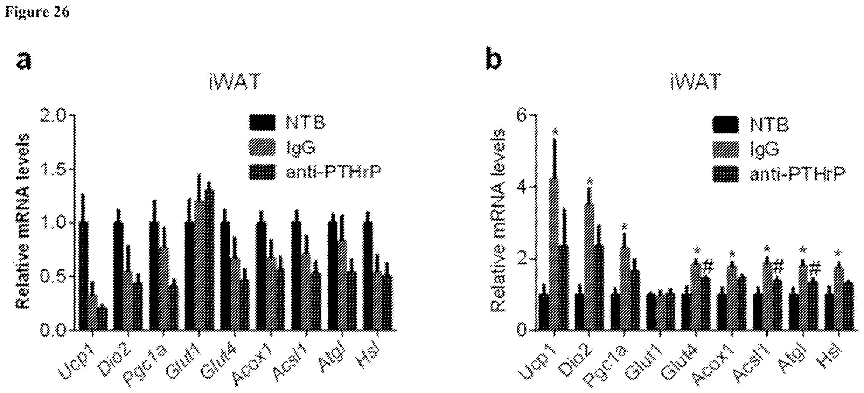

**Figures 26A-26B** show that neutralization of PTHrP prevents tumour-induced adipose tissue browning. Figure 26A show the results of mice inoculated with LLC cells and having received 10 mg kg⁻¹ IgG or anti-PTHrP antibody every 3 days from day 6 to day 15 and killed on day 16 (n = 4, 5 and 6 for the NTB, IgG and anti-PTHrP groups, respectively). At this time point, no increase in the thermogenic gene profile in iWAT was observed. Figure 26B, therefore, shows the results of another neutralization experiment in which the mice were treated similarly to Figure 26A except that they received antibody injections on days 4 and 7 and were killed on day 8. The mRNA levels in iWAT were determined (n = 6 for each group). At this early stage of cachexia, weight loss was not evident. However, the expression of thermogenic genes was increased in iWAT, and the anti-PTHrP treatment blunted these changes. The values are mean ± s.e.m. Statistical analysis was conducted using the two-tailed t-test. * denotes the difference between the NTB group and the IgG group. # denotes the difference between the IgG group and the anti-PTHrP group. *, P < 0.05; #, P < 0.05.

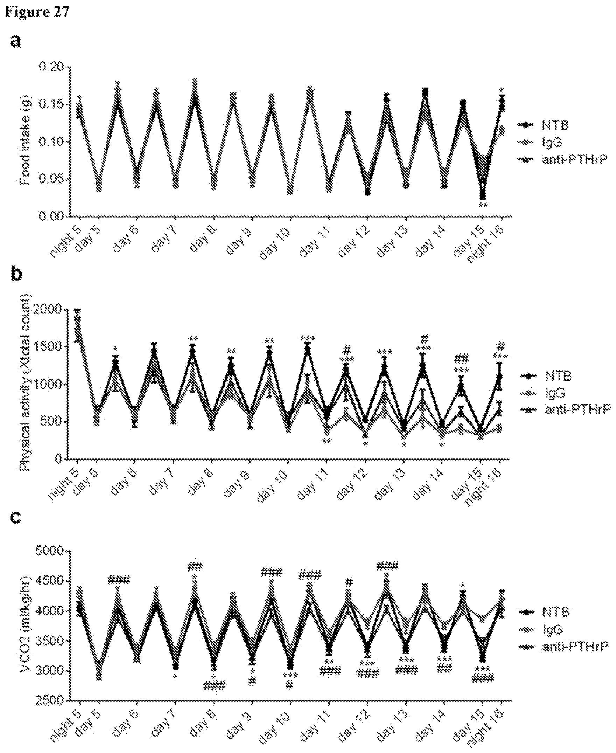

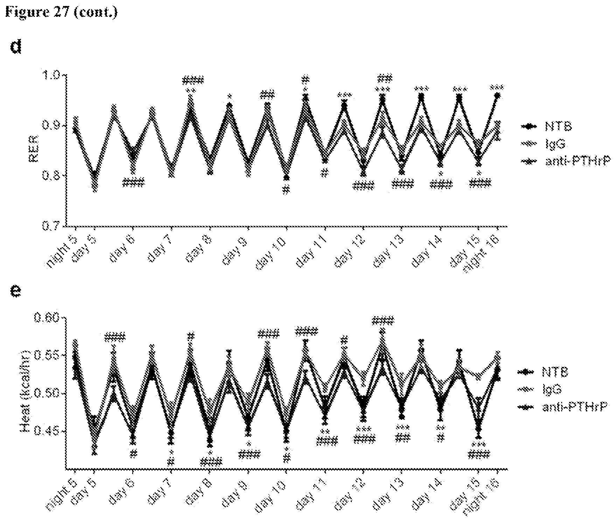

**Figures 27A-27E** show the effects of PTHrP neutralization on whole-body metabolism, food intake and physical activity of cachectic mice. Mice were placed into metabolic cages 4 days after inoculation with LLC cells. They received 10 mg IgG or anti-PTHrP per kg body weight every 3 days from day 6 to day 15 (n = 6 for the NTB group and 5 for the other groups). Food intake (Figure 27A), physical activity (Figure 27B), carbon dioxide production (Figure 27C), respiratory exchange ratio (RER) (Figure 27D) and heat output (Figure 27E) were monitored. The values are mean ± s.e.m. Statistical analysis was conducted using the two-tailed t-test. *, ** and *** denote the difference between the NTB group and the IgG group. #, ## and ### denote the difference between the IgG group and anti-PTHrP group. *, P < 0.05; **, P < 0.005; ***, P < 0.0005; #, P < 0.05; ##, P < 0.005; ###, P < 0.0005.

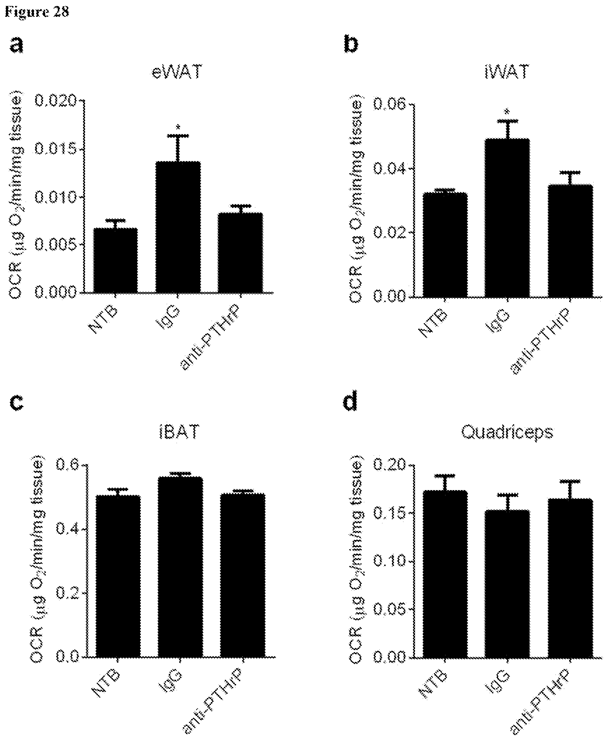

**Figures 28A-28D** show the effects of PTHrP neutralization on *ex vivo* adipose and skeletal muscle tissue respiration. The mice described in Figures 27A-27E were killed on day 16, and their tissues were harvested for the measurement of *ex vivo* respiration using a Clark electrode (n = 6 for the NTB group and 5 for the other groups). The values are mean ± s.e.m. Statistical analysis was conducted using the two-tailed t-test. *P < 0.05 compared with the NTB group.

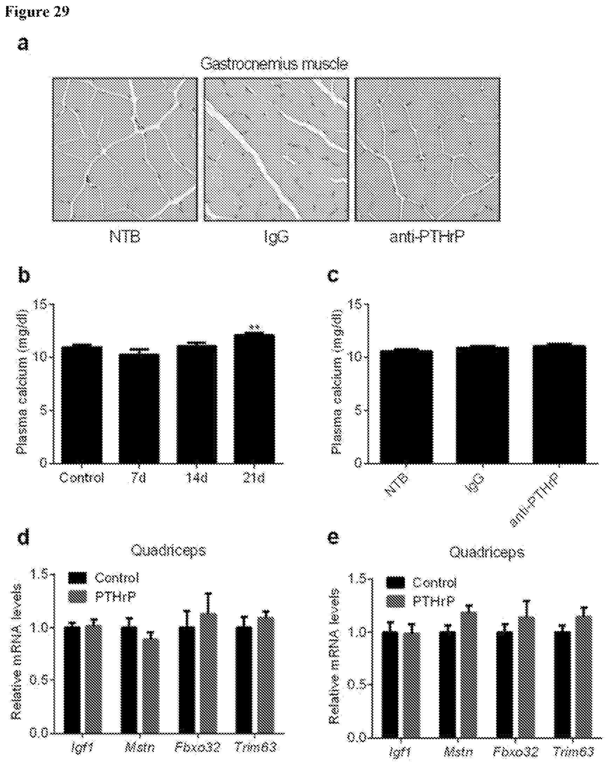

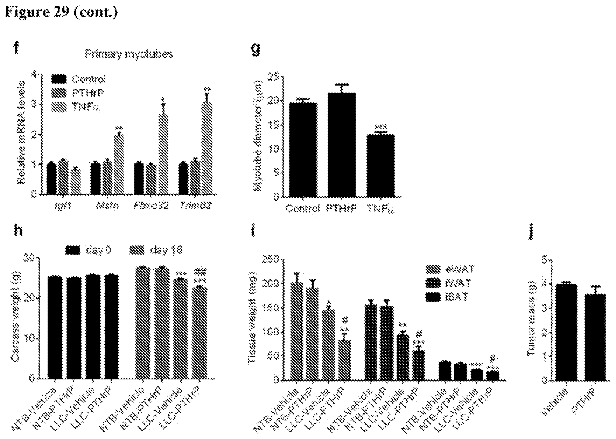

**Figures 29A-29J** show that LLC-tumour-bearing mice did not display hypercalcaemia, and PTHrP treatment alone did not induce expression of muscle-atrophy-associated genes but exacerbated cachexia in the LLC-tumour-bearing mice. Figure 29A shows haematoxylin and eosin staining of a representative cross-sectional area of the gastrocnemius muscles from the experiment described in Figures 25B-25G. Figures 29B-29C show the results of plasma calcium level measured in the experiments described in Figures 25A-25G. Figures 29D-29E show the mRNA levels in the quadriceps muscles from the experiment described in Figures 24C-24D (n = 5 for the control group and 7 for the PTHrP group). Figures 29F-29G show the results of primary myotubes treated with 100 ng ml⁻¹ tumour-necrosis factor-a (TNF-α) or PTHrP for 2 h to test gene expression by qRT-PCR or for 24 h to measure myotube diameter (n = 3). The values are mean ± s.e.m. Statistical analysis was conducted by two-tailed t-test. *, P < 0.05; **, P < 0.005; ***, P < 0.0005, compared with the control group. Figures 29H-29J show the results of mice inoculated with LLC cells killed after receiving daily injections of 1 mg PTHrP per kg body weight between days 10 and 16 (n = 5 for the LLC-Vehicle group and 6 for the other groups). The carcass weight was measured by subtracting the tumour weight from the total weight (Figure 29H). The fat tissues (Figure 29I) and tumours (Figure 29J) were dissected and weighed. The values are mean ± s.e.m. Statistical analysis was conducted using the two-tailed t-test. *, ** and *** denote differences from the NTB-Vehicle group. # and ## denote differences between the LLC-Vehicle group and the LLC-PTHrP group. *, P < 0.05; **, P < 0.005; ***, P < 0.0005; #, P < 0.05; ##, P < 0.005.

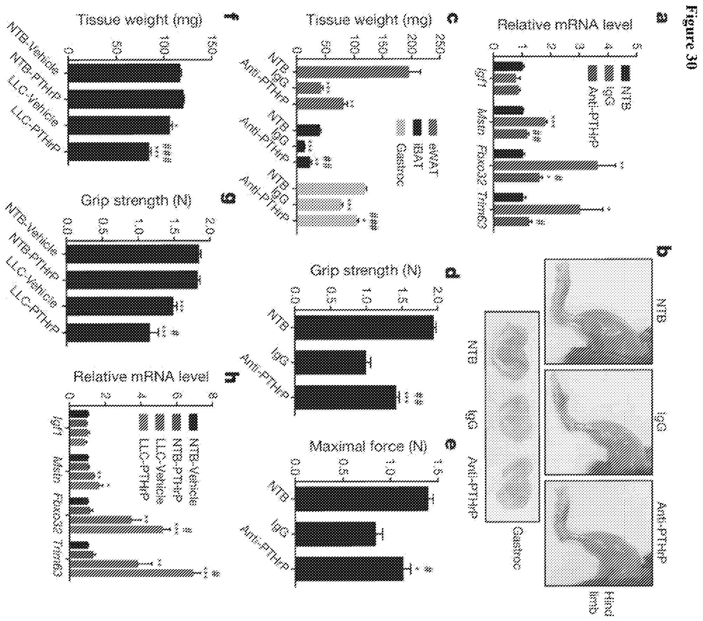

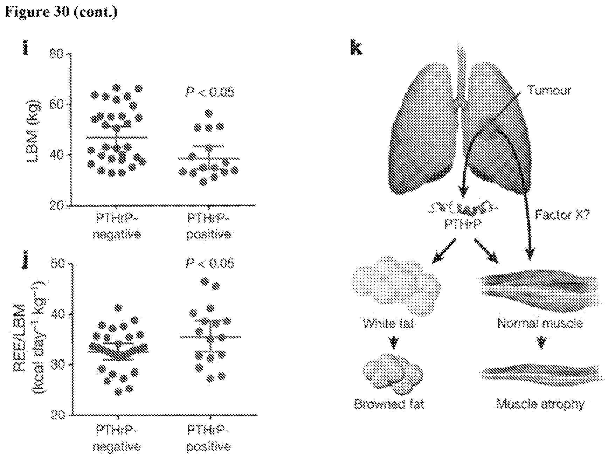

**Figures 30A-30K** show that PTHrP is associated with wasting of the LBM in cachectic mice and humans. Figure 30A show that the mRNA levels in the gastrocnemius muscle from the experiment described in Figures 25B-25G. Figures 30B-309E show the results of mice inoculated with LLC cells and having received 10 mg IgG or anti-PTHrP antibody per kg body weight every 3 days from day 6 to day 21-22 (n = 5 for the anti-PTHrP group and n = 6 for the other groups). Representative images of the hindlimb and gastrocnemius muscles are shown in Figure 30B. Fat and muscle tissues were weighed (Figure 30C). Muscle function was analysed by measuring grip strength (Figure 30D) and *in situ* contraction (Figure 30E). Figures 30F-30H show the results of mice inoculated with LLC cells and killed after receiving daily injections of 1 mg PTHrP per kg body weight between days 10 and 16 (n = 5 for the LLC-Vehicle group (vehicle denotes PBS) and n = 6 for the other groups). The gastrocnemius muscle weight (Figure 30F) and grip strength (Figure 30G) were measured. The mRNA levels in the gastrocnemius muscle were determined by qRT-PCR (Figure 30H). Figures 30I-30J show a comparison of the LBM and REE/LBM of the PTHrP-positive and PTHrP-negative patients. Figure 30K shows a model for PTHrP action in cancer cachexia. For Figures 30A and 30C-30J, the values are mean ± s.e.m. Statistical analysis was conducted using the two-tailed t-test. *, ** and *** denote differences from the NTB group. #, ## and ## denote differences between the IgG group and the anti-PTHrP group or between the LLC-Vehicle group and the LLC-PTHrP group. *, P < 0.05; **, P < 0.005; ***, P < 0.0005; #, P < 0.05; ##, P < 0.005; ###, P < 0.0005.

For any figure showing a bar histogram, curve, or other data associated with a legend, the bars, curve, or other data presented from left to right for each indication correspond directly and in order to the boxes from top to bottom of the legend.

### Detailed Description of the Invention

The present invention is based in part on the discovery that PTH-related and EGF-related polypeptides and biologically active fragments thereof are secreted molecules that have the ability to induce significant thermogenesis. The compositions and methods described herein are useful for modulating a distinct set of target genes characteristic of brown fat cells or downstream effects of brown fat cells including, for example but not limited to, cidea, adiponectin (adipoq), adipsin, otopetrin, type II deiodinase, cig30, ppar gamma 2, pgc1α, ucp1, elovl3, cAMP, Prdm16, cytochrome C, cox4i1, coxIII, cox5b, cox7a1, cox8b, glut4, atpase b2, cox II, atp5o, ndufb5, ap2, ndufs1, GRP109A, acylCoA-thioesterase 4, EARA1, claudin1, PEPCK, fgf21, acylCoA-thioesterase 3, and dio2). Moreover, the compositions and methods described herein are further useful for monitoring and modulating cellular respiration (*i.e.,* basal, total, and/or uncoupled respiration); thermogenesis of adipose cells; insulin sensitivity of adipose, muscle and/or hepatic cells; hepatosteatosis, obesity, type II diabetes, and/or appetite; insulin secretion of pancreatic beta cells; cardiac function to combat cardiac hypertrophy; muscle hypoplasia; the growth and effects of obesity-associated cancer, cachexia, and anorexia; the differentiation, generation, and/or proliferation of adipose cells; blood glucose levels; and diseases or disorders characterized by increased PGC-1 expression or activity. While known PTH and EGF compositions are generally administered in multiple daily doses in order to pharmacologically tailor their effects to treating known indications that require such dosing (e.g., bone remodeling), the agents and methods of the present invention are further useful in modulating thermogenesis over a much longer time period using longer-acting formulations and less-frequent dosing.

In particular the invention is directed to the following:
to an *in vitro* method of modulating a metabolic response comprising contacting an adipose cell with TGFA to increase thermogenesis by the adipose cell.

In a preferred aspect to a method as described above, wherein the TGFA is a polypeptide comprising an amino acid sequence having at least 80% identity to SEQ ID NOs: 2, 4, or 6.

In another preferred aspect to a method as described above, wherein the TGFA is a nucleic acid molecule encoding a TGFA polypeptide and having a nucleic acid sequence at least 80% identity to SEQ ID NOs: 1, 3, or 5.

In another preferred aspect to a method as described above, wherein the TGFA polypeptide lacks a signal peptide.

In another preferred aspect to a method as described above, wherein the TGFA polypeptide further comprises a heterologous polypeptide.

In another preferred aspect to a method as described above,wherein the heterologous polypeptide is selected from the group consisting of a signal peptide, a peptide tag, a dimerization domain, an oligomerization domain, an agent that promotes plasma solubility, an agent that increases *in vivo* protein half-life, an antibody or fragment thereof, and an Fc domain.

In another preferred aspect to a method as described above further comprising the use of an additional agent that increases the metabolic response of the adipose cell.

### I. Definitions

In order that the present invention may be more readily understood, certain terms are first defined. Additional definitions are set forth throughout the detailed description.

The term "amino acid" is intended to embrace all molecules, whether natural or synthetic, which include both an amino functionality and an acid functionality and capable of being included in a polymer of naturally-occurring amino acids. Exemplary amino acids include naturally-occurring amino acids; analogs, derivatives and congeners thereof; amino acid analogs having variant side chains; and all stereoisomers of any of any of the foregoing. The names of the natural amino acids are abbreviated herein in accordance with the recommendations of IUPAC-IUB.

The term "antisense" nucleic acid refers to oligonucleotides which specifically hybridize *(e.g.,* bind) under cellular conditions with a gene sequence, such as at the cellular mRNA and/or genomic DNA level, so as to inhibit expression of that gene, *e.g.,* by inhibiting transcription and/or translation. The binding may be by conventional base pair complementarity, or, for example, in the case of binding to DNA duplexes, through specific interactions in the major groove of the double helix.

The term "binding" or "interacting" refers to an association, which may be a stable association, between two molecules, *e.g.,* between a polypeptide of the invention and a binding partner, due to, for example, electrostatic, hydrophobic, ionic and/or hydrogen-bond interactions under physiological conditions. Exemplary interactions include protein-protein, protein-nucleic acid, protein-small molecule, and small molecule-nucleic acid interactions.

The term "biological sample" when used in reference to a diagnostic assay is intended to include tissues, cells and biological fluids isolated from a subject, as well as tissues, cells and fluids present within a subject.

The term "cachexia" is a condition characterized by weight loss, muscle atrophy, anorexia, fatigue, and weakness. It is commonly seen in patients with chronic progressive diseases such as AIDS, hormone deficiency, chronic obstructive lung disease (COPD), congestive heart failure (CHF), tuberculosis (TB), and cancer. In cachexia, a decline in food intake relative to energy expenditure leads to weight loss. Even with adequate nutritional support, abnormalities in the metabolism of carbohydrates, proteins, and fats causes continued mobilization and ineffective repletion of host tissue. The physiological mechanisms that cause cachexia remain poorly understood, although cachectin/TNF or other inflammatory cytokines have been implicated. Nutritional support can help maintain the body weight of cachectic patients, but does not prevent loss of lean muscle mass. Steroids (particularly progesterone-like drugs) can increase appetite and reverse weight loss, although again there is no evidence that it reverses the loss of muscle mass.

The term "EGF-related molecule" refers to EGF and related ligands that can activate the epidermal growth factor receptor (EGFR). The epidermal growth factor (EGF) family of ligands that can activate the EGFR include TGF-alpha (TGFA), Epiregulin (EREG), EGF, Heparin-Binding EGF (HB-EGF), Epigen (EPGN), Amphiregulin (AREG), Betacellulin (BTC), and the Neuregulins (NRG)-1, NRG-2, NRG-3, and NRG-4. The EGFR ligands share a structural motif, the EGF-like domain, characterized by three intramolecular disulfide bonds that are formed by six similarly spaced conserved cysteine residues. EGFR ligands activate the EGFR to promote cellular proliferation, which can be determined in a number of manners (e.g., by bromodeoxyuridine ("BrDU") incorporation and is measured with a colorimetric BrDU ELISA kit). EGFR is also part of a family of receptors including itself, otherwise known as Her and ErbB1, as well as other distinct tyrosine kinase receptors, including HER2/Neu/ErbB2, HER3/ErbB3 and HER4/ErbB4. It has also been determined herein that at least BTC, EREG, and HB-EGF activated both EGFR and ERBB4 leading to increased thermogenesis and browning of fat. Sequences for EGF-related molecules and their receptor(s) are well known in the art (see, *e.g.,* US Pat. Publ. 2013-0090366).

The term "isolated polypeptide" refers to a polypeptide, in certain embodiments prepared from recombinant DNA or RNA, or of synthetic origin, or some combination thereof, which (1) is not associated with proteins that it is normally found within nature, (2) is isolated from the cell in which it normally occurs, (3) is isolated free of other proteins from the same cellular source, (4) is expressed by a cell from a different species, or (5) does not occur in nature.

The terms "label" or "labeled" refer to incorporation or attachment, optionally covalently or non-covalently, of a detectable marker into a molecule, such as a polypeptide. Various methods of labeling polypeptides are known in the art and may be used. Examples of labels for polypeptides include, but are not limited to, the following: radioisotopes, fluorescent labels, heavy atoms, enzymatic labels or reporter genes, chemiluminescent groups, biotinyl groups, predetermined polypeptide epitopes recognized by a secondary reporter (e.g., leucine zipper pair sequences, binding sites for secondary antibodies, metal binding domains, epitope tags). Examples and use of such labels are described in more detail below. In some embodiments, labels are attached by spacer arms of various lengths to reduce potential steric hindrance.\

The term "manage weight" includes, but is not limited to, treating, preventing or reducing weight gain, and/or suppressing appetite.

The terms "metabolic disorder" and "obesity related disorders" are used interchangeably herein and include a disorder, disease or condition which is caused or characterized by an abnormal or unwanted metabolism *(i.e.,* the chemical changes in living cells by which energy is provided for vital processes and activities) in a subject. Metabolic disorders include diseases, disorders, or conditions associated with aberrant or unwanted (higher or lower) thermogenesis or aberrant or unwanted levels (high or low) adipose cell (*e.g.,* brown or white adipose cell) content or function. In some embodiments, metabolic disorders can be characterized by a misregulation *(e.g.,* downregulation or upregulation) of PGC-1 activity. Metabolic disorders can detrimentally affect cellular functions such as cellular proliferation, growth, differentiation, or migration, cellular regulation of homeostasis, inter- or intra-cellular communication; tissue function, such as liver function, muscle function, or adipocyte function; systemic responses in an organism, such as hormonal responses *(e.g.,* insulin response). Examples of metabolic disorders include obesity, insulin resistance, type II diabetes, hypertension, hyperuricemia, hyperglycemia, fatty liver, non-alcoholic fatty liver disease, polycystic ovarian syndrome, acanthosis nigricans, hyperphagia, endocrine abnormalities, triglyceride storage disease, Bardet-Biedl syndrome, Lawrence-Moon syndrome, Prader-Labhart-Willi syndrome, hypoglycemia, anorexia, and cachexia.

The term "obesity" refers to a body mass index (BMI) of 30 kg/m² or more (National Institute of Health, Clinical Guidelines on the Identification, Evaluation, and Treatment of Overweight and Obesity in Adults (1998)). However, the present invention is also intended to include a disease, disorder, or condition that is characterized by a body mass index (BMI) of 25 kg/m² or more, 26 kg/m² or more, 27 kg/m² or more, 28 kg/m² or more, 29 kg/m² or more, 29.5 kg/m² or more, or 29.9 kg/m² or more, all of which are typically referred to as overweight (National Institute of Health, Clinical Guidelines on the Identification, Evaluation, and Treatment of Overweight and Obesity in Adults (1998)). The obesity described herein may be due to any cause, whether genetic or environmental. Examples of disorders that may result in obesity or be the cause of obesity include overeating and bulimia, polycystic ovarian disease, craniopharyngioma, the Prader-Willi Syndrome, Frohlich's syndrome, Type II diabetics, GH-deficient subjects, normal variant short stature, Turner's syndrome, and other pathological conditions showing reduced metabolic activity or a decrease in resting energy expenditure as a percentage of total fat-free mass, *e.g.,* children with acute lymphoblastic leukemia.

The term "PTH-related molecule" refers to a class of ligands that can bind and transduce a signal through the parathyroid hormone receptor (PTH1R) and are well known in the art (see, *e.g.,* U.S. Pat. Publs. 2005-0124537, 2012-0083448, and 2013-0116180 for further details of the description provided below). The PTH1R is a class B G protein-coupled receptor (GPCR) that transduces signals from two related signaling molecules: parathyroid hormone (PTH) and parathyroid hormone-related protein (PTHrP) (Juppner et al. (1991) Science 254:1024-1026, Gardella and Juppner (2000) Rev. Endocrin. Metabol. Dis. 1:317-329, and Gensure et al. (2005) Biochem. Biophys. Res. Comm. 328:666-678).

As explained further below, classical PTH is an 84-amino acid polypeptide endocrine hormone that was originally identified as being produced by the parathyroid glands and secreted into the circulation in response to low calcium levels (Murray et al. (2005) Endocrinol. Rev. 26:78-113, Potts (2005) J. Endocrinol. 187:311-325 and Potts and Gardella (2007) Annal. N.Y. Acad. Sci. 1117:196-208). The classical actions of PTH are mediated by PTH1R expressed in bone and kidney tissues and include stimulation of osteoclastic bone resorption to maintain calcium homeostasis, and stimulation of calcium reabsorption, synthesis of 1,25-dihydroxyvitamin D3, and phosphate excretion in kidney. Paradoxically, PTH also has stimulatory effects on osteoblastic cells resulting in bone formation (Jilka (2007) Bone 40:1434-1446), providing the molecular basis for the clinical use of PTH as an anabolic therapy for osteoporosis (Neer et al. (2001) New Engl. J. Med. 344:1434-1441). Anabolic PTH therapy requires intermittent administration to avoid the bone resorptive effects that predominate with sustained elevation of PTH in the circulation.

Classical PTHrP is a 141-amino acid polypeptide that was originally isolated as the factor responsible for humoral hypercalcemia of malignancy (Burtis et al. (1987) J. Biol. Chem. 262:7151-7156, Moseley et al. (1987) Proc. Natl. Acad. Sci. USA 84:5048-5052, Suva et al. (1987) Science 237:893-896, and Klein et al. (1987) Endocrinol. 120:504-511) and was subsequently shown to be a critical developmental factor that regulates endochondral bone formation (Karaplis et al. (1994) Genes Dev. 8:277-289, Lanske et al. (1996) Science 273:663-666, and Kronenberg (2006) Annal. N.Y. Acad. Sci. 1068:1-13). PTHrP is produced locally and was originally identified to function in a paracrine/autocrine fashion to activate PTH1R expressed on chondrocytes to regulate their proliferation and differentiation. PTHrP also has anabolic effects when administered to osteoporosis patients (Horwitz et al. (2003) J. Clin. Endocrinol. Metab. 88:569-575), but appears to be more purely anabolic than PTH by uncoupling bone formation from bone resorption (Plotkin et al. (1998) J. Clin. Endocrinol. Metab. 83:2786-2791).

The N-terminal 34-residue peptide fragments of PTH and PTHrP are sufficient to bind and activate PTH1R to the same extent as the native molecules, and PTH (1-34) and PTHrP (1-34) are equally potent for activating cAMP signaling (Juppner et al. (1991) Science 254:1024-1026). Their interaction with the receptor follows a "two-domain" model. Residues 1-14 interact with the 7-transmembrane (7-TM) helical domain embedded in the membrane and residues 15-34 interact with the N-terminal extracellular domain (ECD) of the receptor (Bergwitz et al. (1996) J. Biol. Chem. 271:26469-26472 and Luck et al. (1999) Mol. Endocrinol. 13:670-680). The 1-14 fragments of PTH and PTHrP have eight amino acid sequence identities reflecting the critical role this fragment plays in activating the receptor (Luck et al. (1999) Mol. Endocrinol. 13:670-680). The 15-34 fragments impart high affinity binding to the receptor, but this portion of PTH and PTHrP is less conserved with only three amino acid identities. PTH and PTHrP form similar alpha-helical structures in solution (Marx et al. (2000) Biochem. Biophys. Res. Comm. 267:213-220 and Weidler et al. (1999) FEBS Lett. 444:239-244).

Despite their shared two-domain receptor binding mechanism, common secondary structure, and equipotent activation of signaling, PTH and PTHrP differ in their abilities to bind to two pharmacologically distinct PTH1R conformations that are distinguished by the presence or absence of G protein-coupling. The peptides bind with similar high affinity to the G protein-coupled receptor (RG conformational state), but in the absence of G protein coupling (R0 conformational state) PTHrP binding is significantly diminished whereas PTH binding is only slightly decreased (Dean et al. (2008) Mol. Endocrinol. 22:156-166, Dean et al. (2006) Mol. Endocrinol. 20:931-943, and Hoare et al. (2001) J. Biol. Chem. 276:7741-7753). Thus, PTHrP is more RG-selective than PTH. The different R0/RG selectivity profiles of PTH and PTHrP correlate with distinct temporal effects on cAMP signaling. PTH elicits a longer lasting cAMP signal after ligand wash-out than PTHrP (Dean et al. (2008) Mol. Endocrinol. 22:156-166). Divergent residue 5 (Ile in PTH, His in PTHrP) is a key determinant of the R0/RG selectivity differences of the peptides (Dean et al. (2008) Mol. Endocrinol. 22:156-166 and Hoare et al. (2001) J. Biol. Chem. 276:7741-7753), but the 15-34 fragment of PTH contributes to its strong R0 binding (Dean et al. (2008) Mol. Endocrinol. 22:156-166 and Okazaki et al. (2008) Proc. Natl. Acad. Sci. USA 105:16525-16530) suggesting that interactions with the ECD contribute to the duration of cAMP signaling. Importantly, temporal differences in cAMP signaling can have dramatic effects *in vivo.* Compared to wild type PTH, PTH analogs that exhibit increased R0 binding induce sustained cAMP responses in cells and result in increased trabecular bone volume and increased cortical bone resorption in mice receiving daily injections (Dean et al. (2008) Mol. Endocrinol. 22:156-166). Despite these differential effects on bone, it has been determined herein that PTH and PTHrP, as well as fragments thereof, have thermogenic inducing activity.

The term "subject" refers to mammals and includes humans, primates, livestock animals *(e.g.,* sheep, pigs, cattle, horses, and donkeys), laboratory test animals *(e.g.,* mice, rabbits, rats, and guinea pigs), performance and show animals *(e.g.,* horses, livestock, dogs, and cats), companion animals *(e.g.,* dogs and cats) and captive wild animals. In some embodiments, the mammal is human or a laboratory test animal. In other embodiments, the mammal is a human.

The term "thermogenesis" generally refers to the process of increasing energy expenditure by increasing the metabolic rate and/or burning stored body fat. This can be achieved by "browning of fat" whereby brown fat is induced from adipose precursors or cells having no inherent adipose character or lineage and/or white fat is converted into brown fat. Adipose tissue in mammals and some non-mammal vertebrates is composed of at least two distinct forms of adipose termed white adipose and brown adipose that differ significantly in both structure and function (see Saely et al. (2010) Gerontology (Karger A G, Basel)). White adipose tissue is the primary site of energy storage in the form of fat, and excess accumulation of fat in subcutaneous and visceral white adipose tissue depots leads to weight gain and obesity. White adipose tissue is also involved in releasing hormones and cytokines that modulate whole-body metabolism and insulin resistance. White adipocytes, the primary cellular component of white adipose tissue, are characterized by a single large lipid droplet. In contrast, brown adipose tissue is important for both basal and inducible energy expenditure in the form of thermogenesis mediated by the activity of uncoupling protein 1 (UCP-1), a protein specifically expressed in brown adipose tissue. Increased metabolic activity of brown adipose tissue, *e.g.,* in response to cold exposure or to diet, results in increased thermogenesis and heat production. Brown adipocytes, the primary cellular component of brown adipose tissue, are characterized by numerous small lipid droplets and much higher numbers of mitochondria relative to white adipocytes. Brown adipose tissue can affect whole-body metabolism and may alter insulin sensitivity and modify susceptibility to weight gain. Brown adipose tissue, for example, can have profound effects on body weight, energy balance, plasma triglyceride levels, and glucose metabolism.

In rodents, brown adipose tissue tends to be localized within the interscapular, subscapular, renal, and paraspinal regions, whereas in humans the main depots of brown adipose tissue are supraclavicular, cervical, and paraspinal. Brown adipose tissue functions in cold- and diet-induced thermogenesis, which significantly contributes to the control of body temperature and energy expenditure. Thermogenesis in brown adipose tissue may represent a defense mechanism against obesity by increasing energy expenditure. Ablation of brown adipose tissue in rodents, for example by excision or by using a toxic gene, results in hyperphagia and obesity, supporting a role for brown adipose tissue in metabolic homeostasis. Likewise, the absence of brown adipose tissue activity is more apparent in overweight humans, in particular those of advancing age, correlating with obesity. In addition, a lack of UCP1 activity is sufficient to cause or aggravate obesity in mice. Considering the advances in studies into human brown adipose tissues, data regarding effects of brown adipose tissue on metabolism have been extrapolated to human biology, yielding explanations for human metabolic phenomena (see Nedergaard and Cannon (2010) Cell Metab. 11:268-272).

In general, the accumulation of white adipose tissue, for example due to an imbalance in caloric intake relative to energy expenditure, contributes to increased weight gain and obesity. The pathological accumulation of excess white adipose tissue that characterizes obesity is a major risk factor for the development of other diseases including Type 2 diabetes, cardiovascular disease, hypertension, stroke, arthritis, and various types of cancer. In contrast, brown adipose tissue evolved in mammals to dissipate large amounts of chemical energy as heat. In rodents, the metabolic activity of brown adipose tissue increases in response to feeding, essentially diet-induced adaptive thermogenesis, and may be a compensatory mechanism to limit excess weight gain and obesity. Rodents lacking the ability to undergo UCP-1 dependent thermogenesis are prone to obesity. In humans with limited detectable brown adipose tissue, this compensatory mechanism may be lacking, and as such, excessive caloric intake may lead to accumulation of fat and weight gain. Activation and/or proliferation of brown adipose tissue and increased thermogenesis may provide a mechanism for limiting and/or reducing weight gain and treating obesity and associated secondary diseases (see Seale et al. (2009) Diabetes 58:1482-1484 and Cannon and Nedergaard (2009) Proc. Nutr. Soc. 68:401-407).

Metabolically active brown adipocytes can take up glucose from the peripheral circulation. Cold exposure increases glucose utilization in brown adipose tissue by several fold and is dependent upon intact sympathetic innervations, *e.g.,* localized release of norepinephrine. In humans, glucose, in the form of ¹⁸FDG as used for imaging by PET-CT, is taken up into metabolically active brown adipose tissue depots. Such uptake is increased at lower temperatures (Lee et al. (2010) Am. J. Physiol. Endocrinol. Metab. 299:E601-E606, Saito et al. (2009) Diabetes 58:1526-1531, Yoneshiro et al. (2011) Obesity 19:13-16; van Marken Lichtenbelt et al. (2009) N. Engl. J. Med. 360:1500-1508; Cypess et al. (2009) N. Engl. J. Med. 360:1509-1517; and Virtanen et al. (2009) N. Engl. J. Med. 360:1518-1525). Glucose uptake is also reduced by pretreatment with β-adrenergic blockers (Parysow et al. (2007) Clin. Nucl. Med. 32:351-357 and Soderlund et al. (2007) J. Nucl. Med. Mol. Imag. 34:1018-1022). Likewise, the level of detectable brown adipose tissue is inversely proportional to blood glucose levels. Individuals with little detectable brown adipose tissue have increased glucose levels. In patients scanned more than once, lower fasting glucose levels correlated with increased detectable brown adipose tissue (Lee et al. (2010) Am. J. Physiol. Endocrinol. Metab. 299:E601-E606). The presence of metabolically active brown adipose tissue can lower the levels of glucose in the blood stream, which may have beneficial consequences for the treatment of diabetes and other metabolic disorders including metabolic syndrome. The treatment goal for both Type 1 and Type 2 diabetes is to maintain the levels of glucose at near, normal levels. As such, controlled modulation of brown adipose tissue activity and thermogenesis can be a mechanism for controlling the levels of glucose in the blood stream and consequently treating metabolic disorders.

In addition, numerous PTH-related molecules and EGF-related molecules from humans, as well as orthologs in other species, are well known in the art.

At least two splice variants encoding two distinct human TGF-alpha (TGFA) isoforms exist. Human TGFA transcript variant 1 (GenBank Accession number NM_003236.3, hereinafter simply referred to by accession number without explicit reference to GenBank) encodes the long TGFA isoform 1 (NP_003227.1). Human transcript variant 2 (NM_001099691.2) uses an alternate in-frame splice site in the coding region and encodes the smaller TGFA isoform 2 (NP_001093161.1). For human TGFA isoform 1, amino acid residues 1-23 represent the signal peptide, amino acid residues 24-160 represent TGFA propeptide, and 40-89 represent mature TGFA. Amino acid residues 24-39 represent the N-terminal propeptide sequence, amino acid residues 90-160 represent the C-terminal propeptide sequence, amino acid residue 25 can be glycosylated, amino acid pairs 47 and 60, 55 and 71, and 73 and 82, can form disulfide bonds, amino acid residues 39 and 89 are predicted proteolytic cleavage sites, and amino acid residues 153 and 154 are predicted palmitoylation sites. For human TGFA isoform 2, amino acid residues 1-23 represent the signal peptide and amino acid residues 39-88 represent the mature TGFA peptide. The nucleotide and amino acid sequences of mouse PTHrP are publicly available under NM_031199.3 and NP_112476.1, respectively. For mouse TGFA, amino acid residues 1-23 represent the signal peptide, amino acid residues 24-159 represent the TGFA proprotein, and amino acid residues 39-88 represent the mature TGFA peptide. Amino acid residues 24-38 represent the N-terminal propeptide sequence, amino acid residues 89-159 represent the C-terminal propeptide sequence, amino acid residue 25 can be glycosylated, amino acid pairs 46 and 59, 54 and 70, and 72 and 81, can form disulfide bonds, and amino acid residues 152 and 153 can be palmitoylated. Nucleic acid and polypeptide sequences of TGFA orthologs in species other than mice and humans are well known and include, for example, chimpanzee TGFA (XM_001142892.2 and XP_001142892.1), monkey TGFA (XM_001100015.2, XP_001100015.2, XM_002799271.1, and XP_002799317.1), cow TGFA (XM_002691233.2 and XP_002691279.2), rat TGFA (NM_012671.2 and NP_036803.1), chicken TGFA (NM_001001614.1 and NP_001001614.1), and zebrafish TGFA (NM_001144052.1 and NP_001137524.1).

The nucleotide and amino acid sequences of human epiregulin (EREG) are publicly available under NM_001432.2 and NP_001423.1. For human EREG, amino acid residues 1-29 represent the signal peptide, amino acid residues 30-169 represent the EREG proprotein, and amino acid residues 60-108 represent the mature EREG peptide. Amino acid residues 30-59 represent the N-terminal propeptide sequence, amino acid residues 109-169 represent the C-terminal propeptide sequence, amino acid residue 47 can be glycosylated, and amino acid pairs 68 and 81, 76 and 92, and 94 and 103, can form disulfide bonds. The nucleotide and amino acid sequences of mouse EREG are publicly available under NM_007950.2 and NP_031976.1. For mouse EREG, amino acid residues 1-22 represent the signal peptide, amino acid residues 23-162 represent the EREG proprotein, and amino acid residues 53-101 represent the mature EREG peptide. Amino acid residues 23-52 represent the N-terminal propeptide sequence, amino acid residues 102-162 represent the C-terminal propeptide sequence, amino acid residue 40 can be glycosylated, and amino acid pairs 61 and 74, 69 and 85, and 87 and 96, can form disulfide bonds. Nucleic acid and polypeptide sequences of EREG orthologs in species other than mice and humans are well known and include, for example, chimpanzee EREG (XM_001155238.3 and XP_001155238.1), monkey EREG (XM_001102069.1 and XP_001102069.1), dog EREG (XM_849667.2 and XP_854760.2), cow EREG (XM_002688367.2 and XP_002688413.2), rat EREG (NM_021689.1 and NP_067721.1), and chicken EREG (NM_001001203.1 and NP_001001203.1).

At least three splice variants encoding three distinct human epidermal growth factor (EGF) isoforms exist. Human EGF transcript variant 1 (NM_001963.4) encodes the longest EGF isoform 1 (NP_001954.2). Human transcript variant 2 (NM_001178130.1) lacks an in-frame exon in the coding region compared to variant 1 and encodes the smaller EGF isoform 2 (NP_001171601.1). Human transcript variant 3 (NM_001178131.1) also lacks an in-frame exon in the cording region compared to variant 1 and encodes the smaller EGF isoform 3
(NP_001171602.1). For human EGF isoform 1, amino acid residues 1-22 represent the signal peptide, amino acid residues 23-1207 represent EGF proprotein, and amino acid residues 971-1023 represent the mature EGF peptide. For human EGF isoform 2, amino acid residues 1-22 represent the signal peptide, amino acid residues 23-1166 represent the EGF proprotein, and amino acid residues 930-982 represent the mature EGF peptide. For human EGF isoform 3, amino acid residues 1-22 represent the signal peptide, amino acid residues 23-1165 represent the EGF proprotein, and amino acid residues 929-981 represent the mature EGF peptide. The nucleotide and amino acid sequences of mouse EGF are publicly available under NM_010113.3 and NP_034243.2, respectively. For mouse EGF, amino acid residues 1-28 represent the signal peptide, amino acid residues 29-1217 represent the EGF proprotein, and amino acid residues 977-1029 represent the mature EGF peptide. Nucleic acid and polypeptide sequences of EGF orthologs in species other than mice and humans are well known and include, for example, chimpanzee EGF (XM_517395.3, XP_517395.2, XM 003310447.1, XP_003310495.1, XM_003310446.1, and XP_003310494.1), monkey EGF (XM_001088957.2, XP_001088957.1, XM_002804174.1, and XP_002804220.1), dog EGF (NM_001003094.1 andNP_001003094.1), cow EGF (XM_002688103.1 and XP_002688149.1), rat EGF (NM_012842.1 and NP_036974.1), chicken EGF (NM_001001292.1 and NP_001001292.1), and zebrafish EGF (NM_205731.1 and NP_991294.1).

The nucleotide and amino acid sequences of human heparin-binding EGF-like growth factor (HB-EGF) are publicly available under NM_001945.2 and NP_001936.1. For human HB-EGF, amino acid residues 1-19 represent the signal peptide, amino acid residues 20-208 represent the HB-EGF proprotein, and amino acid residues 63-148 represent the mature HB-EGF peptide. Amino acid residues 20-62 represent the N-terminal propeptide sequence, amino acid residues 149-208 represent the C-terminal propeptide sequence, amino acid residues 37, 38, 44, 47, 75, and 85 can be glycosylated, and amino acid pairs 108 and 121, 116 and 132, and 134 and 143, can form disulfide bonds. The nucleotide and amino acid sequences of mouse HB-EGF are publicly available under NM_010415.2 and NP_034545.1. For mouse HB-EGF, amino acid residues 1-23 represent the signal peptide, amino acid residues 24-208 represent the HB-EGF proprotein, and amino acid residues 63-148 represent the mature HB-EGF peptide. Amino acid residues 24-62 represent the N-terminal propeptide sequence, amino acid residues 149-208 represent the C-terminal propeptide sequence, amino acid residue 85 can be glycosylated, and amino acid pairs 108 and 121, 116 and 132, and 134 and 143, can form disulfide bonds. Nucleic acid and polypeptide sequences of HB-EGF orthologs in species other than mice and humans are well known and include, for example, chimpanzee HB-EGF (XM_001137119.2 and XP_001137119.1), monkey HB-EGF (XM_001085848.2 and XP_001085848.1), dog HB-EGF (XM_843521.2 and XP_848614.1), cow HB-EGF (NM_001144090.1 and NP_001137562.1), rat HB-EGF (NM_012945.1 and NP_037077.1), and chicken HB-EGF (NM204849.1 and NP_990180.1).

At least seven splice variants encoding seven distinct human epithelial mitogen homolog (EPGN) isoforms exist. Human EGF transcript variant 1 (NM_001270989) encodes the canonical EPGN isoform 1 (NP_001257918.1). For human EPGN isoform 1, amino acid residues 1-22 represent the signal peptide, amino acid residues 23-154 represent the EPGN proprotein, and amino acid residues 24-104 represent the mature EPGN peptide. The human EPGN isoform 2 differs from isoform 1 in that it is missing residues 36-44 and residues 137-154 are changed from CLKLKSPYNVCSGERRPL to YEKDKI. The human EPGN isoform 3 differs from isoform 1 in that it is missing residues 96-137. The human EPGN isoform 4 differs from isoform 1 in that it is missing residues 87-137. The human EPGN isoform 5 differs from isoform 1 in that it is missing residues 36-44 and 96-137. The human EPGN isoform 6 differs from isoform 1 in that it is missing residues 36-44 and 87-137. Finally, the human EPGFN isoform 7 differs from isoform 7 in that it is missing residues 15-44 and 87-137. The nucleotide and amino acid sequences of mouse EPGN are publicly available under NM_053087.2 and NP_444317.1, respectively. For mouse EPGN, amino acid residues 1-18 represent the signal peptide and amino acid residues 19-152 represent the EPGN proprotein. Nucleic acid and polypeptide sequences of EPGN orthologs in species other than mice and humans are well known and include, for example, monkey EPGN (XM_001102155.2, XP_001102155.1, XM_001102246.2, XP_001102246.1, XM_002804084.1, XP_002804130.1, XM_001102341.2, and XP_001102341.2), dog EPGN (XM_849660.2 and XP_854753.2), rat EPGN (XM_223280.4 and XP_223280.3), and chicken EPGN (NM_001012404.1 and NP_001012404.1).

The nucleotide and amino acid sequences of human amphiregulin (AREG) are publicly available under NM_001657.2 and NP_001648.1. Human AREG is synthesized as a 252 amino acid precursor glycoprotein with 3 potential N-glycosylation sites. It comprises a 19 amino acid signal peptide (amino acid residues 1-19), followed by an 81 amino acid N-terminal propeptide domain (amino acid residues 20-100), followed by an 84 amino acid mature amphiregulin peptide (amino acid residues 101-184), followed by a 23 amino acid transmembrane domain, followed by a 31 amino acid cytoplasmic propeptide domain at the C-terminus. The nucleotide and amino acid sequences of mouse AREG are publicly available under NM_009704.3 and NP_0333834.1. For mouse AREG, amino acid residues 1-26 represent the signal peptide and amino acid residues 27-248 represent the AREG proprotein, amino acid residues 100-248 represent the mature AREG peptide, amino acid residues 192-215 represent a transmembrane domain, amino acid residues 106 and 241 can be glycosylated, and amino acid pairs 139 and 152, 147 and 163, and 165 and 174, can form disulfide bonds. Nucleic acid and polypeptide sequences of AREG orthologs in species other than mice and humans are well known and include, for example, chimpanzee AREG (XM_517224.4 and XP_517224.2) and rat AREG (NM_017123.1 and NP_058819.1).

The nucleotide and amino acid sequences of human betacellulin (BTC) are publicly available under NM_001729.2 and NP_001720.1. For human BTC, amino acid residues 1-31 represent the signal peptide, amino acid residues 32-178 represent the BTC propeptide, amino acid residues 32-111 represent the mature BTC peptide, amino acid residue 34 can be glycosylated, and amino acid pairs 69 and 82, 77 and 93, and 95 and 104, can form disulfide bonds. The nucleotide and amino acid sequences of mouse BTC are publicly available under NM_007568.5 and NP_031594.1. For mouse BTC, amino acid residues 1-31 represent the signal peptide, amino acid residues 32-177 represent the BTC proprotein, amino acid residues 32-111 represent the mature BTC peptide, amino acid residues 34, 42, and 52 can be glycosylated, and amino acid pairs 69 and 82, 77 and 93, and 95 and 104, can form disulfide bonds. Nucleic acid and polypeptide sequences of BTC orthologs in species other than mice and humans are well known and include, for example, chimpanzee BTC (XM_517223.4 and XP_517223.2), monkey BTC (XM_001101880.2 and XP_001101880.1), cow BTC (NM_173896.2 and NP_776321.2), rat BTC (NM_022256.2 and NP_071592.1), chicken BTC (NM_001004769.1 and NP_001004769.1), and zebrafish BTC (NM_001044764.2 and NP_001038229.2).

At least four splice variants encoding two distinct human PTHrP isoforms exist. Human PTHrP transcript variant 1 (NM_198965.1) encodes the long PTHrP isoform 1 (NP_945316.1). Human transcript variant 2 (NM_002820.2) differs in the 3' coding sequence (CDS) and 3' untranslated region (UTR) compared to transcript variant 1 resulting in the shorter PTHrP isoform 2 (NP_002811.1). Human transcript variant 3 (NM_198964.1) differs in the 5' UTR, 3' CDS, and 3'UTR compared to transcript variant 1 and also encodes the same short PTHrP isoform 2 (NP_945315.1). Finally, human transcript variant 4 (NM_198966.1) differs in the 5' UTR compared to transcript variant 1 and encodes the long PTHrP isoform 1 (NP_945317.1). For human PTHrP isoform 1, amino acid residues 1-24 represent the signal peptide, amino acid residues 25-177 represent the PTHrP propeptide, amino acid residues 37-177 represent the mature PTHrP peptide, amino acid residues 37-70 represent the PTHrP [1-34] peptide, amino acid residues 74-130 represent the PTHrP [38-94] peptide, amino acid residues 143-175 represent the osteostatin peptide, amino acid residue 59 is a predicted proteolytic cleavage site, and amino acid residue 108 is a predicted phosphorylation site. For human PTHrP isoform 2, amino acid residues 1-24 represent the signal peptide, amino acid residues 25-175 represent the PTHrP propeptide, amino acid residues 37-175 represent the mature PTHrP peptide, amino acid residue 59 is a predicted proteolytic cleavage site, and amino acid residue 108 is a predicted phosphorylation site. The nucleotide and amino acid sequences of mouse PTHrP are publicly available under NM_008970.3 and NP_032996.2, respectively. For mouse PTHrP, amino acid residues 1-24 represent the signal peptide, amino acid residues 25-175 represent the PTHrP propeptide, amino acid residues 37-175 represent the mature PTHrP peptide, and amino acids residues 143-173 represent the osteostatin peptide. Nucleic acid and polypeptide sequences of PTH orthologs in species other than mice and humans are well known and include, for example, chimpanzee PTHrP (XM 528764.4, XP_528764.2, XM_001142021.1, and XP_001142021.1), monkey PTHrP (XM_001104176.1, XP_001104176.1, XM_001104259.1, XP_001104259.1, XM 001104338.1, and XP_001104338.1), dog PTHrP (NM_001003303.1 and NP_001003303.1), cow PTHrP (NM_174753.1 and NP_777178.1), rat PTHrP (NM_012636.1 and NP_036768.1), chicken PTHrP (NM_001174106.1, NP_001167577.1, NM_205338.2, and NP_990669.2), and zebrafish PTHrP (NM_001024627.2 and NP_001019798.2).

The nucleotide and amino acid sequences of human PTH are publicly available under Genbank Accession number NM_00315.2 and NP_00306.1, respectively. Amino acid residues 1-25 represent the signal peptide, amino acid residues 26-115 represent the PTH proprotein, amino acid residues 32-115 represent the mature PTH peptide, and amino acid residues 51-69 are important for receptor binding. The nucleotide and amino acid sequences of mouse PTH are publicly available under Genbank Accession number NM_020623.2 and NP_065648.1, respectively, wherein amino acid residues 1-25 of the protein represent the signal peptide, amino acid residues 26-115 represent the PTH proprotein, and amino acid residues 32-115 represent the mature PTH peptide. Nucleic acid and polypeptide sequences of PTH orthologs in species other than mice and humans are well known and include, for example, chimpanzee PTH (XM_003951841.1, XP_003951890.1, XM_001171819.2, and XP_001171819.1), monkey PTH (XM_001094024.2 and XP_001094024.1), dog PTH (NM_001003302.1 and NP_001003302.1), cow PTH (NM_173954.2 and NP_776379.2), rat PTH (NM_017044.1 and NP_058740.1), and chicken PTH (NM_205452.2 and NP_990783.1).

In some embodiments, fragments of the PTH-related or EGF-related molecules described herein having one or more biological activities of the full-length protein can be used, such as the mature peptide or propeptide. In some embodiments, such fragments can comprise or consist of a signal peptide, extracellular, N-terminal propeptide, C-terminal propeptide, complete propeptide, EGF-like, transmembrane, and/or C-terminal domains of the full-length protein without containing the full-length protein sequence. Nucleic acid and amino acid sequences are provided below in Table 1.

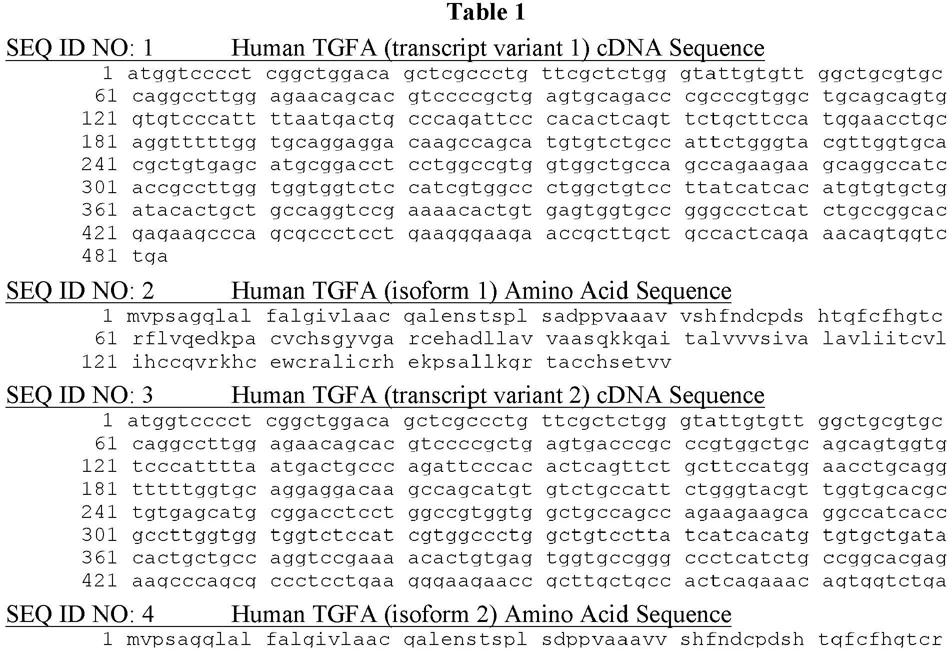

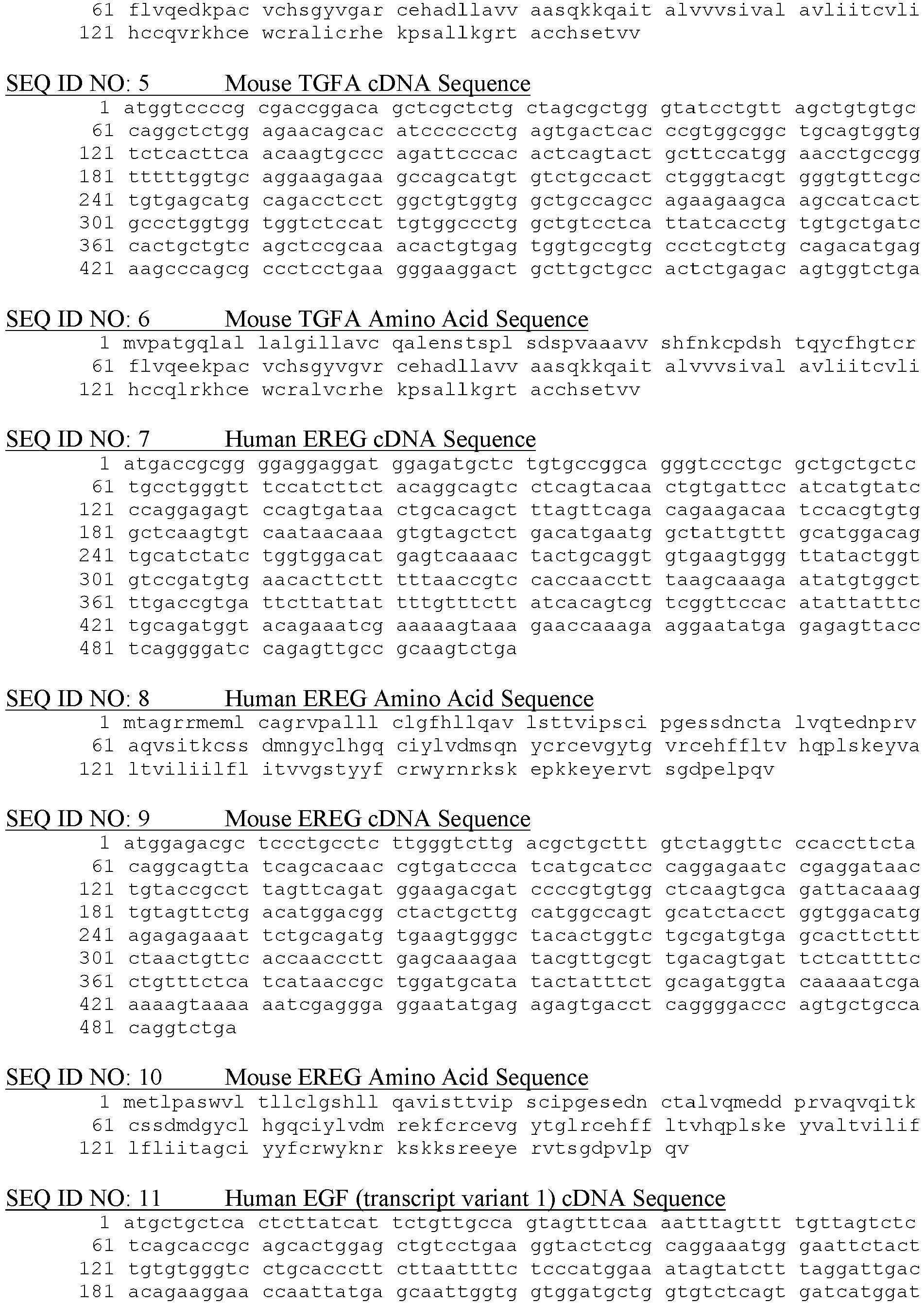

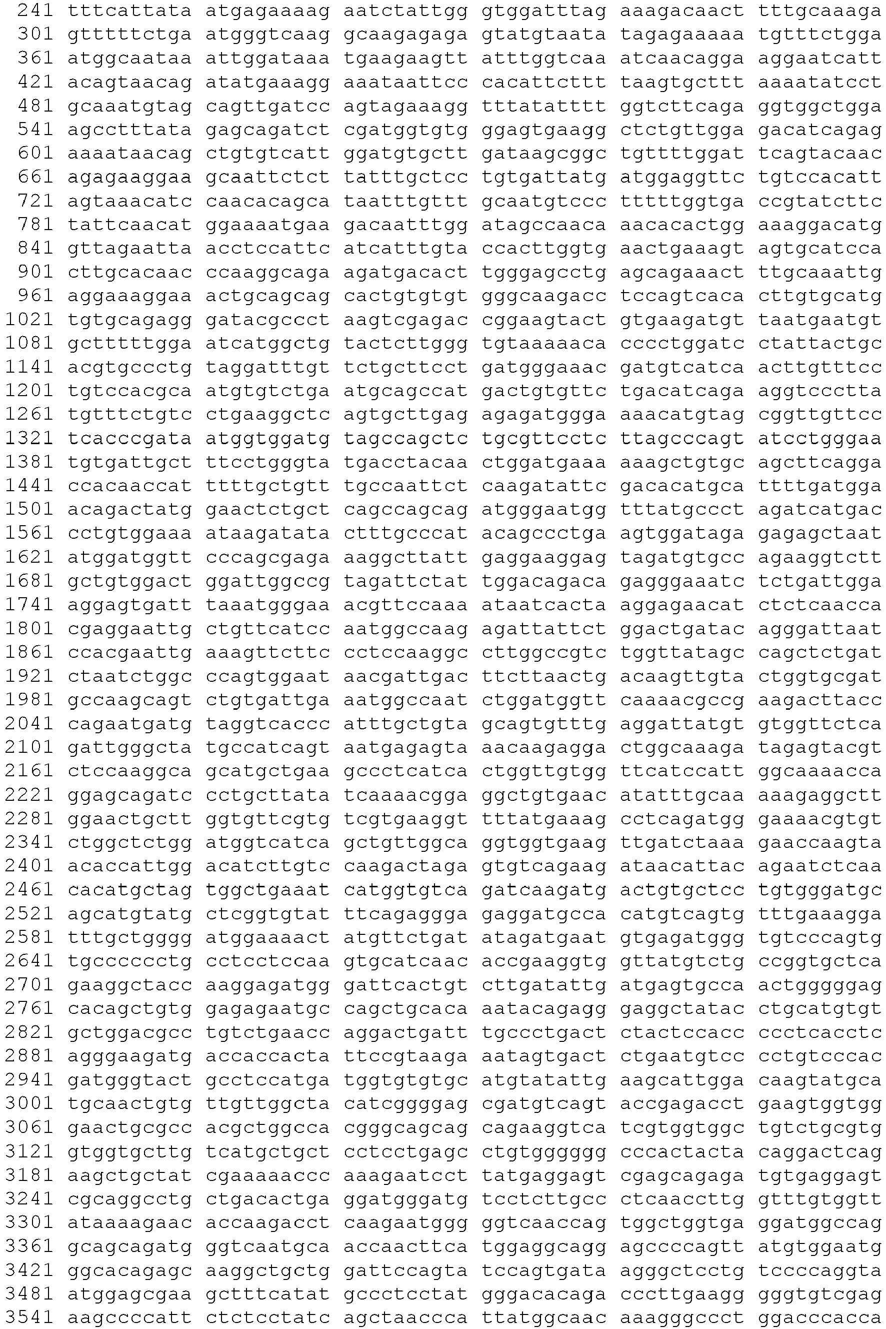

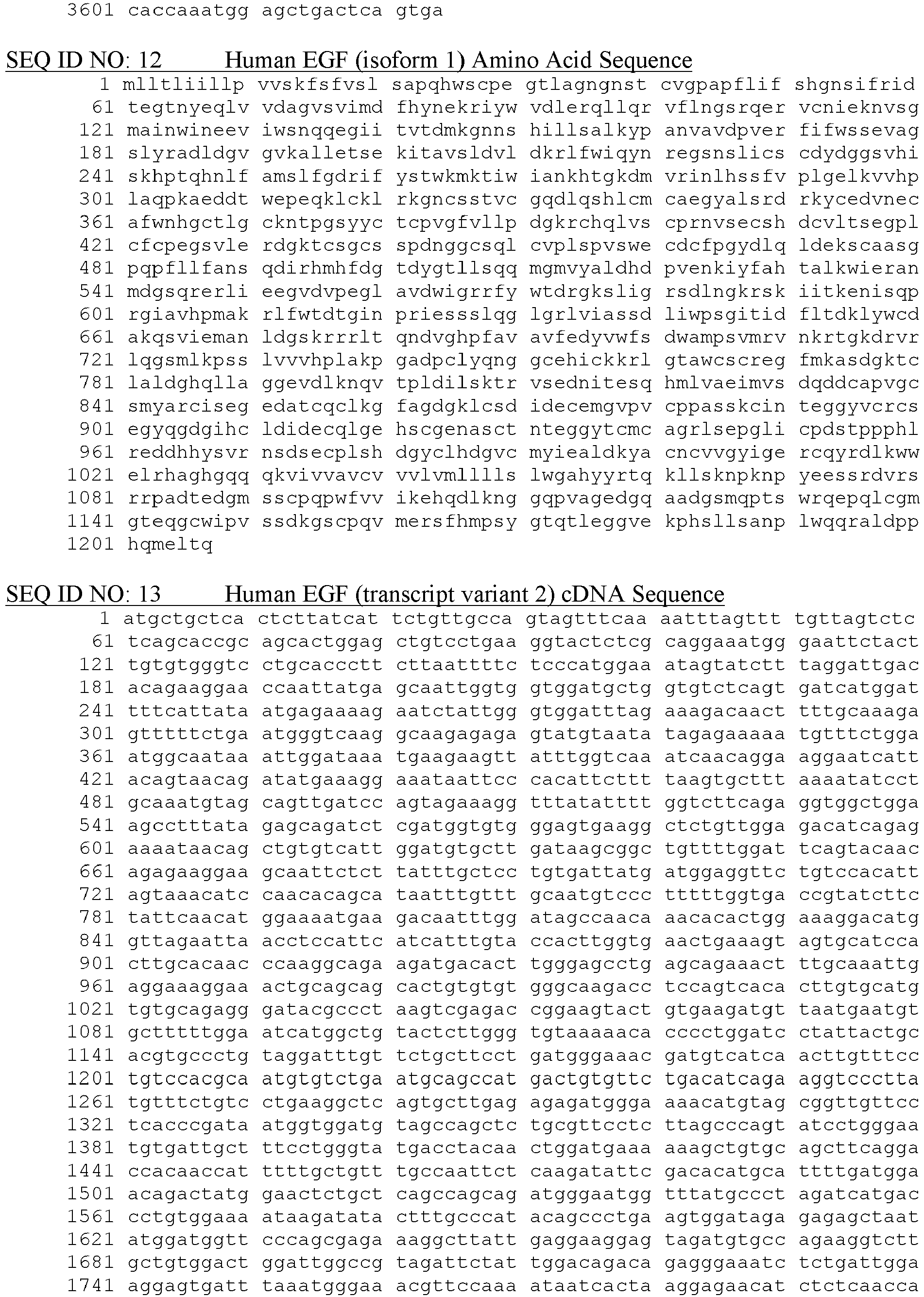

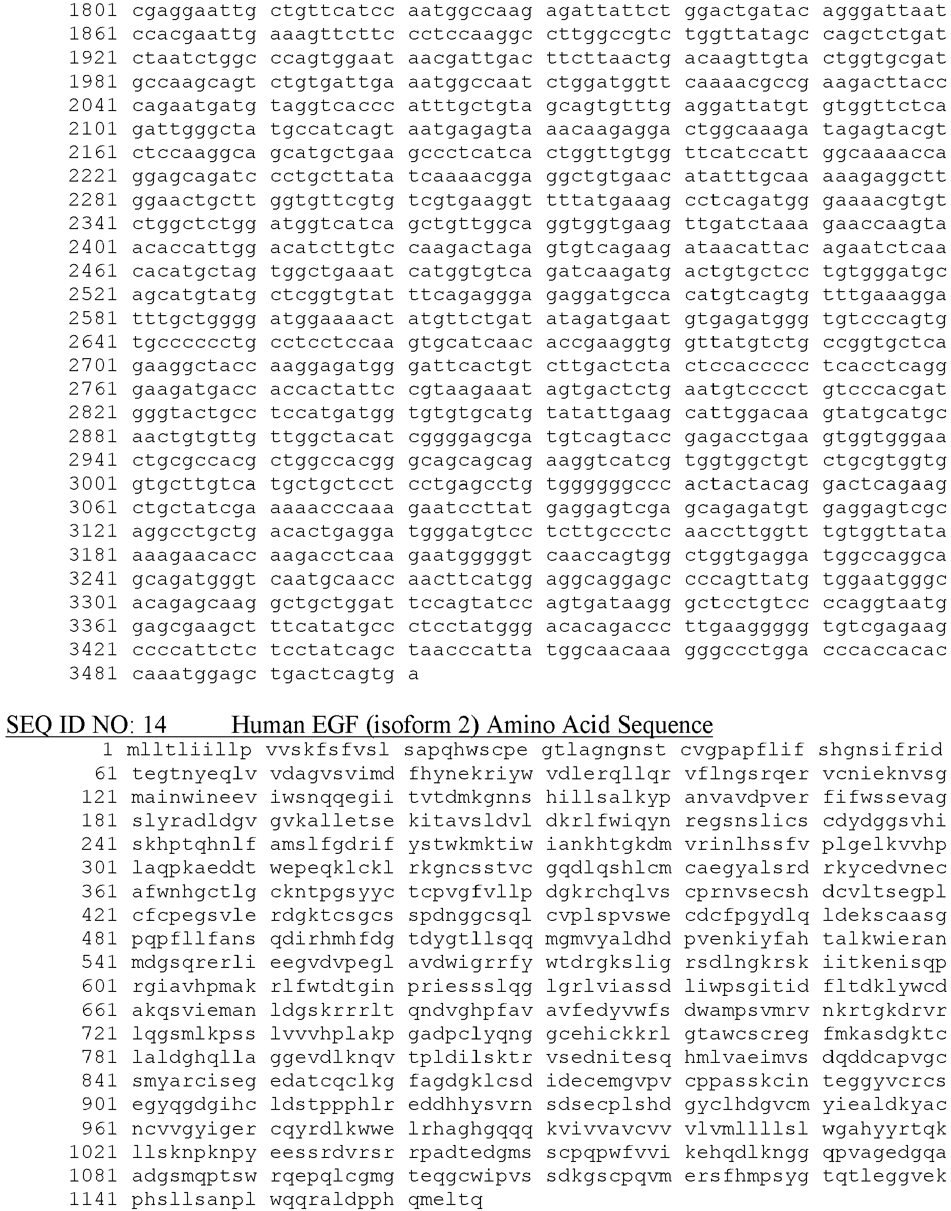

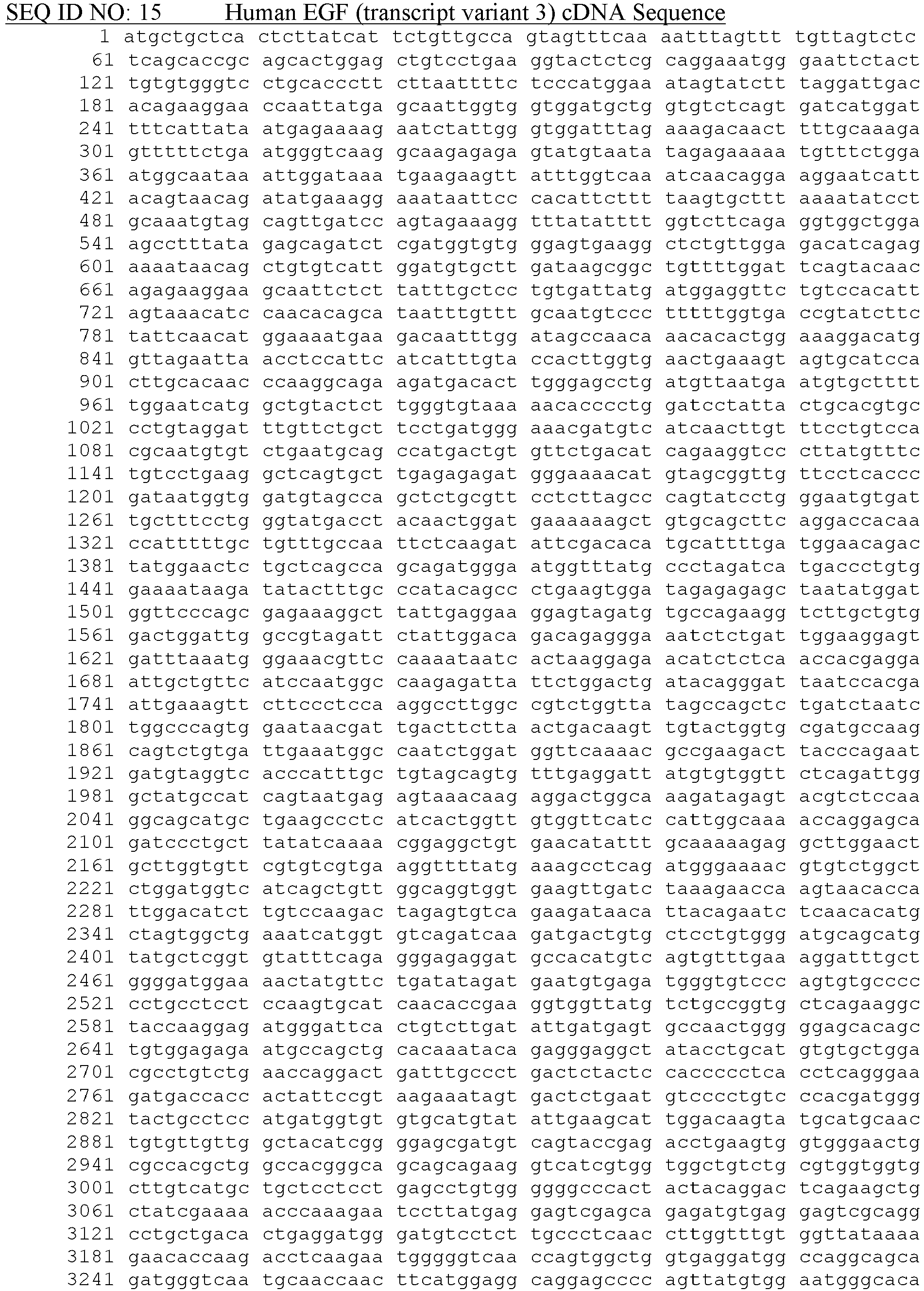

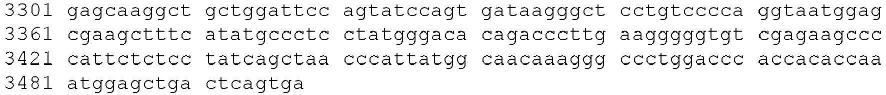

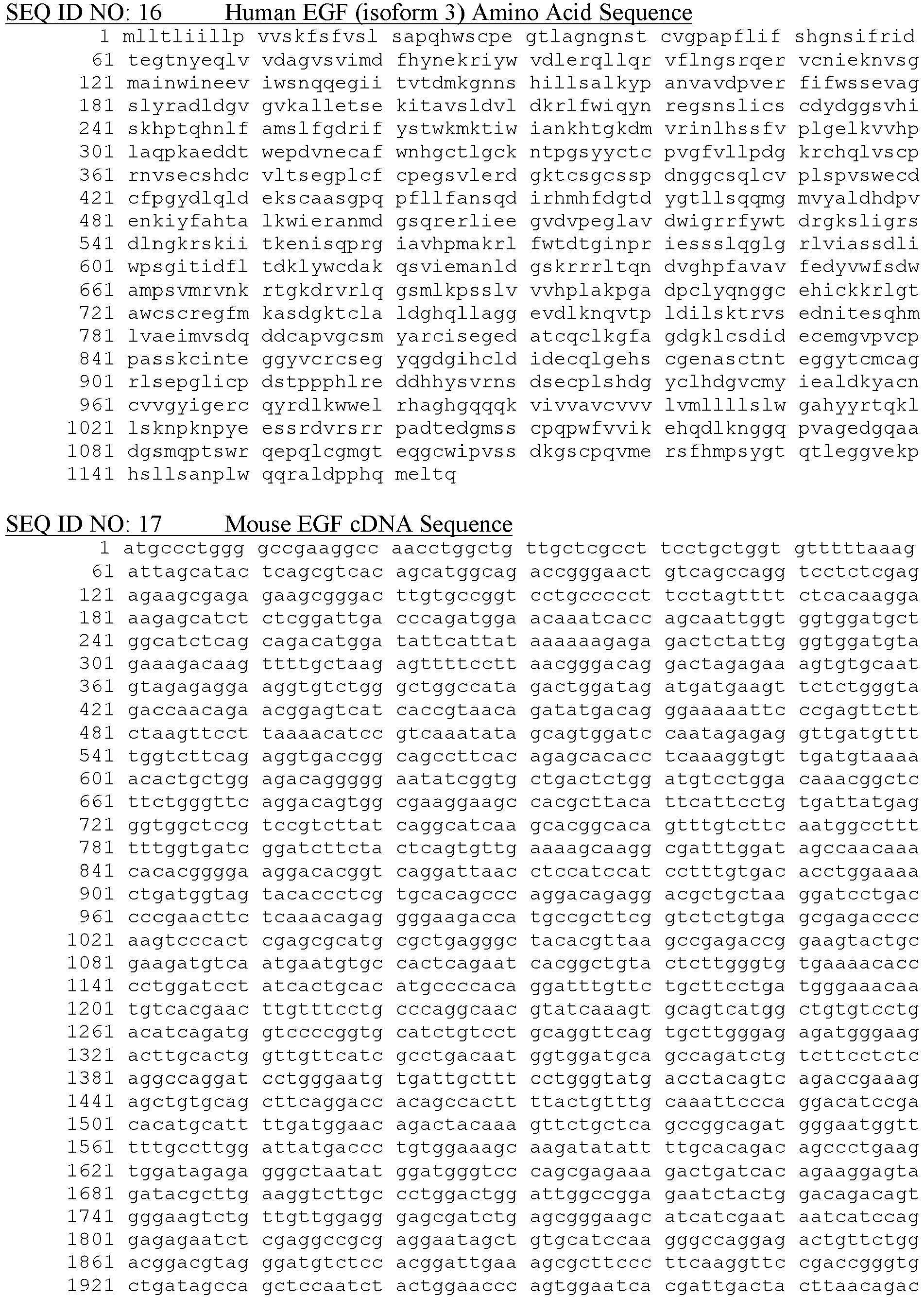

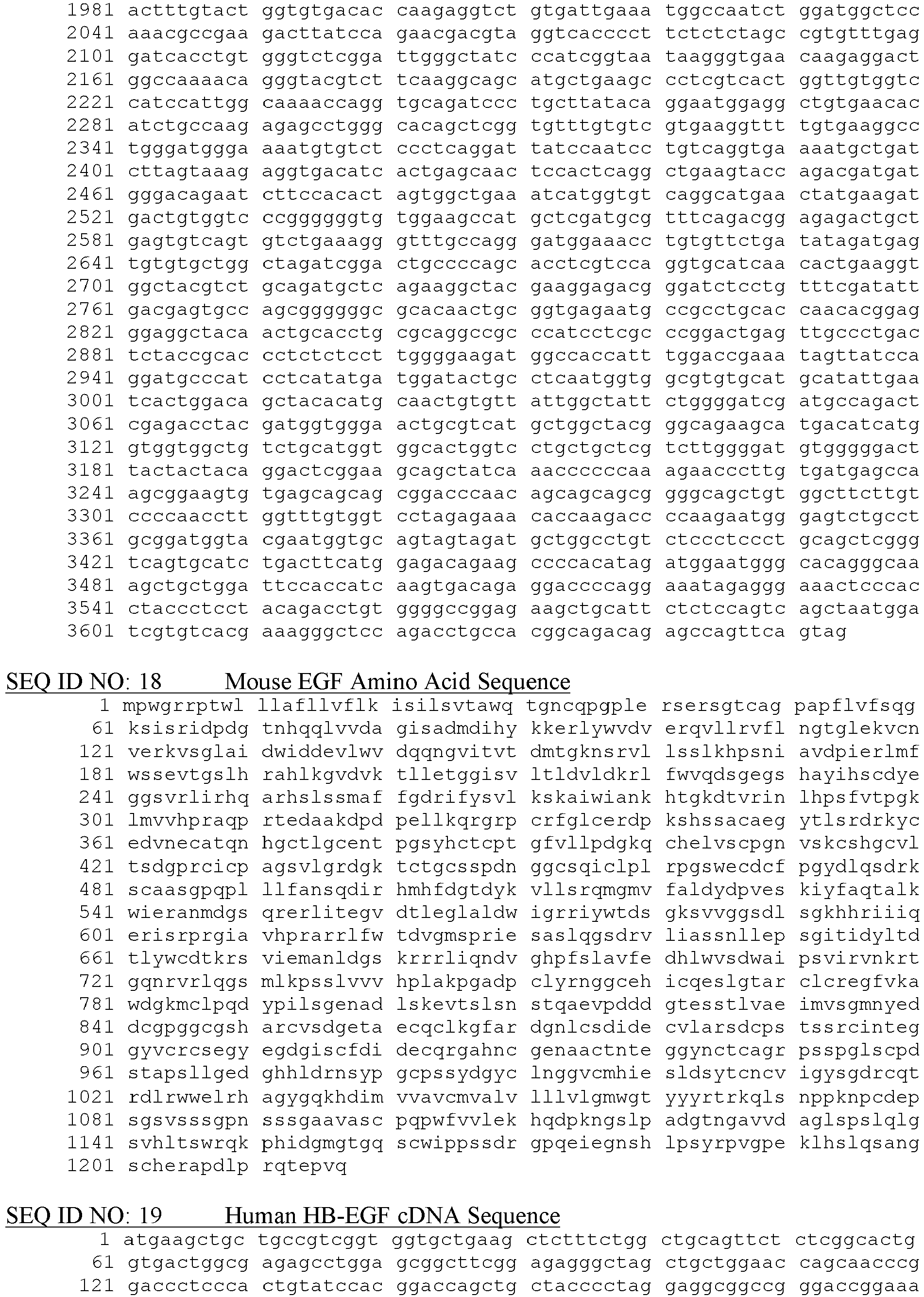

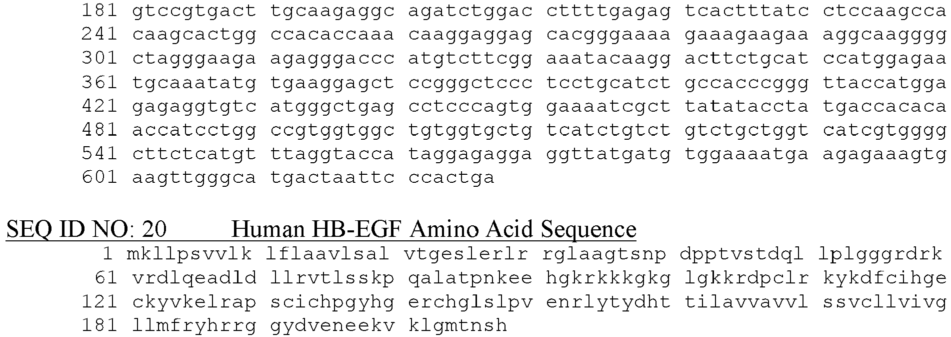

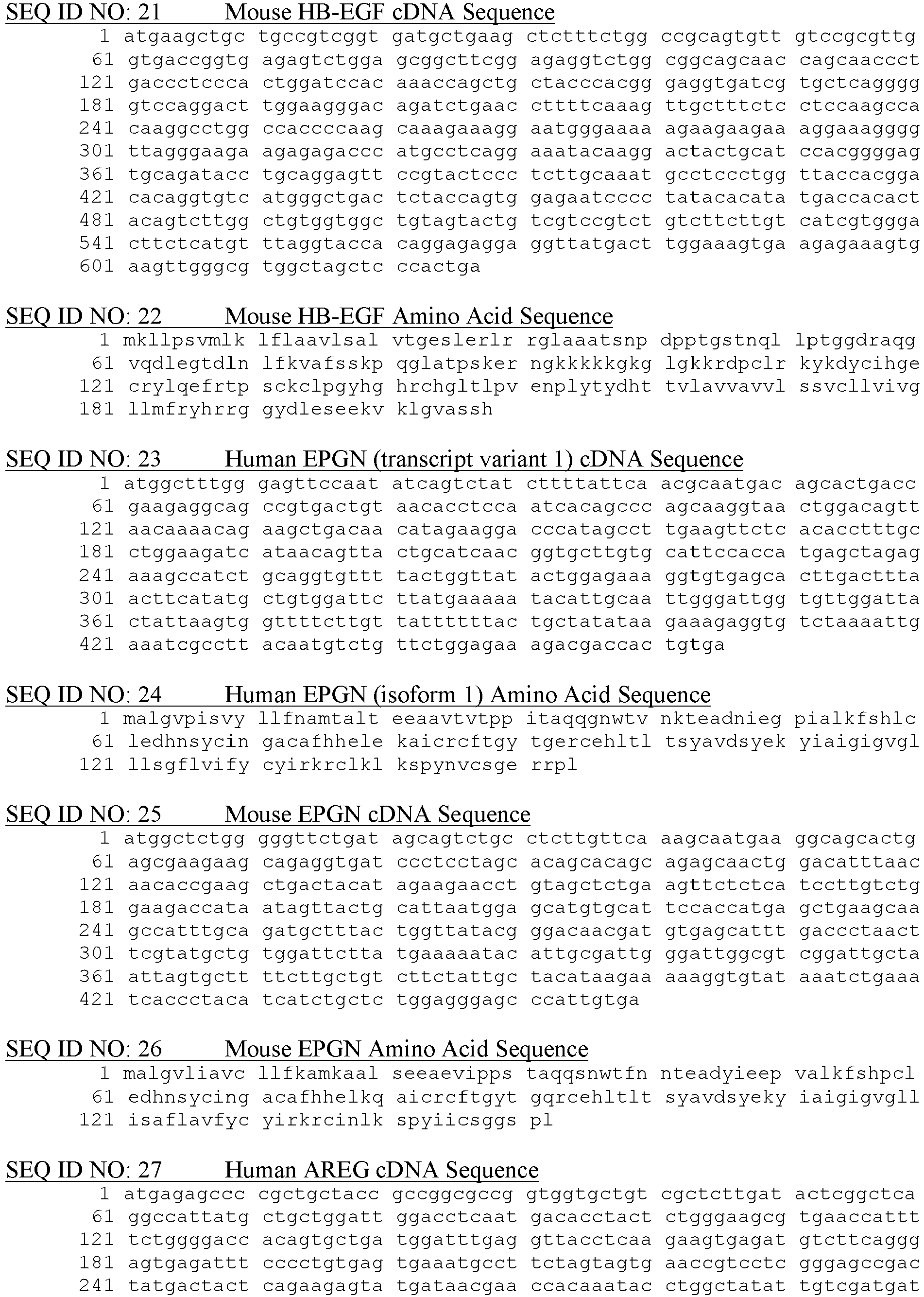

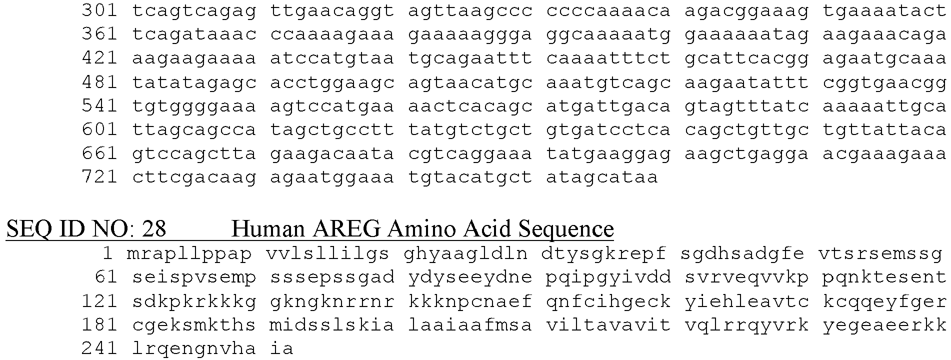

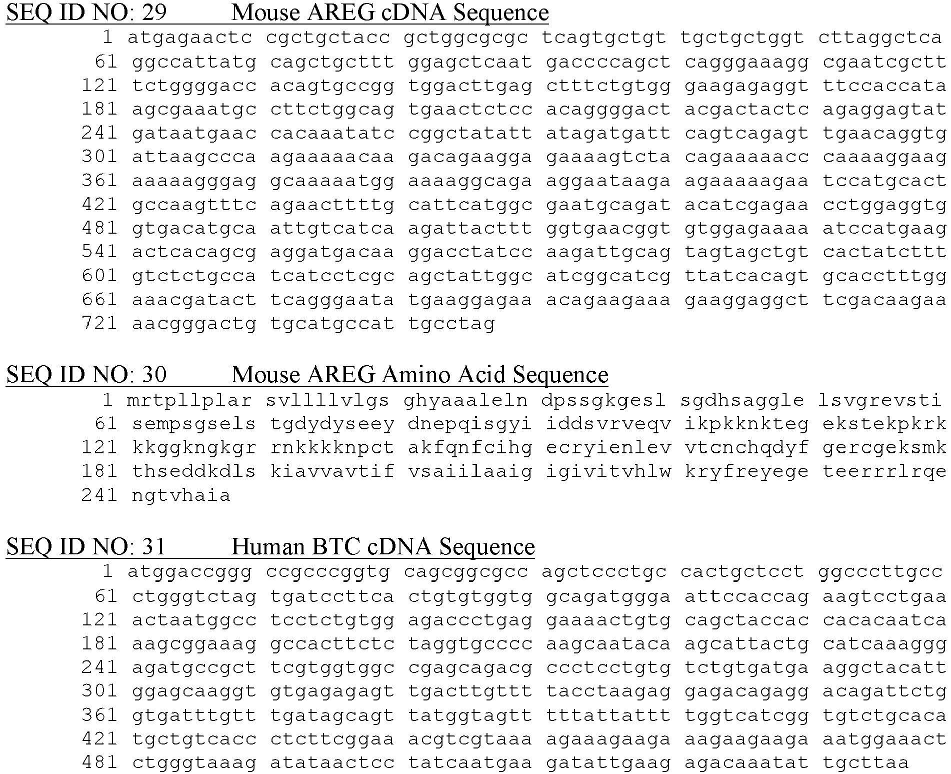

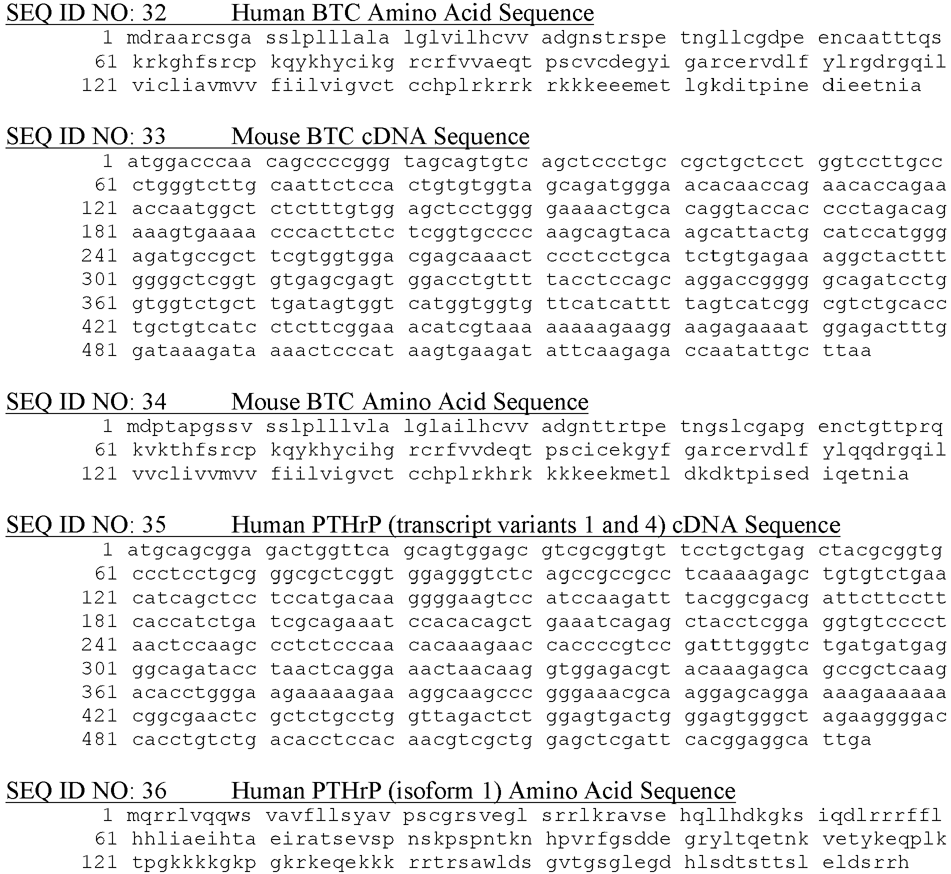

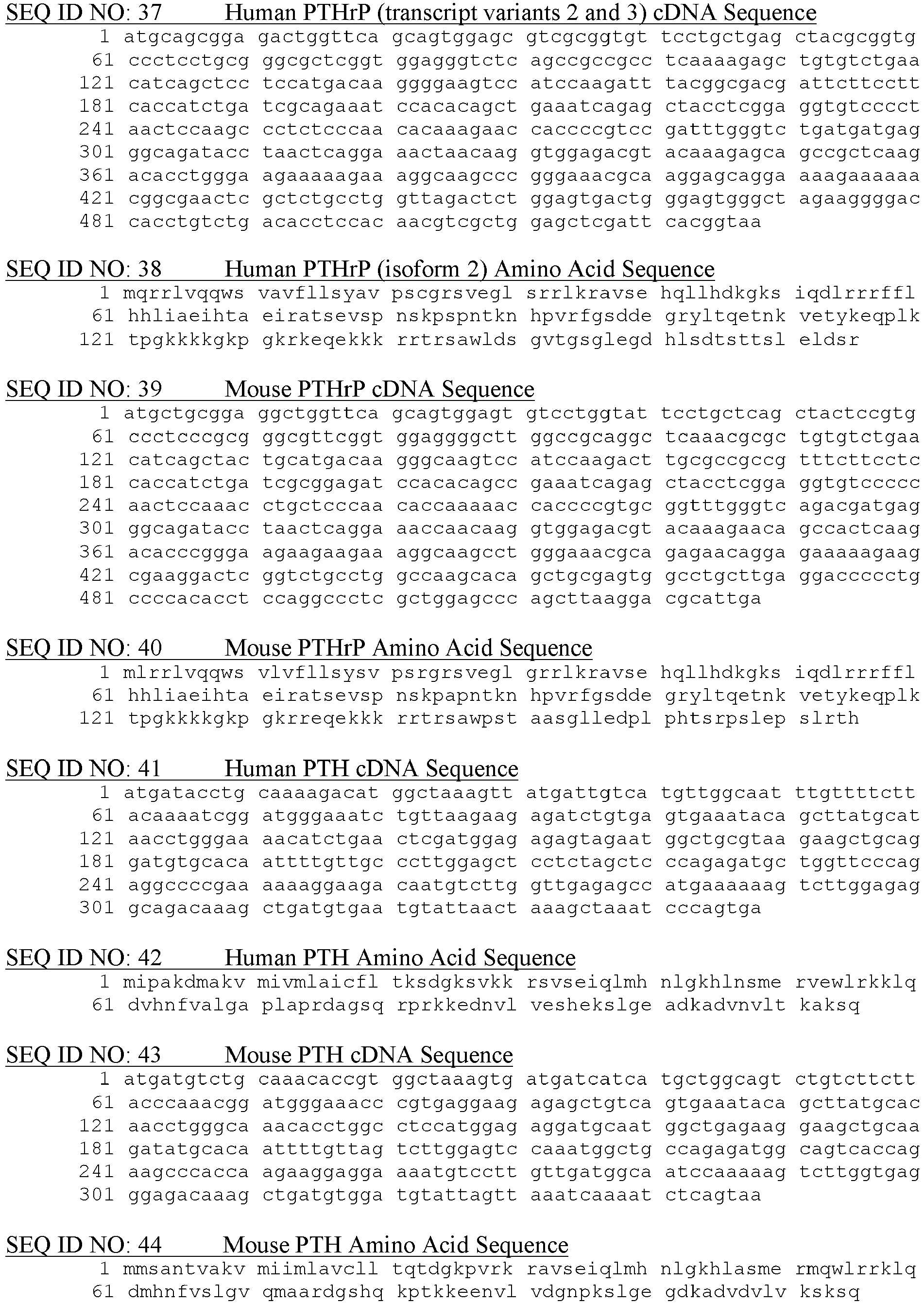

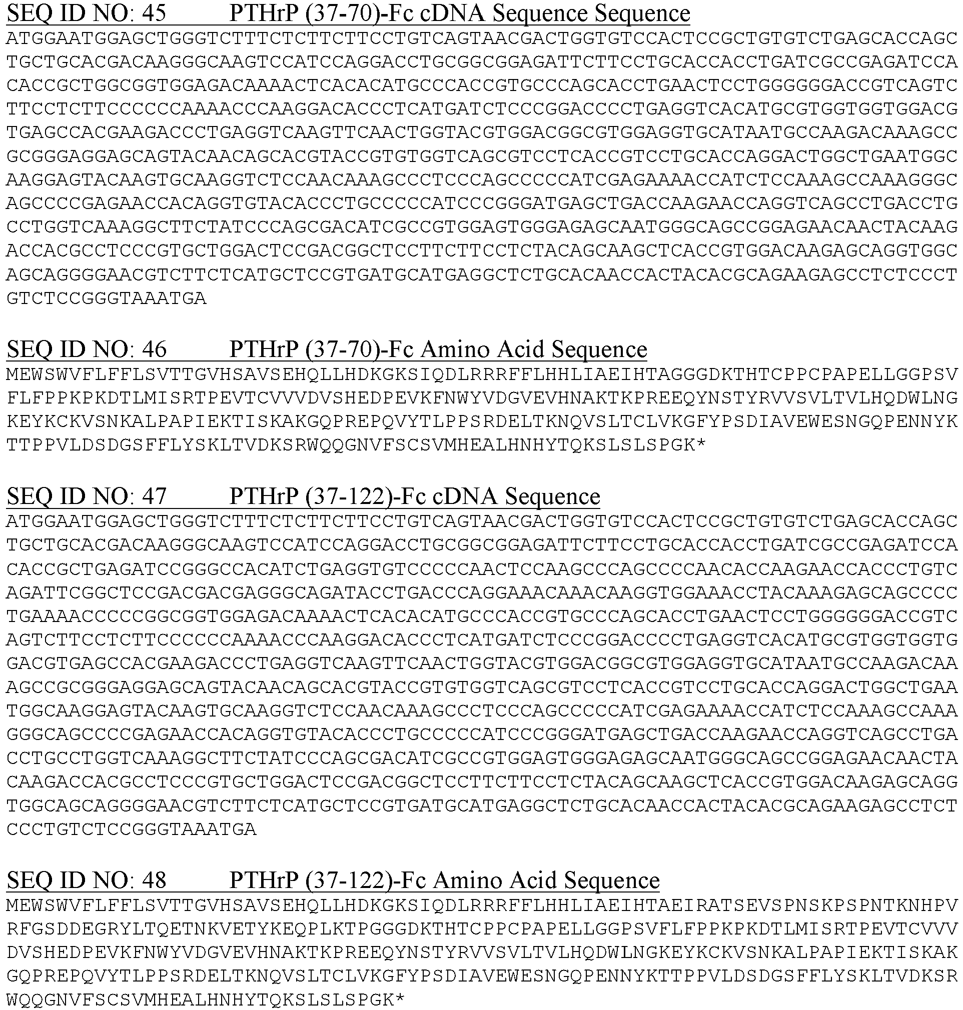

It will be appreciated that specific sequence identifiers (SEQ ID NOs) have been referenced throughout the specification for purposes of illustration and should therefore not be construed to be limiting. Any marker of the present invention, including, but not limited to, the markers described in the specification and markers described herein (e.g., cidea, adiponectin (adipoq), adipsin, otopetrin, type II deiodinase, cig30, ppar gamma 2, pgc1α, ucp1, elovl3, cAMP, Prdm16, cytochrome C, cox4i1, coxIII, cox5b, cox7a1, cox8b, glut4, atpase b2, cox II, atp5o, ndufb5, ap2, ndufs1, GRP109A, acylCoA-thioesterase 4, EARA1, claudin1, PEPCK, fgf21, acylCoA-thioesterase 3, and dio2), are well known in the art and can be used in the embodiments of the invention.

### II. Agents and Compositions

Agents and compositions are provided herein and can be used for the diagnosis, prognosis, prevention, and treatment of metabolic disorders. Such agents and compositions can detect and/or modulate, *e.g.,* up- or down-regulate, expression and/or activity of gene products or fragments thereof encoded by biomarkers of the invention, including the biomarkers listed in Table 1 and the Examples, in order to modulate metabolic responses and/or metabolic disorders. Exemplary agents include antibodies, small molecules, peptides, peptidomimetics, natural ligands, and derivatives of natural ligands, that can either bind and/or activate or inhibit protein biomarkers of the invention, including the biomarkers listed in Table 1 and the Examples, or fragments thereof; RNA interference, antisense, nucleic acid aptamers, etc. that can modulate the expression and/or activity of the biomarkers of the invention, including the biomarkers listed in Table 1 and the Examples, or fragments thereof.

In one embodiment, of the disclosure isolated nucleic acid molecules that specifically hybridize with or encode one or more biomarkers listed in Table 1 and the Examples or biologically active portions thereof are presented. As used herein, the term "nucleic acid molecule" is intended to include DNA molecules *(i.e.,* cDNA or genomic DNA) and RNA molecules *(i.e.,* mRNA) and analogs of the DNA or RNA generated using nucleotide analogs. The nucleic acid molecule can be single-stranded or double-stranded, but preferably is double-stranded DNA. An "isolated" nucleic acid molecule is one which is separated from other nucleic acid molecules which are present in the natural source of the nucleic acid. Preferably, an "isolated" nucleic acid is free of sequences which naturally flank the nucleic acid (*i.e*., sequences located at the 5' and 3' ends of the nucleic acid) in the genomic DNA of the organism from which the nucleic acid is derived. For example, in various embodiments, the isolated nucleic acid molecules corresponding to the one or more biomarkers listed in Table 1 and the Examples can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or chemical precursors or other chemicals when chemically synthesized.

A nucleic acid molecule of the present invention, *e.g.,* a nucleic acid molecule having the nucleotide sequence of one or more biomarkers listed in Table 1 and the Examples or a nucleotide sequence which is at least about 50%, preferably at least about 60%, more preferably at least about 70%, yet more preferably at least about 80%, still more preferably at least about 90%, and most preferably at least about 95% or more *(e.g.,* about 98%) homologous to the nucleotide sequence of one or more biomarkers listed in Table 1 and the Examples or a portion thereof *(i.e.,* 100, 200, 300, 400, 450, 500, or more nucleotides), can be isolated using standard molecular biology techniques and the sequence information provided herein. For example, a human cDNA can be isolated from a human cell line (from Stratagene, La Jolla, CA, or Clontech, Palo Alto, CA) using all or portion of the nucleic acid molecule, or fragment thereof, as a hybridization probe and standard hybridization techniques *(i.e.,* as described in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989). Moreover, a nucleic acid molecule encompassing all or a portion of the nucleotide sequence of one or more biomarkers listed in Table 1 and the Examples or a nucleotide sequence which is at least about 50%, preferably at least about 60%, more preferably at least about 70%, yet more preferably at least about 80%, still more preferably at least about 90%, and most preferably at least about 95% or more homologous to the nucleotide sequence, or fragment thereof, can be isolated by the polymerase chain reaction using oligonucleotide primers designed based upon the sequence of the one or more biomarkers listed in Table 1 and the Examples, or fragment thereof, or the homologous nucleotide sequence. For example, mRNA can be isolated from muscle cells *(i.e.,* by the guanidinium-thiocyanate extraction procedure of Chirgwin et al. (1979) Biochemistry 18: 5294-5299) and cDNA can be prepared using reverse transcriptase *(i.e.,* Moloney MLV reverse transcriptase, available from Gibco/BRL, Bethesda, MD; or AMV reverse transcriptase, available from Seikagaku America, Inc., St. Petersburg, FL). Synthetic oligonucleotide primers for PCR amplification can be designed according to well-known methods in the art. A nucleic acid of the invention can be amplified using cDNA or, alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to the nucleotide sequence of one or more biomarkers listed in Table 1 and the Examples can be prepared by standard synthetic techniques, *i.e.,* using an automated DNA synthesizer.

Probes based on the nucleotide sequences of one or more biomarkers listed in Table 1 and the Examples can be used to detect transcripts or genomic sequences encoding the same or homologous proteins. In preferred embodiments, the probe further comprises a label group attached thereto, *i.e.,* the label group can be a radioisotope, a fluorescent compound, an enzyme, or an enzyme co-factor. Such probes can be used as a part of a diagnostic test kit for identifying cells or tissue which express one or more biomarkers listed in Table 1 and the Examples, such as by measuring a level of nucleic acid in a sample of cells from a subject, *i.e.,* detecting mRNA levels of one or more biomarkers listed in Table 1 and the Examples.

Nucleic acid molecules encoding proteins corresponding to one or more biomarkers listed in Table 1 and the Examples from different species are also contemplated. For example, rat or monkey cDNA can be identified based on the nucleotide sequence of a human and/or mouse sequence and such sequences are well known in the art. In one embodiment, the nucleic acid molecule(s) of the invention encodes a protein or portion thereof which includes an amino acid sequence which is sufficiently homologous to an amino acid sequence of one or more biomarkers listed in Table 1 and the Examples, such that the protein or portion thereof modulates *(e.g.,* enhance), one or more of the following biological activities: a) binding to the biomarker; b) modulating the copy number of the biomarker; c) modulating the expression level of the biomarker; and d) modulating the activity level of the biomarker.

As used herein, the language "sufficiently homologous" refers to proteins or portions thereof which have amino acid sequences which include a minimum number of identical or equivalent *(e.g.,* an amino acid residue which has a similar side chain as an amino acid residue in one or more biomarkers listed in Table 1 and the Examples, or fragment thereof) amino acid residues to an amino acid sequence of the biomarker, or fragment thereof, such that the protein or portion thereof modulates *(e.g.,* enhance) one or more of the following biological activities: a) binding to the biomarker; b) modulating the copy number of the biomarker; c) modulating the expression level of the biomarker; and d) modulating the activity level of the biomarker. In some embodiments, the protein or portion thereof modulates *(e.g.,* enhance), one or more of the following biological activities: 1) it can modulate the expression of one or more of cidea, adiponectin (adipoq), adipsin, otopetrin, type II deiodinase, cig30, ppar gamma 2, pgc1α, ucp1, elovl3, cAMP, Prdm16, cytochrome C, cox4i1, coxIII, cox5b, cox7a1, cox8b, glut4, atpase b2, cox II, atp5o, ndufb5, ap2, ndufs1, GRP109A, acylCoA-thioesterase 4, EARA1, claudin1, PEPCK, fgf21, acylCoA-thioesterase 3, and dio; 2) it can increase cellular respiration *(i.e.,* basal, total, and/or uncoupled respiration); 3) it can increase thermogenesis of adipose cells; 4) it can increase insulin sensitivity of adipose, muscle and/or hepatic cells; 5) it can decrease hepatosteatosis, obesity, type II diabetes, and/or appetite; 6) it can increase insulin secretion of pancreatic beta cells; 7) it can increase cardiac function to combat cardiac hypertrophy; 8) it can improve muscle hypoplasia; 9) it can reduce the growth and effects of obesity-associated cancer, cachexia, and anorexia; 10) it can increase the differentiation, generation, and/or proliferation of adipose cells, 11) it can decrease blood glucose levels, and 12) it can treat diseases or disorders characterized by increased PGC-1 expression or activity, *e.g.,* diabetes or obesity.

In another embodiment, of the disclosure the protein is at least about 50%, preferably at least about 60%, more preferably at least about 70%, 75%, 80%, 85%, 90%, 91%, 92%, 93%, 94%, 95%, 96%, 97%, 98%, 99% or more homologous to the entire amino acid sequence of the biomarker, or a fragment thereof.

Portions of proteins encoded by nucleic acid molecules of the one or more biomarkers listed in Table 1 and the Examples are preferably biologically active portions of the protein. As used herein, the term "biologically active portion" of one or more biomarkers listed in Table 1 and the Examples is intended to include a portion, *e.g.,* a domain/motif, that has one or more of the biological activities of the full-length protein.

Standard binding assays, *e.g.,* immunoprecipitations and yeast two-hybrid assays, as described herein, or functional assays, *e.g.,* RNAi or overexpression experiments, can be performed to determine the ability of the protein or a biologically active fragment thereof to maintain a biological activity of the full-length protein.

The disclosure further encompasses nucleic acid molecules that differ from the nucleotide sequence of the one or more biomarkers listed in Table 1 and the Examples, or fragment thereof due to degeneracy of the genetic code and thus encode the same protein as that encoded by the nucleotide sequence, or fragment thereof. In another embodiment, an isolated nucleic acid molecule of the invention has a nucleotide sequence encoding a protein having an amino acid sequence of one or more biomarkers listed in Table 1 and the Examples, or fragment thereof, or a protein having an amino acid sequence which is at least about 70%, 75%, 80%, 85%, 90%, 91%, 92%, 93%, 94%, 95%, 96%, 97%, 98%, 99% or more homologous to the amino acid sequence of the one or more biomarkers listed in Table 1 and the Examples, or fragment thereof. In another embodiment, a nucleic acid encoding a polypeptide consists of nucleic acid sequence encoding a portion of a full-length fragment of interest that is less than 195, 190, 185, 180, 175, 170, 165, 160, 155, 150, 145, 140, 135, 130, 125, 120, 115, 110, 105, 100, 95, 90, 85, 80, 75, or 70 amino acids in length.

It will be appreciated by those skilled in the art that DNA sequence polymorphisms that lead to changes in the amino acid sequences of the one or more biomarkers listed in Table 1 and the Examples may exist within a population *(e.g.,* a mammalian and/or human population). Such genetic polymorphisms may exist among individuals within a population due to natural allelic variation. As used herein, the terms "gene" and "recombinant gene" refer to nucleic acid molecules comprising an open reading frame encoding one or more biomarkers listed in Table 1 and the Examples, preferably a mammalian, *e.g.,* human, protein. Such natural allelic variations can typically result in 1-5% variance in the nucleotide sequence of the one or more biomarkers listed in Table 1 and the Examples. Any and all such nucleotide variations and resulting amino acid polymorphisms in the one or more biomarkers listed in Table 1 and the Examples that are the result of natural allelic variation and that do not alter the functional activity of the one or more biomarkers listed in Table 1 and the Examples are intended to be within the scope of the invention. Moreover, nucleic acid molecules encoding one or more biomarkers listed in Table 1 and the Examples from other species.

In addition to naturally-occurring allelic variants of the one or more biomarkers listed in Table 1 and the Examples that may exist in the population, the skilled artisan will further appreciate that changes can be introduced by mutation into the nucleotide sequence, or fragment thereof, thereby leading to changes in the amino acid sequence of the encoded one or more biomarkers listed in Table 1 and the Examples, without altering the functional ability of the one or more biomarkers listed in Table 1 and the Examples. For example, nucleotide substitutions leading to amino acid substitutions at "non-essential" amino acid residues can be made in the sequence, or fragment thereof. A "non-essential" amino acid residue is a residue that can be altered from the wild-type sequence of the one or more biomarkers listed in Table 1 and the Examples without altering the activity of the one or more biomarkers listed in Table 1 and the Examples, whereas an "essential" amino acid residue is required for the activity of the one or more biomarkers listed in Table 1 and the Examples. Other amino acid residues, however, *(e.g.,* those that are not conserved or only semi-conserved between mouse and human) may not be essential for activity and thus are likely to be amenable to alteration without altering the activity of the one or more biomarkers listed in Table 1 and the Examples.

The term "sequence identity or homology" refers to the sequence similarity between two polypeptide molecules or between two nucleic acid molecules. When a position in both of the two compared sequences is occupied by the same base or amino acid monomer subunit, *e.g.,* if a position in each of two DNA molecules is occupied by adenine, then the molecules are homologous or sequence identical at that position. The percent of homology or sequence identity between two sequences is a function of the number of matching or homologous identical positions shared by the two sequences divided by the number of positions compared x 100. For example, if 6 of 10, of the positions in two sequences are the same then the two sequences are 60% homologous or have 60% sequence identity. By way of example, the DNA sequences ATTGCC and TATGGC share 50% homology or sequence identity. Generally, a comparison is made when two sequences are aligned to give maximum homology. Unless otherwise specified "loop out regions", *e.g.,* those arising from, from deletions or insertions in one of the sequences are counted as mismatches.

The comparison of sequences and determination of percent homology between two sequences can be accomplished using a mathematical algorithm. Preferably, the alignment can be performed using the Clustal Method. Multiple alignment parameters include GAP Penalty =10, Gap Length Penalty = 10. For DNA alignments, the pairwise alignment parameters can be Htuple=2, Gap penalty=5, Window=4, and Diagonal saved=4. For protein alignments, the pairwise alignment parameters can be Ktuple=1, Gap penalty=3, Window=5, and Diagonals Saved=5.

In a preferred embodiment, the percent identity between two amino acid sequences is determined using the Needleman and Wunsch (J. Mol. Biol. (48):444-453 (1970)) algorithm which has been incorporated into the GAP program in the GCG software package (available online), using either a Blossom 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available online), using a NWSgapdna.CMP matrix and a gap weight of 40, 50, 60, 70, or 80 and a length weight of 1, 2, 3, 4, 5, or 6. In another embodiment, the percent identity between two amino acid or nucleotide sequences is determined using the algorithm of E. Meyers and W. Miller (CABIOS, 4:11-17 (1989)) which has been incorporated into the ALIGN program (version 2.0) (available online), using a PAM120 weight residue table, a gap length penalty of 12 and a gap penalty of 4.

An isolated nucleic acid molecule encoding a protein homologous to one or more biomarkers listed in Table 1 and the Examples, or fragment thereof, can be created by introducing one or more nucleotide substitutions, additions or deletions into the nucleotide sequence, or fragment thereof, or a homologous nucleotide sequence such that one or more amino acid substitutions, additions or deletions are introduced into the encoded protein. Mutations can be introduced by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains *(e.g.,* lysine, arginine, histidine), acidic side chains *(e.g.,* aspartic acid, glutamic acid), uncharged polar side chains *(e.g.,* glycine, asparagine, glutamine, serine, threonine, tyrosine, cysteine), nonpolar side chains *(e.g.,* alanine, valine, leucine, isoleucine, proline, phenylalanine, methionine, tryptophan), branched side chains *(e.g.,* threonine, valine, isoleucine) and aromatic side chains *(e.g.,* tyrosine, phenylalanine, tryptophan, histidine). Thus, a predicted nonessential amino acid residue in one or more biomarkers listed in Table 1 and the Examples is preferably replaced with another amino acid residue from the same side chain family. Alternatively, in another embodiment, mutations can be introduced randomly along all or part of the coding sequence of the one or more biomarkers listed in Table 1 and the Examples, such as by saturation mutagenesis, and the resultant mutants can be screened for an activity described herein to identify mutants that retain desired activity. Following mutagenesis, the encoded protein can be expressed recombinantly according to well-known methods in the art and the activity of the protein can be determined using, for example, assays described herein.

The levels of one or more biomarkers listed in Table 1 and the Examples levels may be assessed by any of a wide variety of well-known methods for detecting expression of a transcribed molecule or protein. Non-limiting examples of such methods include immunological methods for detection of proteins, protein purification methods, protein function or activity assays, nucleic acid hybridization methods, nucleic acid reverse transcription methods, and nucleic acid amplification methods.

In preferred embodiments, the levels of one or more biomarkers listed in Table 1 and the Examples levels are ascertained by measuring gene transcript *(e.g.,* mRNA), by a measure of the quantity of translated protein, or by a measure of gene product activity. Expression levels can be monitored in a variety of ways, including by detecting mRNA levels, protein levels, or protein activity, any of which can be measured using standard techniques. Detection can involve quantification of the level of gene expression *(e.g.,* genomic DNA, cDNA, mRNA, protein, or enzyme activity), or, alternatively, can be a qualitative assessment of the level of gene expression, in particular in comparison with a control level. The type of level being detected will be clear from the context.

In a particular embodiment, the mRNA expression level can be determined both by *in situ* and by *in vitro* formats in a biological sample using methods known in the art. The term "biological sample" is intended to include tissues, cells, biological fluids and isolates thereof, isolated from a subject, as well as tissues, cells and fluids present within a subject. Many expression detection methods use isolated RNA. For *in vitro* methods, any RNA isolation technique that does not select against the isolation of mRNA can be utilized for the purification of RNA from cells (see, *e.g.,* Ausubel et al., ed., Current Protocols in Molecular Biology, John Wiley & Sons, New York 1987-1999). Additionally, large numbers of tissue samples can readily be processed using techniques well known to those of skill in the art, such as, for example, the single-step RNA isolation process of Chomczynski (1989, U.S. Patent No. 4,843,155).

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to a mRNA or genomic DNA encoding one or more biomarkers listed in Table 1 and the Examples. Other suitable probes for use in the diagnostic assays of the invention are described herein. Hybridization of an mRNA with the probe indicates that one or more biomarkers listed in Table 1 and the Examples is being expressed.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in a gene chip array, *e.g.,* an Affymetrix™ gene chip array. A skilled artisan can readily adapt known mRNA detection methods for use in detecting the level of the One or more biomarkers listed in Table 1 and the Examples mRNA expression levels.

An alternative method for determining mRNA expression level in a sample involves the process of nucleic acid amplification, *e.g.,* by RT-PCR (the experimental embodiment set forth in Mullis, 1987, U.S. Patent No. 4,683,202), ligase chain reaction (Barany, 1991, Proc. Natl. Acad. Sci. USA, 88:189-193), self-sustained sequence replication (Guatelli et al., 1990, Proc. Natl. Acad. Sci. USA 87:1874-1878), transcriptional amplification system (Kwoh et al., 1989, Proc. Natl. Acad. Sci. USA 86:1173-1177), Q-Beta Replicase (Lizardi etal., 1988, Bio/Technology 6:1197), rolling circle replication (Lizardi et al., U.S. Patent No. 5,854,033) or any other nucleic acid amplification method, followed by the detection of the amplified molecules using techniques well-known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, mRNA does not need to be isolated from the cells prior to detection. In such methods, a cell or tissue sample is prepared/processed using known histological methods. The sample is then immobilized on a support, typically a glass slide, and then contacted with a probe that can hybridize to the One or more biomarkers listed in Table 1 and the Examples mRNA.

As an alternative to making determinations based on the absolute expression level, determinations may be based on the normalized expression level of one or more biomarkers listed in Table 1 and the Examples. Expression levels are normalized by correcting the absolute expression level by comparing its expression to the expression of a non-biomarker gene, *e.g.,* a housekeeping gene that is constitutively expressed. Suitable genes for normalization include housekeeping genes such as the actin gene, or epithelial cell-specific genes. This normalization allows the comparison of the expression level in one sample, *e.g.,* a subject sample, to another sample, *e.g.,* a normal sample, or between samples from different sources.

The level or activity of a protein corresponding to one or more biomarkers listed in Table 1 and the Examples can also be detected and/or quantified by detecting or quantifying the expressed polypeptide. The polypeptide can be detected and quantified by any of a number of means well known to those of skill in the art. These may include analytic biochemical methods such as electrophoresis, capillary electrophoresis, high performance liquid chromatography (HPLC), thin layer chromatography (TLC), hyperdiffusion chromatography, and the like, or various immunological methods such as fluid or gel precipitin reactions, immunodiffusion (single or double), immunoelectrophoresis, radioimmunoassay (RIA), enzyme-linked immunosorbent assays (ELISAs), immunofluorescent assays, Western blotting, and the like. A skilled artisan can readily adapt known protein/antibody detection methods for use in determining whether cells express the biomarker of interest.

The present disclosure further provides soluble, purified and/or isolated polypeptide forms of one or more biomarkers listed in Table 1 and the Examples, or fragments thereof. In addition, it is to be understood that any and all attributes of the polypeptides described herein, such as percentage identities, polypeptide lengths, polypeptide fragments, biological activities, antibodies, *etc.* can be combined in any order or combination with respect to any biomarker listed in Table 1 and the Examples and combinations thereof.

In one aspect, a polypeptide may comprise a full-length amino acid sequence corresponding to one or more biomarkers listed in Table 1 and the Examples or a full-length amino acid sequence with 1 to about 20 conservative amino acid substitutions. An amino acid sequence of any described herein can also be at least 50, 55, 60, 65, 70, 75, 80, 85, 90, 91, 92, 93, 94, 95, 96, 97, 98, 99, or 99.5% identical to the full-length sequence of one or more biomarkers listed in Table 1 and the Examples, which is either described herein, well known in the art, or a fragment thereof. In another aspect, the present invention contemplates a composition comprising an isolated polypeptide corresponding to one or more biomarkers listed in Table 1 and the Examples and less than about 25%, or alternatively 15%, or alternatively 5%, contaminating biological macromolecules or polypeptides. Moreover, polypeptides having at least 80%, 81%, 82%, 83%, 84%, 85%, 86%, 87%, 88%, 89%, 90%, 91%, 92%, 93%, 94%, 95%, 96%, 97%, 98%, 99%, or more identity over the entire length with a polypeptpide comprising or consisting of those shown in Table 1 or the Examples, or orthologs or fragments thereof and having one or more of the following biological activities: 1) it can modulate the expression of one or more of cidea, adiponectin (adipoq), adipsin, otopetrin, type II deiodinase, cig30, ppar gamma 2, pgc1α, ucp1, elovl3, cAMP, Prdm16, cytochrome C, cox4i1, coxIII, cox5b, cox7a1, cox8b, glut4, atpase b2, cox II, atp5o, ndufb5, ap2, ndufs1, GRP109A, acylCoA-thioesterase 4, EARA1, claudin1, PEPCK, fgf21, acylCoA-thioesterase 3, and dio; 2) it can increase cellular respiration *(i.e.,* basal, total, and/or uncoupled respiration); 3) it can increase thermogenesis of adipose cells; 4) it can increase insulin sensitivity of adipose, muscle and/or hepatic cells; 5) it can decrease hepatosteatosis, obesity, type II diabetes, and/or appetite; 6) it can increase insulin secretion of pancreatic beta cells; 7) it can increase cardiac function to combat cardiac hypertrophy; 8) it can improve muscle hypoplasia; 9) it can reduce the growth and effects of obesity-associated cancer, cachexia, and anorexia; 10) it can increase the differentiation, generation, and/or proliferation of adipose cells, 11) it can decrease blood glucose levels, and 12) it can treat diseases or disorders characterized by increased PGC-1 expression or activity, *e.g.,* diabetes or obesity, are contemplated.

Polypeptides of the present disclosure can also be derivatized according to well-known methods in the art. Exemplary derivatives include compounds in which: 1. the compound or some portion thereof is cyclic *(e.g.,* two or more Cys residues are engineered to cyclize by disulfide bond formation); 2. the compound is cross-linked or is rendered capable of cross-linking between molecules; 3. one or more peptidyl [-C(O)NR-] linkages (bonds) is replaced by a non-peptidyl linkage; 4. the N-terminus is derivatized, such as through acylation or modification to a substituted amine; 5. the free C-terminus is derivatized, such as through esterification or amidation; 6. a disulfide bond is replaced with another, preferably more stable, cross-linking moiety *(e.g.,* an alkylene); 7. one or more individual amino acid residues is modified such as by reacting specifically with selected sidechains or terminal residues *(e.g.,* reacting lysinyl residues and amino terminal residues with succinic or other carboxylic acid anhydrides to reverse the charge of the lsyinyl residues). See US. Pat. Publ. 2005-0124537 for additional art-recognized derivitzation methods.

The description also provides chimeric or fusion proteins of the biomarkers of the invention, including the biomarkers listed in Table 1 and the Examples, or fragments thereof. As used herein, a "chimeric protein" or "fusion protein" comprises one or more biomarkers of the invention, including one or more biomarkers listed in Table 1 and the Examples, or a fragment thereof, operatively linked to another polypeptide having an amino acid sequence corresponding to a protein which is not substantially homologous to the respective biomarker. In a preferred embodiment, the fusion protein comprises at least one biologically active portion of one or more biomarkers of the invention, including one or more biomarkers listed in Table 1 and the Examples, or fragments thereof. Within the fusion protein, the term "operatively linked" is intended to indicate that the biomarker sequences and the non-biomarker sequences are fused in-frame to each other in such a way as to preserve functions exhibited when expressed independently of the fusion. The "another" sequences can be fused to the N-terminus or C-terminus of the biomarker sequences, respectively. Such fusion proteins are useful for increasing protein solubility, affinity, stability, valency, bioavilability, and/or facilitating protein purification, identification, detection, and/or structural characterization.

Exemplary domains, include, for example, glutathione S-transferase (GST), protein A, protein G, calmodulin-binding peptide, thioredoxin, maltose binding protein, HA, myc, poly arginine, poly His, poly His-Asp or FLAG fusion proteins and tags. Additional exemplary domains include domains that alter protein localization *in vivo,* such as signal peptides, type III secretion system-targeting peptides, transcytosis domains, nuclear localization signals, *etc.* In various embodiments, a polypeptide of the present invention may comprise one or more heterologous fusions. Polypeptides may contain multiple copies of the same fusion domain or may contain fusions to two or more different domains. The fusions may occur at the N-terminus of the polypeptide, at the C-terminus of the polypeptide, or at both the N- and C-terminus of the polypeptide. It is also within the scope of the disclosure to include linker sequences between a polypeptide of the invention and the fusion domain in order to facilitate construction of the fusion protein or to optimize protein expression or structural constraints of the fusion protein.

In one embodiment, "linker" group is used to connect the fusion partners. Any "linker" group is optional. When present, its chemical structure is not critical, since it serves primarily as a spacer. The linker is preferably made up of amino acids linked together by peptide bonds. Thus, in preferred embodiments, the linker is made up of from 1 to 20 amino acids linked by peptide bonds, wherein the amino acids are selected from the 20 naturally occurring amino acids. Some of these amino acids may be glycosylated, as is well understood by those in the art.

In a more preferred embodiment, the 1 to 20 amino acids, *e.g.,* a linker of at least 8, 9, 10, 15, 20 amino acids, and can be selected from glycine, alanine, proline, asparagine, glutamine, and lysine. Even more preferably, a linker is made up of a majority of amino acids that are sterically unhindered, such as glycine and alanine. Thus, the linker can be, *e.g.,* an unstructured recombinant polymer (URP), *e.g.,* a URP that is 9, 10, 11, 12, 13, 14, 15, 20 amino acids in length, *i.e.,* the linker has limited secondary structure or lacks secondary structure as determined by, *e.g.,* the Chou-Fasman algorithm. An exemplary linker comprises or consists of the amino acid sequence GGGGAGGGG. Other polyglycine linkers include Gly4, Gly5, and poly(Gly-Ala). Polyalanine linkers are contemplated as well. Other specific examples of linkers include, without limitation, (Gly)3Lys(Gly)4; (Gly)3AsnGlySer(Gly)2; (Gly)3Cys(Gly)4; and GlyProAsnGlyGly. To explain the above nomenclature, for example, (Gly)3Lys(Gly)4 means Gly-Gly-Gly-Lys-Gly-Gly-Gly-Gly. Combinations of Gly and Ala are also preferred. The linkers shown here are exemplary and linkers may be much longer and may include other residues. Non-peptide linkers are also possible. For example, alkyl linkers can be used and can further be substituted by any non-sterically hindering group, such as lower alkyl (*e.g.,* C₁-C₆), lower acyl, halogen *(e.g.,* Cl, Br), CN, NH2, phenyl, etc. An exemplary non-peptide linker is a PEG linker, wherein the linker has a molecular weight of 100 to 5000 kD, preferably 100 to 500 kD.

In another embodiment, of the disclosure the polypeptide may be constructed so as to contain protease cleavage sites between the fusion polypeptide and polypeptide of the invention in order to remove the tag after protein expression or thereafter. Examples of suitable endoproteases, include, for example, Factor Xa and TEV proteases.

In some embodiments, polypeptides of the present invention, or fragments thereof, are fused to an antibody *(e.g.,* IgG 1, IgG2, IgG3, IgG4) fragment *(e.g.,* Fc polypeptides). Techniques for preparing these fusion proteins are known, and are described, for example, in WO 99/31241 and in Cosman et.al. (2001) Immunity 14:123-133. Fusion to an Fc polypeptide offers the additional advantage of increasing *in vivo* protein half-life and bioavailability and facilitating purification by affinity chromatography over Protein A or Protein G columns. In some embodiments, "Fc variants" are useful as a fusion partner and represent a molecule or sequence that lacks one or more native Fc sites or residues that affect or are involved in (1) disulfide bond formation, (2) incompatibility with a selected host cell (3) N-terminal heterogeneity upon expression in a selected host cell, (4) glycosylation, (5) interaction with complement, (6) binding to an Fc receptor other than a salvage receptor, or (7) antibody-dependent cellular cytotoxicity (ADCC) (see, WO 97/34631, WO 96/32478, and US Pat. Publ. 2005-0124537). In addition, polypeptides of the present invention, and fragments thereof, can be modified according to well-known pharmacological methods in the art (e.g., pegylation, glycosylation, oligomerization, etc.) in order to further enhance desirable biological activities, such as increased bioavailability and decreased proteolytic degradation.

In still another embodiment, a polypeptide of the present invention may be labeled with a fluorescent label to facilitate their detection, purification, or structural characterization. In an exemplary embodiment, a polypeptide of the present invention may be fused to a heterologous polypeptide sequence which produces a detectable fluorescent signal, including, for example, green fluorescent protein (GFP), enhanced green fluorescent protein (EGFP), Renilla Reniformis green fluorescent protein, GFPmut2, GFPuv4, enhanced yellow fluorescent protein (EYFP), enhanced cyan fluorescent protein (ECFP), enhanced blue fluorescent protein (EBFP), citrine and red fluorescent protein from discosoma (dsRED).

Such a fusion protein can be produced by recombinant expression of a nucleotide sequence encoding the first peptide and a nucleotide sequence encoding the second peptide. Fusion proteins and peptides produced by recombinant techniques can be secreted and isolated from a mixture of cells and medium containing the protein or peptide. Alternatively, the protein or peptide can be retained cytoplasmically and the cells harvested, lysed and the protein isolated. A cell culture typically includes host cells, media and other byproducts. Suitable media for cell culture are well known in the art. Protein and peptides can be isolated from cell culture media, host cells, or both using techniques known in the art for purifying proteins and peptides. Techniques for transfecting host cells and purifying proteins and peptides are known in the art.

Preferably, a fusion protein of the invention is produced by standard recombinant DNA techniques. For example, DNA fragments coding for the different polypeptide sequences are ligated together in-frame in accordance with conventional techniques, for example employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992).

In addition to modified polypeptides, other agents are preferably used. In some embodiments, inhibitors of EGF receptor and/or PTH receptor are preferred. EGFR inhibitors or targeted therapies are well known in the art and include, for example, gefitinib (Iressa™), erlotinib (Tarceva™), cetuximab (Erbitux™), lapatinib (Tykerb™), panitumumab (Vectibix™), vandetanib (Caprelsa™), afatinib/BIBW2992, CI-1033/canertinib, neratinib/HKI-272, CP-724714, TAK-285, AST-1306, ARRY 334543, ARRY-380, AG-1478, dacomitinib/PF299804, OSI-420/desmethyl erlotinib, AZD8931, AEE788, pelitinib/EKB-569, CUDC-101, WZ8040, WZ4002, WZ3146, AG-490, XL647, PD153035, BMS-599626, sorafenib, imatinib, sunitinib, and dasatinib. Similarly, PTH receptor inhibitors or targeted therapies are well-known in the art and include, for example, antibody-based, small-molecule based, and peptide based inhibitors of the PTH receptor (see, for example, Carter et al. (2007) Proc. Natl. Acad. Sci. USA 104:6846-6851, Rosen et al. (1997) Calcif. Tissue Int. 61:455-459, and Shimizu *et al.* (2005) 280:1797-1807).

The present disclosure further provides compositions related to producing, detecting, characterizing, or modulating the level or activity of such polypeptides, or fragment thereof, such as nucleic acids, vectors, host cells, and the like. Such compositions may serve as compounds that modulate the expression and/or activity of one or more biomarkers listed in Table 1 and the Examples. For example, anti-PTH, anti-PTHrP1, and/or anti-PTH receptor antibodies that may bind specifically to their target can be used to reduce PTH/PTHrP1-mediated signaling and thereby modulate a metabolic response or metabolic disorder.

An isolated polypeptide or a fragment thereof (or a nucleic acid encoding such a polypeptide) corresponding to one or more biomarkers of the invention, including the biomarkers listed in Table 1 and the Examples or fragments thereof, can be used as an immunogen to generate antibodies that bind to said immunogen, using standard techniques for polyclonal and monoclonal antibody preparation according to well-known methods in the art. An antigenic peptide comprises at least 8 amino acid residues and encompasses an epitope present in the respective full length molecule such that an antibody raised against the peptide forms a specific immune complex with the respective full length molecule. Preferably, the antigenic peptide comprises at least 10 amino acid residues. In one embodiment such epitopes can be specific for a given polypeptide molecule from one species, such as mouse or human *(i.e.,* an antigenic peptide that spans a region of the polypeptide molecule that is not conserved across species is used as immunogen; such non conserved residues can be determined using an alignment such as that provided herein).

For example, a polypeptide immunogen typically is used to prepare antibodies by immunizing a suitable subject (*e.g*., rabbit, goat, mouse or other mammal) with the immunogen. An appropriate immunogenic preparation can contain, for example, a recombinantly expressed or chemically synthesized molecule or fragment thereof to which the immune response is to be generated. The preparation can further include an adjuvant, such as Freund's complete or incomplete adjuvant, or similar immunostimulatory agent. Immunization of a suitable subject with an immunogenic preparation induces a polyclonal antibody response to the antigenic peptide contained therein.

Polyclonal antibodies can be prepared as described above by immunizing a suitable subject with a polypeptide immunogen. The polypeptide antibody titer in the immunized subject can be monitored over time by standard techniques, such as with an enzyme linked immunosorbent assay (ELISA) using immobilized polypeptide. If desired, the antibody directed against the antigen can be isolated from the mammal (*e.g*., from the blood) and further purified by well-known techniques, such as protein A chromatography, to obtain the IgG fraction. At an appropriate time after immunization, *e.g*., when the antibody titers are highest, antibody-producing cells can be obtained from the subject and used to prepare monoclonal antibodies by standard techniques, such as the hybridoma technique (originally described by Kohler and Milstein (1975) Nature 256:495-497) (see also Brown et al. (1981) J. Immunol. 127:539-46; Brown etal. (1980) J. Biol. Chem. 255:4980-83; Yeh etal. (1976) Proc. Natl. Acad. Sci. 76:2927-31; Yeh et al. (1982) Int. J. Cancer 29:269-75), the more recent human B cell hybridoma technique (Kozbor et al. (1983) Immunol. Today 4:72), the EBV-hybridoma technique (Cole et al. (1985) Monoclonal Antibodies and Cancer Therapy, Alan R. Liss, Inc., pp. 77-96) or trioma techniques. The technology for producing monoclonal antibody hybridomas is well known (see generally Kenneth, R. H. in Monoclonal Antibodies: A New Dimension In Biological Analyses, Plenum Publishing Corp., New York, New York (1980); Lerner, E. A. (1981) Yale J. Biol. Med. 54:387-402; Gefter, M. L. et al. (1977) Somatic Cell Genet. 3:231-36). Briefly, an immortal cell line (typically a myeloma) is fused to lymphocytes (typically splenocytes) from a mammal immunized with an immunogen as described above, and the culture supernatants of the resulting hybridoma cells are screened to identify a hybridoma producing a monoclonal antibody that binds to the polypeptide antigen, preferably specifically.

Any of the many well-known protocols used for fusing lymphocytes and immortalized cell lines can be applied for the purpose of generating a monoclonal antibody against one or more biomarkers of the invention, including the biomarkers listed in Table 1 and the Examples, or a fragment thereof (see, *e.g.,* Galfre et al. (1977) Nature 266:55052; Gefter *et al.* (1977) *supra;* Lerner (1981) *supra;* Kenneth (1980) *supra*)*.* Moreover, the ordinary skilled worker will appreciate that there are many variations of such methods which also would be useful. Typically, the immortal cell line *(e.g.,* a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, *e.g*., the P3-NS1/1-Ag4-1, P3-x63-Ag8.653 or Sp2/O-Ag14 myeloma lines. These myeloma lines are available from the American Type Culture Collection (ATCC), Rockville, MD. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind a given polypeptide, *e.g.,* using a standard ELISA assay.

As an alternative to preparing monoclonal antibody-secreting hybridomas, a monoclonal specific for one of the above described polypeptides can be identified and isolated by screening a recombinant combinatorial immunoglobulin library (*e.g*., an antibody phage display library) with the appropriate polypeptide to thereby isolate immunoglobulin library members that bind the polypeptide. Kits for generating and screening phage display libraries are commercially available (*e.g.,* the Pharmacia *Recombinant Phage Antibody System,* Catalog No. 27-9400-01; and the Stratagene *SurfZAP*™ *Phage Display Kit,* Catalog No. 240612). Additionally, examples of methods and reagents particularly amenable for use in generating and screening an antibody display library can be found in, for example, Ladner et al. U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Biotechnology (NY) 9:1369-1372; Hay et al. (1992) Hum. Antibod. Hybridomas 3:81-85; Huse et al. (1989) Science 246:1275-1281; Griffiths etal. (1993) EMBO J. 12:725-734; Hawkins et al. (1992) J. Mol. Biol. 226:889-896; Clarkson et al. (1991) Nature 352:624-628; Gram et al. (1992) Proc. Natl. Acad. Sci. USA 89:3576-3580; Garrard et al. (1991) Biotechnology (NY) 9:1373-1377; Hoogenboom et al. (1991) Nucleic Acids Res. 19:4133-4137; Barbas et al. (1991) Proc. Natl. Acad. Sci. USA 88:7978-7982; and McCafferty et al. (1990) Nature 348:552-554.

Additionally, recombinant polypeptide antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in Robinson et al. International Patent Publication PCT/US86/02269; Akira *et al.* European Patent Application 184,187; Taniguchi, M. European Patent Application 171,496; Morrison *et al.* European Patent Application 173,494; Neuberger et al. PCT Application WO 86/01533; Cabilly et al. U.S. Patent No. 4,816,567; Cabilly *et al.* European Patent Application 125,023; Better et al. (1988) Science 240:1041-1043; Liu et al. (1987) Proc. Natl. Acad. Sci. USA 84:3439-3443; Liu et al. (1987) J. Immunol. 139:3521-3526; Sun et al. (1987) Proc. Natl. Acad. Sci. 84:214-218; Nishimura et al. (1987) Cancer Res. 47:999-1005; Wood et al. (1985) Nature 314:446-449; Shaw et al. (1988) J. Natl. Cancer Inst. 80:1553-1559); Morrison, S. L. (1985) Science 229:1202-1207; Oi et al. (1986) Biotechniques 4:214; Winter U.S. Patent 5,225,539; Jones et al. (1986) Nature 321:552-525; Verhoeyan et al. (1988) Science 239:1534; and Beidler et al. (1988) J. Immunol. 141:4053-4060.

In addition, humanized antibodies can be made according to standard protocols such as those disclosed in U.S. Patent 5,565,332. In another embodiment, antibody chains or specific binding pair members can be produced by recombination between vectors comprising nucleic acid molecules encoding a fusion of a polypeptide chain of a specific binding pair member and a component of a replicable generic display package and vectors containing nucleic acid molecules encoding a second polypeptide chain of a single binding pair member using techniques known in the art, *e.g.,* as described in U.S. Patents 5,565,332, 5,871,907, or 5,733,743. The use of intracellular antibodies to inhibit protein function in a cell is also known in the art (see *e.g.,* Carlson, J. R. (1988) Mol. Cell. Biol. 8:2638-2646; Biocca, S. et al. (1990) EMBO J. 9:101-108; Werge, T. M. et al. (1990) FEBS Lett. 274:193-198; Carlson, J. R. (1993) Proc. Natl. Acad. Sci. USA 90:7427-7428; Marasco, W. A. et al. (1993) Proc. Natl. Acad. Sci. USA 90:7889-7893; Biocca, S. et al. (1994) Biotechnology (NY) 12:396-399; Chen, S-Y. et al. (1994) Hum. Gene Ther. 5:595-601; Duan, L et al. (1994) Proc. Natl. Acad. Sci. USA 91:5075-5079; Chen, S-Y. et al. (1994) Proc. Natl. Acad. Sci. USA 91:5932-5936; Beerli, R. R. et al. (1994) J. Biol. Chem. 269:23931-23936; Beerli, R. R. et al. (1994) Biochem. Biophys. Res. Commun. 204:666-672; Mhashilkar, A. M. et al. (1995) EMBO J. 14:1542-1551; Richardson, J. H. et al. (1995) Proc. Natl. Acad. Sci. USA 92:3137-3141; PCT Publication No. WO 94/02610 by Marasco et al.; and PCT Publication No. WO 95/03832 by Duan et al.).

Additionally, fully human antibodies could be made against biomarkers of the invention, including the biomarkers listed in Table 1 and the Examples, or fragments thereof. Fully human antibodies can be made in mice that are transgenic for human immunoglobulin genes, *e.g.,* according to Hogan, et al., "Manipulating the Mouse Embryo: A Laboratory Manuel," Cold Spring Harbor Laboratory. Briefly, transgenic mice are immunized with purified immunogen. Spleen cells are harvested and fused to myeloma cells to produce hybridomas. Hybridomas are selected based on their ability to produce antibodies which bind to the immunogen. Fully human antibodies would reduce the immunogenicity of such antibodies in a human.

In one embodiment, an antibody for use in the instant invention is a bispecific antibody. A bispecific antibody has binding sites for two different antigens within a single antibody polypeptide. Antigen binding may be simultaneous or sequential. Triomas and hybrid hybridomas are two examples of cell lines that can secrete bispecific antibodies. Examples of bispecific antibodies produced by a hybrid hybridoma or a trioma are disclosed in U.S. Patent 4,474,893. Bispecific antibodies have been constructed by chemical means (Staerz et al. (1985) Nature 314:628, and Perez et al. (1985) Nature 316:354) and hybridoma technology (Staerz and Bevan (1986) Proc. Natl. Acad. Sci. USA, 83:1453, and Staerz and Bevan (1986) Immunol. Today 7:241). Bispecific antibodies are also described in U.S. Patent 5,959,084. Fragments of bispecific antibodies are described in U.S. Patent 5,798,229.

Bispecific agents can also be generated by making heterohybridomas by fusing hybridomas or other cells making different antibodies, followed by identification of clones producing and co-assembling both antibodies. They can also be generated by chemical or genetic conjugation of complete immunoglobulin chains or portions thereof such as Fab and Fv sequences. The antibody component can bind to a polypeptide or a fragment thereof of one or more biomarkers of the invention, including one or more biomarkers listed in Table 1 and the Examples, or a fragment thereof. In one embodiment, the bispecific antibody could specifically bind to both a polypeptide or a fragment thereof and its natural binding partner(s) or a fragment(s) thereof.

In another aspect of this disclosure peptides or peptide mimetics can be used to antagonize or promote the activity of one or more biomarkers of the invention, including one or more biomarkers listed in Table 1 and the Examples, or a fragment(s) thereof. In one embodiment, variants of one or more biomarkers listed in Table 1 and the Examples which function as a modulating agent for the respective full length protein, can be identified by screening combinatorial libraries of mutants, *e.g*., truncation mutants, for antagonist activity. In one embodiment, a variegated library of variants is generated by combinatorial mutagenesis at the nucleic acid level and is encoded by a variegated gene library. A variegated library of variants can be produced, for instance, by enzymatically ligating a mixture of synthetic oligonucleotides into gene sequences such that a degenerate set of potential polypeptide sequences is expressible as individual polypeptides containing the set of polypeptide sequences therein. There are a variety of methods which can be used to produce libraries of polypeptide variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential polypeptide sequences. Methods for synthesizing degenerate oligonucleotides are known in the art (see, *e.g.,* Narang, S. A. (1983) Tetrahedron 39:3; Itakura et al. (1984) Annu. Rev. Biochem. 53:323; Itakura et al. (1984) Science 198:1056; Ike et al. (1983) Nucleic Acid Res. 11:477.

In addition, libraries of fragments of a polypeptide coding sequence can be used to generate a variegated population of polypeptide fragments for screening and subsequent selection of variants of a given polypeptide. In one embodiment, a library of coding sequence fragments can be generated by treating a double stranded PCR fragment of a polypeptide coding sequence with a nuclease under conditions wherein nicking occurs only about once per polypeptide, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal, C-terminal and internal fragments of various sizes of the polypeptide.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of polypeptides. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a technique which enhances the frequency of functional mutants in the libraries, can be used in combination with the screening assays to identify variants of interest (Arkin and Youvan (1992) Proc. Natl. Acad. Sci. USA 89:7811-7815; Delagrave et al. (1993) Protein Eng. 6(3):327-331). In one embodiment, cell based assays can be exploited to analyze a variegated polypeptide library. For example, a library of expression vectors can be transfected into a cell line which ordinarily synthesizes one or more biomarkers of the invention, including one or more biomarkers listed in Table 1 and the Examples, or a fragment thereof. The transfected cells are then cultured such that the full length polypeptide and a particular mutant polypeptide are produced and the effect of expression of the mutant on the full length polypeptide activity in cell supernatants can be detected, *e.g.,* by any of a number of functional assays. Plasmid DNA can then be recovered from the cells which score for inhibition, or alternatively, potentiation of full length polypeptide activity, and the individual clones further characterized.

Systematic substitution of one or more amino acids of a polypeptide amino acid sequence with a D-amino acid of the same type (*e.g*., D-lysine in place of L-lysine) can be used to generate more stable peptides. In addition, constrained peptides comprising a polypeptide amino acid sequence of interest or a substantially identical sequence variation can be generated by methods known in the art (Rizo and Gierasch (1992) Annu. Rev. Biochem. 61:387); for example, by adding internal cysteine residues capable of forming intramolecular disulfide bridges which cyclize the peptide.

The amino acid sequences disclosed herein will enable those of skill in the art to produce polypeptides corresponding peptide sequences and sequence variants thereof. Such polypeptides can be produced in prokaryotic or eukaryotic host cells by expression of polynucleotides encoding the peptide sequence, frequently as part of a larger polypeptide. Alternatively, such peptides can be synthesized by chemical methods. Methods for expression of heterologous proteins in recombinant hosts, chemical synthesis of polypeptides, and *in vitro* translation are well known in the art and are described further in Maniatis et al. Molecular Cloning: A Laboratory Manual (1989), 2nd Ed., Cold Spring Harbor, N.Y.; Berger and Kimmel, Methods in Enzymology, Volume 152, Guide to Molecular Cloning Techniques (1987), Academic Press, Inc., San Diego, Calif.; Merrifield, J. (1969) J. Am. Chem. Soc. 91:501; Chaiken I. M. (1981) CRC Crit. Rev. Biochem. 11: 255; Kaiser et al. (1989) Science 243:187; Merrifield, B. (1986) Science 232:342; Kent, S. B. H. (1988) Annu. Rev. Biochem. 57:957; and Offord, R. E. (1980) Semisynthetic Proteins, Wiley Publishing).

Peptides can be produced, typically by direct chemical synthesis. Peptides can be produced as modified peptides, with nonpeptide moieties attached by covalent linkage to the N-terminus and/or C-terminus. In certain preferred embodiments, either the carboxy-terminus or the amino-terminus, or both, are chemically modified. The most common modifications of the terminal amino and carboxyl groups are acetylation and amidation, respectively. Amino-terminal modifications such as acylation *(e.g.,* acetylation) or alkylation *(e.g.,* methylation) and carboxy-terminal-modifications such as amidation, as well as other terminal modifications, including cyclization, can be incorporated into various embodiments of the invention. Certain amino-terminal and/or carboxy-terminal modifications and/or peptide extensions to the core sequence can provide advantageous physical, chemical, biochemical, and pharmacological properties, such as: enhanced stability, increased potency and/or efficacy, resistance to serum proteases, desirable pharmacokinetic properties, and others. Peptides disclosed herein can be used therapeutically to treat disease, *e.g*., by altering costimulation in a patient.

Peptidomimetics (Fauchere, J. (1986) Adv. Drug Res. 15:29; Veber and Freidinger (1985) TINS p.392; and Evans et al. (1987) J. Med. Chem. 30:1229) are usually developed with the aid of computerized molecular modeling. Peptide mimetics that are structurally similar to therapeutically useful peptides can be used to produce an equivalent therapeutic or prophylactic effect. Generally, peptidomimetics are structurally similar to a paradigm polypeptide (*i.e.,* a polypeptide that has a biological or pharmacological activity), but have one or more peptide linkages optionally replaced by a linkage selected from the group consisting of: -CH2NH-, -CH2S-, -CH2-CH2-, -CH=CH- (cis and trans), -COCH2-, - CH(OH)CH2-, and -CH2SO-, by methods known in the art and further described in the following references: Spatola, A. F. in "Chemistry and Biochemistry of Amino Acids, Peptides, and Proteins" Weinstein, B., ed., Marcel Dekker, New York, p. 267 (1983); Spatola, A. F., Vega Data (March 1983), Vol. 1, Issue 3, "Peptide Backbone Modifications" (general review); Morley, J. S. (1980) Trends Pharm. Sci. pp. 463-468 (general review); Hudson, D. et al. (1979) Int. J. Pept. Prot. Res. 14:177-185 (-CH2NH-, CH2CH2-); Spatola, A. F. et al. (1986) Life Sci. 38:1243-1249 (-CH2-S); Hann, M. M. (1982) J. Chem. Soc. Perkin Trans. I. 307-314 (-CH-CH-, cis and trans); Almquist, R. G. et al. (190) J. Med. Chem. 23:1392-1398 (-COCH2-); Jennings-White, C. et al. (1982) Tetrahedron Lett. 23:2533 (-COCH2-); Szelke, M. *et al.* European Appln. EP 45665 (1982) CA: 97:39405 (1982)(-CH(OH)CH2-); Holladay, M. W. et al. (1983) Tetrahedron Lett. (1983) 24:4401-4404 (-C(OH)CH2-); and Hruby, V. J. (1982) Life Sci. (1982) 31:189-199 (-CH2-S-). A particularly preferred non-peptide linkage is -CH2NH-. Such peptide mimetics may have significant advantages over polypeptide embodiments, including, for example: more economical production, greater chemical stability, enhanced pharmacological properties (half-life, absorption, potency, efficacy, etc.), altered specificity (*e.g*., a broad-spectrum of biological activities), reduced antigenicity, and others. Labeling of peptidomimetics usually involves covalent attachment of one or more labels, directly or through a spacer (*e.g.,* an amide group), to non-interfering position(s) on the peptidomimetic that are predicted by quantitative structure-activity data and/or molecular modeling. Such non-interfering positions generally are positions that do not form direct contacts with the macropolypeptides(s) to which the peptidomimetic binds to produce the therapeutic effect. Derivitization (*e.g*., labeling) of peptidomimetics should not substantially interfere with the desired biological or pharmacological activity of the peptidomimetic.

Also encompassed by the present invention are small molecules which can modulate (either enhance or inhibit) interactions, *e.g*., between biomarkers listed in Table 1 and the Examples and their natural binding partners, or inhibit activity. The small molecules of the present invention can be obtained using any of the numerous approaches in combinatorial library methods known in the art, including: spatially addressable parallel solid phase or solution phase libraries; synthetic library methods requiring deconvolution; the 'one-bead one-compound' library method; and synthetic library methods using affinity chromatography selection. (Lam, K. S. (1997) Anticancer Drug Des. 12:145).

Examples of methods for the synthesis of molecular libraries can be found in the art, for example in: DeWitt et al. (1993) Proc. Natl. Acad. Sci. USA 90:6909; Erb et al. (1994) Proc. Natl. Acad. Sci. USA 91:11422; Zuckermann et al. (1994) J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233.

Libraries of compounds can be presented in solution (e.g., Houghten (1992) Biotechniques 13:412-421), or on beads (Lam (1991) Nature 354:82-84), chips (Fodor (1993) Nature 364:555-556), bacteria (Ladner USP 5,223,409), spores (Ladner USP '409), plasmids (Cull et al. (1992) Proc. Natl. Acad. Sci. USA 89:1865-1869) or on phage (Scott and Smith (1990) Science 249:386-390); (Devlin (1990) Science 249:404-406); (Cwirla et al. (1990) Proc. Natl. Acad. Sci. USA 87:6378-6382); (Felici (1991) J. Mol. Biol. 222:301-310); (Ladner *supra.*)*.* Compounds can be screened in cell based or non-cell based assays. Compounds can be screened in pools (*e.g*., multiple compounds in each testing sample) or as individual compounds.

Also provided herein are compositions comprising one or more nucleic acids comprising or capable of expressing at least 1, 2, 3, 4, 5, 10, 20 or more small nucleic acids or antisense oligonucleotides or derivatives thereof, wherein said small nucleic acids or antisense oligonucleotides or derivatives thereof in a cell specifically hybridize (*e.g*., bind) under cellular conditions, with cellular nucleic acids (*e.g*., small non-coding RNAS such as miRNAs, pre-miRNAs, pri-miRNAs, miRNA*, anti-miRNA, a miRNA binding site, a variant and/or functional variant thereof, cellular mRNAs or a fragments thereof). In one embodiment, expression of the small nucleic acids or antisense oligonucleotides or derivatives thereof in a cell can enhance or upregulate one or more biological activities associated with the corresponding wild-type, naturally occurring, or synthetic small nucleic acids. In another embodiment, expression of the small nucleic acids or antisense oligonucleotides or derivatives thereof in a cell can inhibit expression or biological activity of cellular nucleic acids and/or proteins, *e.g*., by inhibiting transcription, translation and/or small nucleic acid processing of, for example, one or more biomarkers of the invention, including one or more biomarkers listed in Table 1 and the Examples, or fragment(s) thereof. In one embodiment, the small nucleic acids or antisense oligonucleotides or derivatives thereof are small RNAs (*e.g*., microRNAs) or complements of small RNAs. In another embodiment, the small nucleic acids or antisense oligonucleotides or derivatives thereof can be single or double stranded and are at least six nucleotides in length and are less than about 1000, 900, 800, 700, 600, 500, 400, 300, 200, 100, 50, 40, 30, 25, 24, 23, 22, 21,20, 19, 18, 17, 16, 15, or 10 nucleotides in length. In another embodiment, a composition may comprise a library of nucleic acids comprising or capable of expressing small nucleic acids or antisense oligonucleotides or derivatives thereof, or pools of said small nucleic acids or antisense oligonucleotides or derivatives thereof. A pool of nucleic acids may comprise about 2-5, 5-10, 10-20, 10-30 or more nucleic acids comprising or capable of expressing small nucleic acids or antisense oligonucleotides or derivatives thereof.

In one embodiment, binding may be by conventional base pair complementarity, or, for example, in the case of binding to DNA duplexes, through specific interactions in the major groove of the double helix. In general, "antisense" refers to the range of techniques generally employed in the art, and includes any process that relies on specific binding to oligonucleotide sequences.

It is well known in the art that modifications can be made to the sequence of a miRNA or a pre-miRNA without disrupting miRNA activity. As used herein, the term "functional variant" of a miRNA sequence refers to an oligonucleotide sequence that varies from the natural miRNA sequence, but retains one or more functional characteristics of the miRNA. In some embodiments, a functional variant of a miRNA sequence retains all of the functional characteristics of the miRNA. In certain embodiments, a functional variant of a miRNA has a nucleobase sequence that is a least about 60%, 65%, 70%, 75%, 80%, 85%, 90%, 91%, 92%, 93%, 94%, 95%, 96%, 97%, 98% or 99% identical to the miRNA or precursor thereof over a region of about 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100 or more nucleobases, or that the functional variant hybridizes to the complement of the miRNA or precursor thereof under stringent hybridization conditions. Accordingly, in certain embodiments the nucleobase sequence of a functional variant is capable of hybridizing to one or more target sequences of the miRNA.

miRNAs and their corresponding stem-loop sequences described herein may be found in miRBase, an online searchable database of miRNA sequences and annotation, found on the world wide web at microrna.sanger.ac.uk. Entries in the miRBase Sequence database represent a predicted hairpin portion of a miRNA transcript (the stem-loop), with information on the location and sequence of the mature miRNA sequence. The miRNA stem-loop sequences in the database are not strictly precursor miRNAs (pre-miRNAs), and may in some instances include the pre-miRNA and some flanking sequence from the presumed primary transcript. The miRNA nucleobase sequences described herein encompass any version of the miRNA, including the sequences described in Release 10.0 of the miRBase sequence database and sequences described in any earlier Release of the miRBase sequence database. A sequence database release may result in the re-naming of certain miRNAs. A sequence database release may result in a variation of a mature miRNA sequence.

In some embodiments, miRNA sequences of the invention may be associated with a second RNA sequence that may be located on the same RNA molecule or on a separate RNA molecule as the miRNA sequence. In such cases, the miRNA sequence may be referred to as the active strand, while the second RNA sequence, which is at least partially complementary to the miRNA sequence, may be referred to as the complementary strand. The active and complementary strands are hybridized to create a double-stranded RNA that is similar to a naturally occurring miRNA precursor. The activity of a miRNA may be optimized by maximizing uptake of the active strand and minimizing uptake of the complementary strand by the miRNA protein complex that regulates gene translation. This can be done through modification and/or design of the complementary strand.

In some embodiments, the complementary strand is modified so that a chemical group other than a phosphate or hydroxyl at its 5' terminus. The presence of the 5' modification apparently eliminates uptake of the complementary strand and subsequently favors uptake of the active strand by the miRNA protein complex. The 5' modification can be any of a variety of molecules known in the art, including NH₂, NHCOCH₃, and biotin. In another embodiment, the uptake of the complementary strand by the miRNA pathway is reduced by incorporating nucleotides with sugar modifications in the first 2-6 nucleotides of the complementary strand. It should be noted that such sugar modifications can be combined with the 5' terminal modifications described above to further enhance miRNA activities.

In some embodiments, the complementary strand is designed so that nucleotides in the 3' end of the complementary strand are not complementary to the active strand. This results in double-strand hybrid RNAs that are stable at the 3' end of the active strand but relatively unstable at the 5' end of the active strand. This difference in stability enhances the uptake of the active strand by the miRNA pathway, while reducing uptake of the complementary strand, thereby enhancing miRNA activity.

Small nucleic acid and/or antisense constructs of the methods and compositions presented herein can be delivered, for example, as an expression plasmid which, when transcribed in the cell, produces RNA which is complementary to at least a unique portion of cellular nucleic acids (*e.g*., small RNAs, mRNA, and/or genomic DNA). Alternatively, the small nucleic acid molecules can produce RNA which encodes mRNA, miRNA, pre-miRNA, pri-miRNA, miRNA*, anti-miRNA, or a miRNA binding site, or a variant thereof. For example, selection of plasmids suitable for expressing the miRNAs, methods for inserting nucleic acid sequences into the plasmid, and methods of delivering the recombinant plasmid to the cells of interest are within the skill in the art. See, for example, Zeng et al. (2002), Molecular Cell 9:1327-1333; Tuschl (2002), Nat. Biotechnol, 20:446-448; Brummelkamp et al. (2002), Science 296:550-553; Miyagishi et al. (2002), Nat. Biotechnol. 20:497-500; Paddison et al. (2002), Genes Dev. 16:948-958; Lee et al. (2002), Nat. Biotechnol. 20:500-505; and Paul et al. (2002), Nat. Biotechnol. 20:505-508.

Alternatively, small nucleic acids and/or antisense constructs are oligonucleotide probes that are generated *ex vivo* and which, when introduced into the cell, results in hybridization with cellular nucleic acids. Such oligonucleotide probes are preferably modified oligonucleotides that are resistant to endogenous nucleases, *e.g*., exonucleases and/or endonucleases, and are therefore stable *in vivo.* Exemplary nucleic acid molecules for use as small nucleic acids and/or antisense oligonucleotides are phosphoramidate, phosphothioate and methylphosphonate analogs of DNA (see also U.S. Patents 5,176,996; 5,264,564; and 5,256,775). Additionally, general approaches to constructing oligomers useful in antisense therapy have been reviewed, for example, by Van der Krol et al. (1988) BioTechniques 6:958-976; and Stein et al. (1988) Cancer Res 48:2659-2668.

Antisense approaches may involve the design of oligonucleotides (either DNA or RNA) that are complementary to cellular nucleic acids (*e.g*., complementary to biomarkers listed in Table 1 and the Examples). Absolute complementarity is not required. In the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with a nucleic acid *(e.g.,* RNA) it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of the mRNA, *e.g.,* the 5' untranslated sequence up to and including the AUG initiation codon, should work most efficiently at inhibiting translation. However, sequences complementary to the 3' untranslated sequences of mRNAs have recently been shown to be effective at inhibiting translation of mRNAs as well (Wagner, R. (1994) Nature 372:333). Therefore, oligonucleotides complementary to either the 5' or 3' untranslated, non-coding regions of genes could be used in an antisense approach to inhibit translation of endogenous mRNAs. Oligonucleotides complementary to the 5' untranslated region of the mRNA may include the complement of the AUG start codon. Antisense oligonucleotides complementary to mRNA coding regions are less efficient inhibitors of translation but could also be used in accordance with the methods and compositions presented herein. Whether designed to hybridize to the 5', 3' or coding region of cellular mRNAs, small nucleic acids and/or antisense nucleic acids should be at least six nucleotides in length, and can be less than about 1000, 900, 800, 700, 600, 500, 400, 300, 200, 100, 50, 40, 30, 25, 24, 23, 22, 21,20, 19, 18, 17, 16, 15, or 10 nucleotides in length.

Regardless of the choice of target sequence, it is preferred that *in vitro* studies are first performed to quantitate the ability of the antisense oligonucleotide to inhibit gene expression. In one embodiment these studies utilize controls that distinguish between antisense gene inhibition and nonspecific biological effects of oligonucleotides. In another embodiment these studies compare levels of the target nucleic acid or protein with that of an internal control nucleic acid or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

Small nucleic acids and/or antisense oligonucleotides can be DNA or RNA or chimeric mixtures or derivatives or modified versions thereof, single-stranded or double-stranded. Small nucleic acids and/or antisense oligonucleotides can be modified at the base moiety, sugar moiety, or phosphate backbone, for example, to improve stability of the molecule, hybridization, etc., and may include other appended groups such as peptides (*e.g.,* for targeting host cell receptors), or agents facilitating transport across the cell membrane (see, *e.g.,* Letsinger et al. (1989) Proc. Natl. Acad. Sci. U.S.A. 86:6553-6556; Lemaitre et al. (1987) Proc. Natl. Acad. Sci. 84:648-652; PCT Publication No. WO88/09810, published December 15, 1988) or the blood-brain barrier (see, *e.g.,* PCT Publication No. WO89/10134, published April 25, 1988), hybridization-triggered cleavage agents. (See, *e.g.,* Krol et al. (1988) BioTechniques 6:958-976) or intercalating agents. (See, *e.g.,* Zon (1988), Pharm. Res. 5:539-549). To this end, small nucleic acids and/or antisense oligonucleotides may be conjugated to another molecule, *e.g.,* a peptide, hybridization triggered cross-linking agent, transport agent, hybridization-triggered cleavage agent, etc.

Small nucleic acids and/or antisense oligonucleotides may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxytiethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Small nucleic acids and/or antisense oligonucleotides may also comprise at least one modified sugar moiety selected from the group including but not limited to arabinose, 2-fluoroarabinose, xylulose, and hexose.

In certain embodiments, a compound comprises an oligonucleotide (*e.g*., a miRNA or miRNA encoding oligonucleotide) conjugated to one or more moieties which enhance the activity, cellular distribution or cellular uptake of the resulting oligonucleotide. In certain such embodiments, the moiety is a cholesterol moiety (*e.g*., antagomirs) or a lipid moiety or liposome conjugate. Additional moieties for conjugation include carbohydrates, phospholipids, biotin, phenazine, folate, phenanthridine, anthraquinone, acridine, fluoresceins, rhodamines, coumarins, and dyes. In certain embodiments, a conjugate group is attached directly to the oligonucleotide. In certain embodiments, a conjugate group is attached to the oligonucleotide by a linking moiety selected from amino, hydroxyl, carboxylic acid, thiol, unsaturations (*e.g*., double or triple bonds), 8-amino-3,6-dioxaoctanoic acid (ADO), succinimidyl 4-(N-maleimidomethyl) cyclohexane-1-carboxylate (SMCC), 6-aminohexanoic acid (AHEX or AHA), substituted C1-C10 alkyl, substituted or unsubstituted C2-C10 alkenyl, and substituted or unsubstituted C2-C10 alkynyl. In certain such embodiments, a substituent group is selected from hydroxyl, amino, alkoxy, carboxy, benzyl, phenyl, nitro, thiol, thioalkoxy, halogen, alkyl, aryl, alkenyl and alkynyl.

In certain such embodiments, the compound comprises the oligonucleotide having one or more stabilizing groups that are attached to one or both termini of the oligonucleotide to enhance properties such as, for example, nuclease stability. Included in stabilizing groups are cap structures. These terminal modifications protect the oligonucleotide from exonuclease degradation, and can help in delivery and/or localization within a cell. The cap can be present at the 5'-terminus (5'-cap), or at the 3'-terminus (3'-cap), or can be present on both termini. Cap structures include, for example, inverted deoxy abasic caps.

Suitable cap structures include a 4',5'-methylene nucleotide, a 1-(beta-D-erythrofuranosyl) nucleotide, a 4'-thio nucleotide, a carbocyclic nucleotide, a 1,5-anhydrohexitol nucleotide, an L-nucleotide, an alpha-nucleotide, a modified base nucleotide, a phosphorodithioate linkage, a threo-pentofuranosyl nucleotide, an acyclic 3',4'-seco nucleotide, an acyclic 3,4-dihydroxybutyl nucleotide, an acyclic 3,5-dihydroxypentyl nucleotide, a 3'-3'-inverted nucleotide moiety, a 3'-3'-inverted abasic moiety, a 3'-2'-inverted nucleotide moiety, a 3'-2'-inverted abasic moiety, a 1,4-butanediol phosphate, a 3'-phosphoramidate, a hexylphosphate, an aminohexyl phosphate, a 3'-phosphate, a 3'-phosphorothioate, a phosphorodithioate, a bridging methylphosphonate moiety, and a non-bridging methylphosphonate moiety 5'-amino-alkyl phosphate, a 1,3-diamino-2-propyl phosphate, 3-aminopropyl phosphate, a 6-aminohexyl phosphate, a 1,2-aminododecyl phosphate, a hydroxypropyl phosphate, a 5'-5'-inverted nucleotide moiety, a 5'-5'-inverted abasic moiety, a 5'-phosphoramidate, a 5'-phosphorothioate, a 5'-amino, a bridging and/or non-bridging 5'-phosphoramidate, a phosphorothioate, and a 5'-mercapto moiety.

Small nucleic acids and/or antisense oligonucleotides can also contain a neutral peptide-like backbone. Such molecules are termed peptide nucleic acid (PNA)-oligomers and are described, *e.g.,* in Perry-O'Keefe et al. (1996) Proc. Natl. Acad. Sci. U.S.A. 93:14670 and in Eglom et al. (1993) Nature 365:566. One advantage of PNA oligomers is their capability to bind to complementary DNA essentially independently from the ionic strength of the medium due to the neutral backbone of the DNA. In yet another embodiment, small nucleic acids and/or antisense oligonucleotides comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In a further embodiment, small nucleic acids and/or antisense oligonucleotides are α-anomeric oligonucleotides. An α-anomeric oligonucleotide forms specific double-stranded hybrids with complementary RNA in which, contrary to the usual b-units, the strands run parallel to each other (Gautier et al. (1987) Nucl. Acids Res. 15:6625-6641). The oligonucleotide is a 2'-0-methylribonucleotide (Inoue et al. (1987) Nucl. Acids Res. 15:6131-6148), or a chimeric RNA-DNA analogue (Inoue et al. (1987) FEBS Lett. 215:327-330).

Small nucleic acids and/or antisense oligonucleotides of the methods and compositions presented herein may be synthesized by standard methods known in the art, *e.g.,* by use of an automated DNA synthesizer (such as are commercially available from Biosearch, Applied Biosystems, etc.). As examples, phosphorothioate oligonucleotides may be synthesized by the method of Stein et al. (1988) Nucl. Acids Res. 16:3209, methylphosphonate oligonucleotides can be prepared by use of controlled pore glass polymer supports (Sarin et al. (1988) Proc. Natl. Acad. Sci. U.S.A. 85:7448-7451), etc. For example, an isolated miRNA can be chemically synthesized or recombinantly produced using methods known in the art. In some instances, miRNA are chemically synthesized using appropriately protected ribonucleoside phosphoramidites and a conventional DNA/RNA synthesizer. Commercial suppliers of synthetic RNA molecules or synthesis reagents include, *e.g*., Proligo (Hamburg, Germany), Dharmacon Research (Lafayette, Colo., USA), Pierce Chemical (part of Perbio Science, Rockford, I11., USA), Glen Research (Sterling, Va., USA), ChemGenes (Ashland, Mass., USA), Cruachem (Glasgow, UK), and Exiqon (Vedbaek, Denmark).

Small nucleic acids and/or antisense oligonucleotides can be delivered to cells *in vivo.* A number of methods have been developed for delivering small nucleic acids and/or antisense oligonucleotides DNA or RNA to cells; *e.g*., antisense molecules can be injected directly into the tissue site, or modified antisense molecules, designed to target the desired cells *(e.g.,* antisense linked to peptides or antibodies that specifically bind receptors or antigens expressed on the target cell surface) can be administered systematically.

In one embodiment, small nucleic acids and/or antisense oligonucleotides may comprise or be generated from double stranded small interfering RNAs (siRNAs), in which sequences fully complementary to cellular nucleic acids (*e.g*., mRNAs) sequences mediate degradation or in which sequences incompletely complementary to cellular nucleic acids *(e.g.,* mRNAs) mediate translational repression when expressed within cells. In another embodiment, double stranded siRNAs can be processed into single stranded antisense RNAs that bind single stranded cellular RNAs (*e.g*., microRNAs) and inhibit their expression. RNA interference (RNAi) is the process of sequence-specific, post-transcriptional gene silencing in animals and plants, initiated by double-stranded RNA (dsRNA) that is homologous in sequence to the silenced gene *in vivo,* long dsRNA is cleaved by ribonuclease III to generate 21- and 22-nucleotide siRNAs. It has been shown that 21-nucleotide siRNA duplexes specifically suppress expression of endogenous and heterologous genes in different mammalian cell lines, including human embryonic kidney (293) and HeLa cells (Elbashir et al. (2001) Nature 411:494-498). Accordingly, translation of a gene in a cell can be inhibited by contacting the cell with short double stranded RNAs having a length of about 15 to 30 nucleotides or of about 18 to 21 nucleotides or of about 19 to 21 nucleotides. Alternatively, a vector encoding for such siRNAs or short hairpin RNAs (shRNAs) that are metabolized into siRNAs can be introduced into a target cell (see, *e.g.,* McManus et al. (2002) RNA 8:842; Xia et al. (2002) Nature Biotechnology 20:1006; and Brummelkamp et al. (2002) Science 296:550). Vectors that can be used are commercially available, *e.g*., from OligoEngine under the name pSuper RNAi System™.

Ribozyme molecules designed to catalytically cleave cellular mRNA transcripts can also be used to prevent translation of cellular mRNAs and expression of cellular polypeptides, or both (See, *e.g.,* PCT International Publication WO90/11364, published October 4, 1990; Sarver et al. (1990) Science 247:1222-1225 and U.S. Patent No. 5,093,246). While ribozymes that cleave mRNA at site specific recognition sequences can be used to destroy cellular mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach (1988) Nature 334:585-591. The ribozyme may be engineered so that the cleavage recognition site is located near the 5' end of cellular mRNAs; *i.e.,* to increase efficiency and minimize the intracellular accumulation of non-functional mRNA transcripts.

The ribozymes of the methods and compositions presented herein also include RNA endoribonucleases (hereinafter "Cech-type ribozymes") such as the one which occurs naturally in *Tetrahymena thermophila* (known as the IVS, or L-19 IVS RNA) and which has been extensively described by Thomas Cech and collaborators (Zaug, et al. (1984) Science 224:574-578; Zaug, et al. (1986) Science 231:470-475; Zaug, et al. (1986) Nature 324:429-433; published International patent application No. WO88/04300 by University Patents Inc.; Been, et al. (1986) Cell 47:207-216). The Cech-type ribozymes have an eight base pair active site which hybridizes to a target RNA sequence whereafter cleavage of the target RNA takes place. The methods and compositions presented herein encompasses those Cech-type ribozymes which target eight base-pair active site sequences that are present in cellular genes.

As in the antisense approach, the ribozymes can be composed of modified oligonucleotides (*e.g*., for improved stability, targeting, etc.). A preferred method of delivery involves using a DNA construct "encoding" the ribozyme under the control of a strong constitutive pol III or pol II promoter, so that transfected cells will produce sufficient quantities of the ribozyme to destroy endogenous cellular messages and inhibit translation. Because ribozymes unlike antisense molecules, are catalytic, a lower intracellular concentration is required for efficiency.

Nucleic acid molecules to be used in triple helix formation for the inhibition of transcription of cellular genes are preferably single stranded and composed of deoxyribonucleotides. The base composition of these oligonucleotides should promote triple helix formation via Hoogsteen base pairing rules, which generally require sizable stretches of either purines or pyrimidines to be present on one strand of a duplex. Nucleotide sequences may be pyrimidine-based, which will result in TAT and CGC triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, containing a stretch of G residues. These molecules will form a triple helix with a DNA duplex that is rich in GC pairs, in which the majority of the purine residues are located on a single strand of the targeted duplex, resulting in CGC triplets across the three strands in the triplex.

Alternatively, the potential sequences that can be targeted for triple helix formation may be increased by creating a so called "switchback" nucleic acid molecule. Switchback molecules are synthesized in an alternating 5'-3', 3'-5' manner, such that they base pair with first one strand of a duplex and then the other, eliminating the necessity for a sizable stretch of either purines or pyrimidines to be present on one strand of a duplex.

Small nucleic acids (*e.g*., miRNAs, pre-miRNAs, pri-miRNAs, miRNA*, anti-miRNA, or a miRNA binding site, or a variant thereof), antisense oligonucleotides, ribozymes, and triple helix molecules of the methods and compositions presented herein may be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Moreover, various well-known modifications to nucleic acid molecules may be introduced as a means of increasing intracellular stability and half-life. Possible modifications include but are not limited to the addition of flanking sequences of ribonucleotides or deoxyribonucleotides to the 5' and/or 3' ends of the molecule or the use of phosphorothioate or 2' O-methyl rather than phosphodiesterase linkages within the oligodeoxyribonucleotide backbone. One of skill in the art will readily understand that polypeptides, small nucleic acids, and antisense oligonucleotides can be further linked to another peptide or polypeptide *(e.g.,* a heterologous peptide), *e.g.,* that serves as a means of protein detection. Non-limiting examples of label peptide or polypeptide moieties useful for detection in the invention include, without limitation, suitable enzymes such as horseradish peroxidase, alkaline phosphatase, beta-galactosidase, or acetylcholinesterase; epitope tags, such as FLAG, MYC, HA, or HIS tags; fluorophores such as green fluorescent protein; dyes; radioisotopes; digoxygenin; biotin; antibodies; polymers; as well as others known in the art, for example, in Principles of Fluorescence Spectroscopy, Joseph R. Lakowicz (Editor), Plenum Pub Corp, 2nd edition (July 1999).

In addition to the agents described herein, additional agents are particularly useful for modulating the expression and/or activity of a biomarker of Table 1 or the Examples, or fragments thereof according to the present invention. For example,

The modulatory agents described herein (e.g., antibodies, small molecules, peptides, fusion proteins, or small nucleic acids) can be incorporated into pharmaceutical compositions and administered to a subject *in vivo.* The compositions may contain a single such molecule or agent or any combination of agents described herein. Based on the genetic pathway analyses described herein, it is believed that such combinations of agents is especially effective in diagnosing, prognosing, preventing, and treating metabolic disorders. Thus, "single active agents" described herein can be combined with other pharmacologically active compounds ("second active agents") known in the art according to the methods and compositions provided herein. It is believed that certain combinations work synergistically in the treatment of particular types of metabolic disorders. Second active agents can be large molecules (*e.g*., proteins) or small molecules (*e.g*., synthetic inorganic, organometallic, or organic molecules).

### III. Methods of Selecting Agents and Compositions

Another aspect of the disclosure relates to methods of selecting agents (*e.g.*, antibodies. fusion proteins, peptides, small molecules, or small nucleic acids) which bind to, upregulate, downregulate, or modulate one or more biomarkers of the invention listed in Table 1 and the Examples. Such methods can use screening assays, including cell based and non-cell based assays.

In one embodiment, the invention provides assays for screening candidate or test compounds which bind to or modulate the expression or activity level of, one or more biomarkers of the invention, including one or more biomarkers listed in Table 1 and the Examples, or a fragment thereof. Such compounds include, without limitation, antibodies, proteins, fusion proteins, nucleic acid molecules, and small molecules.

In one embodiment, an assay is a cell-based assay, comprising contacting a cell expressing one or more biomarkers of the invention, including one or more biomarkers listed in Table 1 and the Examples, or a fragment thereof, with a test compound and determining the ability of the test compound to modulate (*e.g*., stimulate or inhibit) the level of interaction between the biomarker and its natural binding partners as measured by direct binding or by measuring a parameter of a metabolic disorder.

For example, in a direct binding assay, the biomarker polypeptide, a binding partner polypeptide of the biomarker, or a fragment(s) thereof, can be coupled with a radioisotope or enzymatic label such that binding of the biomarker polypeptide or a fragment thereof to its natural binding partner(s) or a fragment(s) thereof can be determined by detecting the labeled molecule in a complex. For example, the biomarker polypeptide, a binding partner polypeptide of the biomarker, or a fragment(s) thereof, can be labeled with ¹²⁵I, ³⁵S, ¹⁴C, or ³H, either directly or indirectly, and the radioisotope detected by direct counting of radioemmission or by scintillation counting. Alternatively, the polypeptides of interest a can be enzymatically labeled with, for example, horseradish peroxidase, alkaline phosphatase, or luciferase, and the enzymatic label detected by determination of conversion of an appropriate substrate to product.

It is also within the scope of this disclosure to determine the ability of a compound to modulate the interactions between one or more biomarkers of the invention, including one or more biomarkers listed in Table 1 and the Examples, or a fragment thereof, and its natural binding partner(s) or a fragment(s) thereof, without the labeling of any of the interactants (e.g., using a microphysiometer as described in McConnell et al. (1992) Science 257:1906-1912). As used herein, a "microphysiometer" (*e.g*., Cytosensor) is an analytical instrument that measures the rate at which a cell acidifies its environment using a light-addressable potentiometric sensor (LAPS). Changes in this acidification rate can be used as an indicator of the interaction between compound and receptor.

In a preferred embodiment, determining the ability of the blocking agents *(e.g.,* antibodies, fusion proteins, peptides, nucleic acid molecules, or small molecules) to antagonize the interaction between a given set of polypeptides can be accomplished by determining the activity of one or more members of the set of interacting molecules. For example, the activity of one or more biomarkers of the invention, including one or more biomarkers listed in Table 1 and the Examples, or a fragment thereof, can be determined by detecting induction of cytokine or chemokine response, detecting catalytic/enzymatic activity of an appropriate substrate, detecting the induction of a reporter gene (comprising a target-responsive regulatory element operatively linked to a nucleic acid encoding a detectable marker, *e.g*., chloramphenicol acetyl transferase), or detecting a cellular response regulated by the biomarker or a fragment thereof (e.g., modulations of biological pathways identified herein, such as modulated proliferation, apoptosis, cell cycle, and/or ligand-receptor binding activity). Determining the ability of the blocking agent to bind to or interact with said polypeptide can be accomplished by measuring the ability of an agent to modulate immune responses, for example, by detecting changes in type and amount of cytokine secretion, changes in apoptosis or proliferation, changes in gene expression or activity associated with cellular identity, or by interfering with the ability of said polypeptide to bind to antibodies that recognize a portion thereof.

In yet another embodiment, an assay of the present disclosure is a cell-free assay in which one or more biomarkers of the invention, including one or more biomarkers listed in Table 1 and the Examples, or a fragment thereof, *e.g.,* a biologically active fragment thereof, is contacted with a test compound, and the ability of the test compound to bind to the polypeptide, or biologically active portion thereof, is determined. Binding of the test compound to the biomarker or a fragment thereof, can be determined either directly or indirectly as described above. Determining the ability of the biomarker or a fragment thereof to bind to its natural binding partner(s) or a fragment(s) thereof can also be accomplished using a technology such as real-time Biomolecular Interaction Analysis (BIA) (Sjolander, S. and Urbaniczky, C. (1991) Anal. Chem. 63:2338-2345 and Szabo et al. (1995) Curr. Opin. Struct. Biol. 5:699-705). As used herein, "BIA" is a technology for studying biospecific interactions in real time, without labeling any of the interactants (*e.g*., BIAcore). Changes in the optical phenomenon of surface plasmon resonance (SPR) can be used as an indication of real-time reactions between biological polypeptides. One or more biomarkers polypeptide or a fragment thereof can be immobilized on a BIAcore chip and multiple agents, *e.g*., blocking antibodies, fusion proteins, peptides, or small molecules, can be tested for binding to the immobilized biomarker polypeptide or fragment thereof. An example of using the BIA technology is described by Fitz et al. (1997) Oncogene 15:613.

The cell-free assays of the present disclosure are amenable to use of both soluble and/or membrane-bound forms of proteins. In the case of cell-free assays in which a membrane-bound form protein is used it may be desirable to utilize a solubilizing agent such that the membrane-bound form of the protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

In one or more embodiments of the above described assay methods, it may be desirable to immobilize either the biomarker polypeptide, the natural binding partner(s) polypeptide of the biomarker, or fragments thereof, to facilitate separation of complexed from uncomplexed forms of one or both of the proteins, as well as to accommodate automation of the assay. Binding of a test compound in the assay can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase-base fusion proteins, can be adsorbed onto glutathione Sepharose® beads (Sigma Chemical, St. Louis, MO) or glutathione derivatized microtiter plates, which are then combined with the test compound, and the mixture incubated under conditions conducive to complex formation (*e.g*., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of binding or activity determined using standard techniques.

In an alternative embodiment, determining the ability of the test compound to modulate the activity of one or more biomarkers of the invention, including one or more biomarkers listed in Table 1 and the Examples, or a fragment thereof, or of natural binding partner(s) thereof can be accomplished by determining the ability of the test compound to modulate the expression or activity of a gene, *e.g.,* nucleic acid, or gene product, *e.g.,* polypeptide, that functions downstream of the interaction. For example, inflammation (*e.g*., cytokine and chemokine) responses can be determined, the activity of the interactor polypeptide on an appropriate target can be determined, or the binding of the interactor to an appropriate target can be determined as previously described.

In another embodiment, modulators of one or more biomarkers of the invention, including one or more biomarkers listed in Table 1 and the Examples, or a fragment thereof, are identified in a method wherein a cell is contacted with a candidate compound and the expression or activity level of the biomarker is determined. The level of expression of biomarker mRNA or polypeptide or fragments thereof in the presence of the candidate compound is compared to the level of expression of biomarker mRNA or polypeptide or fragments thereof in the absence of the candidate compound. The candidate compound can then be identified as a modulator of biomarker expression based on this comparison. For example, when expression of biomarker mRNA or polypeptide or fragments thereof is greater (statistically significantly greater) in the presence of the candidate compound than in its absence, the candidate compound is identified as a stimulator of biomarker expression. Alternatively, when expression of biomarker mRNA or polypeptide or fragments thereof is reduced (statistically significantly less) in the presence of the candidate compound than in its absence, the candidate compound is identified as an inhibitor of biomarker expression. The expression level of biomarker mRNA or polypeptide or fragments thereof in the cells can be determined by methods described herein for detecting biomarker mRNA or polypeptide or fragments thereof.

In yet another aspect of the disclosure, a biomarker of the disclosure, including one or more biomarkers listed in Table 1 and the Examples, or a fragment thereof, can be used as "bait proteins" in a two-hybrid assay or three-hybrid assay (see, *e.g.,* U.S. Pat. No. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8:1693-1696; and Brent WO94/10300), to identify other polypeptides which bind to or interact with the biomarker or fragments thereof and are involved in activity of the biomarkers. Such biomarker-binding proteins are also likely to be involved in the propagation of signals by the biomarker polypeptides or biomarker natural binding partner(s) as, for example, downstream elements of one or more biomarkers -mediated signaling pathway.

The two-hybrid system is based on the modular nature of most transcription factors, which consist of separable DNA-binding and activation domains. Briefly, the assay utilizes two different DNA constructs. In one construct, the gene that codes for one or more biomarkers polypeptide is fused to a gene encoding the DNA binding domain of a known transcription factor *(e.g.,* GAL-4). In the other construct, a DNA sequence, from a library of DNA sequences, that encodes an unidentified polypeptide ("prey" or "sample") is fused to a gene that codes for the activation domain of the known transcription factor. If the "bait" and the "prey" polypeptides are able to interact, *in vivo,* forming one or more biomarkers - dependent complex, the DNA-binding and activation domains of the transcription factor are brought into close proximity. This proximity allows transcription of a reporter gene *(e.g.,* LacZ) which is operably linked to a transcriptional regulatory site responsive to the transcription factor. Expression of the reporter gene can be detected and cell colonies containing the functional transcription factor can be isolated and used to obtain the cloned gene which encodes the polypeptide which interacts with one or more biomarkers polypeptide of the disclosure, including one or more biomarkers listed in Table 1 and the Examples or a fragment thereof.

In another aspect, the disclosure pertains to a combination of two or more of the assays described herein. For example, a modulating agent can be identified using a cell-based or a cell-free assay, and the ability of the agent to modulate the activity of one or more biomarkers polypeptide or a fragment thereof can be confirmed *in vivo, e.g.,* in an animal such as an animal model for cellular transformation and/or tumorigenesis.

This disclosure further pertains to novel agents identified by the above-described screening assays. Accordingly, it is within the scope of this disclosure to further use an agent identified as described herein in an appropriate animal model. For example, an agent identified as described herein can be used in an animal model to determine the efficacy, toxicity, or side effects of treatment with such an agent. Alternatively, an agent identified as described herein can be used in an animal model to determine the mechanism of action of such an agent. Furthermore, this disclosure pertains to uses of novel agents identified by the above-described screening assays for treatments as described herein.

### IV. Uses and Methods of the Disclosure

The biomarkers of the disclosure described herein, including the biomarkers listed in Table 1 and the Examples or fragments thereof, can be used in one or more of the following methods: a) screening assays; b) predictive medicine (e.g., diagnostic assays, prognostic assays, and monitoring of clinical trials); and c) methods of treatment (e.g., therapeutic and prophylactic, *e.g*., by up- or down-modulating the copy number, level of expression, and/or level of activity of the one or more biomarkers).

The biomarkers described herein or agents that modulate the expression and/or activity of such biomarkers can be used, for example, to (a) express one or more biomarkers of the disclosure, including one or more biomarkers listed in Table 1 and the Examples or a fragment thereof *(e.g.,* via a recombinant expression vector in a host cell in gene therapy applications or synthetic nucleic acid molecule), (b) detect biomarker mRNA or a fragment thereof *(e.g.,* in a biological sample) or a genetic alteration in one or more biomarkers gene, and/or (c) modulate biomarker activity, as described further below. The biomarkers or modulatory agents thereof can be used to treat conditions or disorders characterized by insufficient or excessive production of one or more biomarkers polypeptide or fragment thereof or production of biomarker polypeptide inhibitors. In addition, the biomarker polypeptides or fragments thereof can be used to screen for naturally occurring biomarker binding partner(s), to screen for drugs or compounds which modulate biomarker activity, as well as to treat conditions or disorders characterized by insufficient or excessive production of biomarker polypeptide or a fragment thereof or production of biomarker polypeptide forms which have decreased, aberrant or unwanted activity compared to biomarker wild-type polypeptides or fragments.

### A. Screening Assays

In one aspect, the present disclosure relates to a method for preventing in a subject, a disease or condition associated with an unwanted, more than desirable, or less than desirable, expression and/or activity of one or more biomarkers described herein. Subjects at risk for a disease that would benefit from treatment with the claimed agents or methods can be identified, for example, by any one or combination of diagnostic or prognostic assays known in the art and described herein (see, for example, agents and assays described in III. Methods of Selecting Agents and Compositions).

### B. Predictive Medicine

The present disclosure also pertains to the field of predictive medicine in which diagnostic assays, prognostic assays, and monitoring of clinical trials are used for prognostic (predictive) purposes to thereby treat an individual prophylactically. Accordingly, one aspect of the present disclosure relates to diagnostic assays for determining the expression and/or activity level of biomarkers of the disclosure, including biomarkers listed in Table 1 and the Examples or fragments thereof, in the context of a biological sample (*e.g*., blood, serum, cells, or tissue) to thereby determine whether an individual is afflicted with a disease or disorder, or is at risk of developing a disorder, associated with aberrant or unwanted biomarker expression or activity. The present disclosure also provides for prognostic (or predictive) assays for determining whether an individual is at risk of developing a disorder associated with biomarker polypeptide, nucleic acid expression or activity. For example, mutations in one or more biomarkers gene can be assayed in a biological sample.

Such assays can be used for prognostic or predictive purpose to thereby prophylactically treat an individual prior to the onset of a disorder characterized by or associated with biomarker polypeptide, nucleic acid expression or activity.

Another aspect of the disclosure pertains to monitoring the influence of agents *(e.g.,* drugs, compounds, and small nucleic acid-based molecules) on the expression or activity of biomarkers of the disclosure, including biomarkers listed in Table 1 and the Examples, or fragments thereof, in clinical trials. These and other agents are described in further detail in the following sections.

### 1. Diagnostic Assays

The present disclosure provides, in part, methods, systems, and code for accurately classifying whether a biological sample is associated with a metabolic disorder. In some embodiments, the present disclosure is useful for classifying a sample (*e.g*., from a subject) as a sample characterized as having a metabolic disorder using a statistical algorithm and/or empirical data (*e.g.,* the presence or level of one or biomarkers described herein).

An exemplary method for detecting the level of expression or activity of one or more biomarkers of the disclosure, including one or more biomarkers listed in Table 1 and the Examples or fragments thereof, and thus useful for classifying whether a sample is associated with a metabolic disorder, involves obtaining a biological sample from a test subject and contacting the biological sample with a compound or an agent capable of detecting the biomarker (*e.g*., polypeptide or nucleic acid that encodes the biomarker or fragments thereof) such that the level of expression or activity of the biomarker is detected in the biological sample. In some embodiments, the presence or level of at least one, two, three, four, five, six, seven, eight, nine, ten, fifty, hundred, or more biomarkers of the disclosure are determined in the individual's sample. In certain instances, the statistical algorithm is a single learning statistical classifier system. Exemplary statistical analyses are presented in the Examples and can be used in certain embodiments. In other embodiments, a single learning statistical classifier system can be used to classify a sample as a sample associated with a metabolic disorder or a normal sample based upon a prediction or probability value and the presence or level of one or more biomarkers described herein. The use of a single learning statistical classifier system typically classifies the sample as a sample associated with a metabolic disorder with a sensitivity, specificity, positive predictive value, negative predictive value, and/or overall accuracy of at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

Other suitable statistical algorithms are well known to those of skill in the art. For example, learning statistical classifier systems include a machine learning algorithmic technique capable of adapting to complex data sets *(e.g.,* panel of markers of interest) and making decisions based upon such data sets. In some embodiments, a single learning statistical classifier system such as a classification tree (*e.g*., random forest) is used. In other embodiments, a combination of 2, 3, 4, 5, 6, 7, 8, 9, 10, or more learning statistical classifier systems are used, preferably in tandem. Examples of learning statistical classifier systems include, but are not limited to, those using inductive learning (*e.g.,* decision/classification trees such as random forests, classification and regression trees (C&RT), boosted trees, etc.), Probably Approximately Correct (PAC) learning, connectionist learning (*e.g*., neural networks (NN), artificial neural networks (ANN), neuro fuzzy networks (NFN), network structures, perceptrons such as multi-layer perceptrons, multi-layer feed-forward networks, applications of neural networks, Bayesian learning in belief networks, etc.), reinforcement learning (*e.g*., passive learning in a known environment such as naive learning, adaptive dynamic learning, and temporal difference learning, passive learning in an unknown environment, active learning in an unknown environment, learning action-value functions, applications of reinforcement learning, etc.), and genetic algorithms and evolutionary programming. Other learning statistical classifier systems include support vector machines (*e.g*., Kernel methods), multivariate adaptive regression splines (MARS), Levenberg-Marquardt algorithms, Gauss-Newton algorithms, mixtures of Gaussians, gradient descent algorithms, and learning vector quantization (LVQ). In certain embodiments, the method of the present disclosure further comprises sending the classification results to a clinician, *e.g*., an endocrinologist or primary care physician.

In another embodiment, the method of the present disclosure further provides a diagnosis in the form of a probability that the individual has a metabolic disorder. For example, the individual can have about a 0%, 5%, 10%, 15%, 20%, 25%, 30%, 35%, 40%, 45%, 50%, 55%, 60%, 65%, 70%, 75%, 80%, 85%, 90%, 95%, or greater probability of having cancer or a clinical subtype thereof. In yet another embodiment, the method of the present disclosure further provides a prognosis of cancer in the individual. For example, the prognosis can be surgery, development of a clinical subtype of the metabolic disorder, development of one or more symptoms, or recovery from the disease. In some instances, the method of classifying a sample as a sample associated with a metabolic disorder is further based on the symptoms (*e.g.*, clinical factors) of the individual from which the sample is obtained. The symptoms or group of symptoms can be, for example, those associated with the IPI. In some embodiments, the diagnosis of an individual as having a metabolic disorder is followed by administering to the individual a therapeutically effective amount of a drug useful for treating one or more symptoms associated with the metabolic disorder.

In some embodiments, an agent for detecting biomarker mRNA, genomic DNA, or fragments thereof is a labeled nucleic acid probe capable of hybridizing to biomarker mRNA, genomic DNA., or fragments thereof. The nucleic acid probe can be, for example, full-length biomarker nucleic acid, or a portion thereof, such as an oligonucleotide of at least 15, 30, 50, 100, 250 or 500 nucleotides in length and sufficient to specifically hybridize under stringent conditions well known to a skilled artisan to biomarker mRNA or genomic DNA. Other suitable probes for use in the diagnostic assays of the disclosure are described herein. In some embodiments, the nucleic acid probe is designed to detect transcript variants *(i.e.,* different splice forms) of a gene.

A preferred agent for detecting one or more biomarkers listed in Table 1 and the Examples or a fragment thereof is an antibody capable of binding to the biomarker, preferably an antibody with a detectable label. Antibodies can be polyclonal, or more preferably, monoclonal. An intact antibody, or a fragment thereof (*e.g.,* Fab or F(ab')2) can be used. The term "labeled", with regard to the probe or antibody, is intended to encompass direct labeling of the probe or antibody by coupling (*i.e.,* physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells, and biological fluids isolated from a subject, as well as tissues, cells, and fluids present within a subject. That is, the detection method of the disclosure can be used to detect biomarker mRNA, polypeptide, genomic DNA, or fragments thereof, in a biological sample *in vitro* as well as *in vivo.* For example, *in vitro* techniques for detection of biomarker mRNA or a fragment thereof include Northern hybridizations and *in situ* hybridizations. *In vitro* techniques for detection of biomarker polypeptide include enzyme linked immunosorbent assays (ELISAs), Western blots, immunoprecipitations and immunofluorescence. *In vitro* techniques for detection of biomarker genomic DNA or a fragment thereof include Southern hybridizations. Furthermore, *in vivo* techniques for detection of one or more biomarkers polypeptide or a fragment thereof include introducing into a subject a labeled anti- biomarker antibody. For example, the antibody can be labeled with a radioactive marker whose presence and location in a subject can be detected by standard imaging techniques.

In one embodiment, the biological sample contains polypeptide molecules from the test subject. Alternatively, the biological sample can contain mRNA molecules from the test subject or genomic DNA molecules from the test subject. A preferred biological sample is a hematological tissue (*e.g.,* a sample comprising blood, plasma, B cell, bone marrow, *etc.*) sample isolated by conventional means from a subject.

In another embodiment, the methods further involve obtaining a control biological sample from a control subject, contacting the control sample with a compound or agent capable of detecting polypeptide, mRNA, cDNA, small RNAs, mature miRNA, pre-miRNA, pri-miRNA, miRNA*, anti-miRNA, or a miRNA binding site, or a variant thereof, genomic DNA, or fragments thereof of one or more biomarkers listed in Table 1 and the Examples such that the presence of biomarker polypeptide, mRNA, genomic DNA, or fragments thereof, is detected in the biological sample, and comparing the presence of biomarker polypeptide, mRNA, cDNA, small RNAs, mature miRNA, pre-miRNA, pri-miRNA, miRNA*, anti-miRNA, or a miRNA binding site, or a variant thereof, genomic DNA, or fragments thereof in the control sample with the presence of biomarker polypeptide, mRNA, cDNA, small RNAs, mature miRNA, pre-miRNA, pri-miRNA, miRNA*, anti-miRNA, or a miRNA binding site, or a variant thereof, genomic DNA, or fragments thereof in the test sample.

The disclosure also encompasses kits for detecting the presence of a polypeptide, mRNA, cDNA, small RNAs, mature miRNA, pre-miRNA, pri-miRNA, miRNA*, anti-miRNA, or a miRNA binding site, or a variant thereof, genomic DNA, or fragments thereof, of or against one or more biomarkers listed in Table 1 and the Examples in a biological sample. For example, the kit can comprise a labeled compound or agent capable of detecting one or more biomarkers polypeptide, mRNA, cDNA, small RNAs, mature miRNA, pre-miRNA, pri-miRNA, miRNA*, anti-miRNA, or a miRNA binding site, or a variant thereof, genomic DNA, or fragments thereof, in a biological sample; means for determining the amount of the biomarker polypeptide, mRNA, cDNA, small RNAs, mature miRNA, pre-miRNA, pri-miRNA, miRNA*, anti-miRNA, or a miRNA binding site, or a variant thereof, genomic DNA, or fragments thereof,f in the sample; and means for comparing the amount of the biomarker polypeptide, mRNA, cDNA, small RNAs, mature miRNA, pre-miRNA, pri-miRNA, miRNA*, anti-miRNA, or a miRNA binding site, or a variant thereof, genomic DNA, or fragments thereof, in the sample with a standard. The compound or agent can be packaged in a suitable container. The kit can further comprise instructions for using the kit to detect the biomarker polypeptide, mRNA, cDNA, small RNAs, mature miRNA, pre-miRNA, pri-miRNA, miRNA*, anti-miRNA, or a miRNA binding site, or a variant thereof, genomic DNA, or fragments thereof.

In some embodiments, therapies tailored to treat stratified patient populations based on the described diagnostic assays are further provided.

### 2. Prognostic Assays

The diagnostic methods described herein can furthermore be utilized to identify subjects having or at risk of developing a disease or disorder associated with aberrant expression or activity of one or more biomarkers of the disclosure, including one or more biomarkers listed in Table 1 and the Examples, or a fragment thereof. As used herein, the term "aberrant" includes biomarker expression or activity levels which deviates from the normal expression or activity in a control.

The assays described herein, such as the preceding diagnostic assays or the following assays, can be utilized to identify a subject having or at risk of developing a disorder associated with a misregulation of biomarker activity or expression, such as in a metabolic disorder. Alternatively, the prognostic assays can be used to identify a subject having or at risk for developing a disorder associated with a misregulation of biomarker activity or expression. Thus, the present disclosure provides a method for identifying and/or classifying a disease associated with aberrant expression or activity of one or more biomarkers of the disclosure, including one or more biomarkers listed in Table 1 and the Examples, or a fragment thereof. Furthermore, the prognostic assays described herein can be used to determine whether a subject can be administered an agent *(e.g.,* an agonist, antagonist, peptidomimetic, polypeptide, peptide, nucleic acid, small molecule, or other drug candidate) to treat a disease or disorder associated with aberrant biomarker expression or activity. For example, such methods can be used to determine whether a subject can be effectively treated with an agent for treating the metabolic disorder. Thus, the present disclosure provides methods for determining whether a subject can be effectively treated with an agent for a disease associated with aberrant biomarker expression or activity in which a test sample is obtained and biomarker polypeptide or nucleic acid expression or activity is detected (*e.g*., wherein a significant increase or decrease in biomarker polypeptide or nucleic acid expression or activity relative to a control is diagnostic for a subject that can be administered the agent to treat a disorder associated with aberrant biomarker expression or activity). In some embodiments, significant increase or decrease in biomarker expression or activity comprises at least 2 2.1, 2.2, 2.3, 2.4, 2.5, 2.6, 2.7, 2.8, 2.9, 3, 3.5, 4, 4.5, 5, 5.5, 6, 6.5, 7, 7.5, 8, 8.5, 9, 9.5, 10, 10.5, 11, 12, 13, 14, 15, 16, 17, 18, 19, 20 times or more higher or lower, respectively, than the expression activity or level of the marker in a control sample.

The methods of the disclosure can also be used to detect genetic alterations in one or more biomarkers of the disclosure, including one or more biomarkers listed in Table 1 and the Examples or a fragment thereof, thereby determining if a subject with the altered biomarker is at risk for developing a metabolic disorder characterized by aberrant biomarker activity or expression levels. In preferred embodiments, the methods include detecting, in a sample of cells from the subject, the presence or absence of a genetic alteration characterized by at least one alteration affecting the integrity of a gene encoding one or more biomarkers polypeptide, or the mis-expression of the biomarker (*e.g*., mutations and/or splice variants). For example, such genetic alterations can be detected by ascertaining the existence of at least one of 1) a deletion of one or more nucleotides from one or more biomarkers gene, 2) an addition of one or more nucleotides to one or more biomarkers gene, 3) a substitution of one or more nucleotides of one or more biomarkers gene, 4) a chromosomal rearrangement of one or more biomarkers gene, 5) an alteration in the level of a messenger RNA transcript of one or more biomarkers gene, 6) aberrant modification of one or more biomarkers gene, such as of the methylation pattern of the genomic DNA, 7) the presence of a non-wild type splicing pattern of a messenger RNA transcript of one or more biomarkers gene, 8) a non-wild type level of one or more biomarkers polypeptide, 9) allelic loss of one or more biomarkers gene, and 10) inappropriate post-translational modification of one or more biomarkers polypeptide. As described herein, there are a large number of assays known in the art which can be used for detecting alterations in one or more biomarkers gene. A preferred biological sample is a tissue or serum sample isolated by conventional means from a subject.

In certain embodiments, detection of the alteration involves the use of a probe/primer in a polymerase chain reaction (PCR) (see, *e.g.,* U.S. Patents 4,683,195 and 4,683,202), such as anchor PCR or RACE PCR, or, alternatively, in a ligation chain reaction (LCR) (see, *e.g.,* Landegran et al. (1988) Science 241:1077-1080; and Nakazawa et al. (1994) Proc. Natl. Acad. Sci. USA 91:360-364), the latter of which can be particularly useful for detecting point mutations in one or more biomarkers gene (see Abravaya et al. (1995) Nucleic Acids Res. 23:675-682). This method can include the steps of collecting a sample of cells from a subject, isolating nucleic acid (*e.g*., genomic DNA, mRNA, cDNA, small RNA, mature miRNA, pre-miRNA, pri-miRNA, miRNA*, anti-miRNA, or a miRNA binding site, or a variant thereof) from the cells of the sample, contacting the nucleic acid sample with one or more primers which specifically hybridize to one or more biomarkers gene of the disclosure, including the biomarker genes listed in Table 1 and the Examples, or fragments thereof, under conditions such that hybridization and amplification of the biomarker gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self-sustained sequence replication (Guatelli, J. C. et al. (1990) Proc. Natl. Acad. Sci. USA 87:1874-1878), transcriptional amplification system (Kwoh, D. Y. et al. (1989) Proc. Natl. Acad. Sci. USA 86:1173-1177), Q-Beta Replicase (Lizardi, P. M. et al. (1988) Bio-Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in one or more biomarkers gene of the disclosure, including one or more biomarkers listed in Table 1 and the Examples, or a fragment thereof, from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Patent 5,498,531) can be used to score for the presence of specific mutations by development or loss of a ribozyme cleavage site.

In other embodiments, genetic mutations in one or more biomarkers gene of the disclosure, including a gene listed in Table 1 and the Examples, or a fragment thereof, can be identified by hybridizing a sample and control nucleic acids, *e.g.,* DNA, RNA, mRNA, small RNA, cDNA, mature miRNA, pre-miRNA, pri-miRNA, miRNA*, anti-miRNA, or a miRNA binding site, or a variant thereof, to high density arrays containing hundreds or thousands of oligonucleotide probes (Cronin, M. T. et al. (1996) Hum. Mutat. 7:244-255; Kozal, M. J. et al. (1996) Nat. Med. 2:753-759). For example, genetic mutations in one or more biomarkers can be identified in two dimensional arrays containing light-generated DNA probes as described in Cronin *et al.* (1996) supra. Briefly, a first hybridization array of probes can be used to scan through long stretches of DNA in a sample and control to identify base changes between the sequences by making linear arrays of sequential, overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence one or more biomarkers gene of the disclosure, including a gene listed in Table 1 and the Examples, or a fragment thereof, and detect mutations by comparing the sequence of the sample biomarker gene with the corresponding wild-type (control) sequence. Examples of sequencing reactions include those based on techniques developed by Maxam and Gilbert (1977) Proc. Natl. Acad. Sci. USA 74:560 or Sanger (1977) Proc. Natl. Acad Sci. USA 74:5463. It is also contemplated that any of a variety of automated sequencing procedures can be utilized when performing the diagnostic assays (Naeve, C. W. (1995) Biotechniques 19:448-53), including sequencing by mass spectrometry (see, *e.g.,* PCT International Publication No. WO 94/16101; Cohen et al. (1996) Adv. Chromatogr. 36:127-162; and Griffin et al. (1993) Appl. Biochem. Biotechnol. 38:147-159).

Other methods for detecting mutations in one or more biomarkers gene of the disclosure, including a gene listed in Table 1 and the Examples, or fragments thereof, include methods in which protection from cleavage agents is used to detect mismatched bases in RNA/RNA or RNA/DNA heteroduplexes (Myers et al. (1985) Science 230:1242). In general, the art technique of "mismatch cleavage" starts by providing heteroduplexes formed by hybridizing (labeled) RNA or DNA containing the wild-type sequence with potentially mutant RNA or DNA obtained from a tissue sample. The double-stranded duplexes are treated with an agent which cleaves single-stranded regions of the duplex such as which will exist due to base pair mismatches between the control and sample strands. For instance, RNA/DNA duplexes can be treated with RNase and DNA/DNA hybrids treated with SI nuclease to enzymatically digest the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al. (1988) Proc. Natl. Acad. Sci. USA 85:4397 and Saleeba et al. (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in biomarker genes of the disclosure, including genes listed in Table 1 and the Examples, or fragments thereof, obtained from samples of cells. For example, the mutY enzyme of E. coli cleaves A at G/A mismatches and the thymidine DNA glycosylase from HeLa cells cleaves T at G/T mismatches (Hsu et al. (1994) Carcinogenesis 15:1657-1662). The duplex is treated with a DNA mismatch repair enzyme, and the cleavage products, if any, can be detected from electrophoresis protocols or the like. See, for example, U.S. Patent 5,459,039.

In other embodiments, alterations in electrophoretic mobility will be used to identify mutations in biomarker genes of the disclosure, including genes listed in Table 1 and the Examples, or fragments thereof. For example, single strand conformation polymorphism (SSCP) may be used to detect differences in electrophoretic mobility between mutant and wild type nucleic acids (Orita et al. (1989) Proc Natl. Acad. Sci USA 86:2766; see also Cotton (1993) Mutat. Res. 285:125-144 and Hayashi (1992) Genet. Anal. Tech. Appl. 9:73-79). Single-stranded DNA fragments of sample and control nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet. 7:5).

In yet another embodiment the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to ensure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) Biophys. Chem. 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) Nature 324:163; Saiki et al. (1989) Proc. Natl. Acad. Sci. USA 86:6230). Such allele specific oligonucleotides are hybridized to PCR amplified target DNA or a number of different mutations when the oligonucleotides are attached to the hybridizing membrane and hybridized with labeled target DNA. In some embodiments, the hybridization reactions can occur using biochips, microarrays, etc., or other array technology that are well known in the art.

Alternatively, allele specific amplification technology which depends on selective PCR amplification may be used in conjunction with the instant disclosure. Oligonucleotides used as primers for specific amplification may carry the mutation of interest in the center of the molecule (so that amplification depends on differential hybridization) (Gibbs et al. (1989) Nucleic Acids Res. 17:2437-2448) or at the extreme 3' end of one primer where, under appropriate conditions, mismatch can prevent, or reduce polymerase extension (Prossner (1993) Tibtech 11:238). In addition it may be desirable to introduce a novel restriction site in the region of the mutation to create cleavage-based detection (Gasparini et al. (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing prepackaged diagnostic kits comprising at least one probe nucleic acid or antibody reagent described herein, which may be conveniently used, *e.g.,* in clinical settings to diagnose patients exhibiting symptoms or family history of a disease or illness involving one or more biomarkers of the disclosure, including one or more biomarkers listed in Table 1 and the Examples, or fragments thereof.

### 3. Monitoring of Effects During Clinical Trials

Monitoring the influence of agents (*e.g*., drugs) on the expression or activity of one or more biomarkers of the disclosure, including one or more biomarkers listed in Table 1 and the Examples, or a fragment thereof can be applied not only in basic drug screening, but also in clinical trials. For example, the effectiveness of an agent determined by a screening assay as described herein to increase expression and/or activity of one or more biomarkers of the disclosure, including one or more biomarkers listed in Table 1 and the Examples or a fragment thereof, can be monitored in clinical trials of subjects exhibiting decreased expression and/or activity of one or more biomarkers of the disclosure, including one or more biomarkers of the disclosure, including one or more biomarkers listed in Table 1 and the Examples, or a fragment thereof, relative to a control reference. Alternatively, the effectiveness of an agent determined by a screening assay to decrease expression and/or activity of one or more biomarkers of the disclosure, including one or more biomarkers listed in Table 1 and the Examples, or a fragment thereof, can be monitored in clinical trials of subjects exhibiting decreased expression and/or activity of the biomarker of the disclosure, including one or more biomarkers listed in Table 1 and the Examples or a fragment thereof relative to a control reference. In such clinical trials, the expression and/or activity of the biomarker can be used as a "read out" or marker of the phenotype of a particular cell.

In some embodiments, the present disclosure provides a method for monitoring the effectiveness of treatment of a subject with an agent (*e.g.,* an agonist, antagonist, peptidomimetic, polypeptide, peptide, nucleic acid, small molecule, or other drug candidate identified by the screening assays described herein) including the steps of (i) obtaining a pre-administration sample from a subject prior to administration of the agent; (ii) detecting the level of expression and/or activity of one or more biomarkers of the disclosure, including one or more biomarkers listed in Table 1 and the Examples or fragments thereof in the preadministration sample; (iii) obtaining one or more post-administration samples from the subject; (iv) detecting the level of expression or activity of the biomarker in the post-administration samples; (v) comparing the level of expression or activity of the biomarker or fragments thereof in the pre-administration sample with the that of the biomarker in the post administration sample or samples; and (vi) altering the administration of the agent to the subject accordingly. For example, increased administration of the agent may be desirable to increase the expression or activity of one or more biomarkers to higher levels than detected *(e.g.,* to increase the effectiveness of the agent.) Alternatively, decreased administration of the agent may be desirable to decrease expression or activity of the biomarker to lower levels than detected *(e.g.,* to decrease the effectiveness of the agent). According to such an embodiment, biomarker expression or activity may be used as an indicator of the effectiveness of an agent, even in the absence of an observable phenotypic response.

### D. Methods of Treatmen (not claimed)

The present disclosure provides for both prophylactic and therapeutic methods of treating a subject at risk of (or susceptible to) a disorder characterized by insufficient or excessive production of biomarkers of the present disclosure, including biomarkers listed in Table 1 and the Examples or fragments thereof, which have aberrant expression or activity compared to a control. In some embodiments, modulating the biomarkers of the present disclosure increases differentiated brown fat cells, increases in thermogenesis, and/or increases energy expenditure and can therefore be used to treat metabolic disorders such as obesity, cardiac hypertrophy, type II diabetes, and in need of more excersise. In other embodiments, decreases in differentiated brown fat cells, decreased thermogenesis, and/or decrease energy expenditure result and can therefore be used to treat the effects of such conditions as cachexia, anorexia, and obesity-associated cancer. Moreover, agents of the disclosure described herein can be used to detect and isolate the biomarkers or fragments thereof, regulate the bioavailability of the biomarkers or fragments thereof, and modulate biomarker expression levels or activity.

The term "sufficient to activate" is intended to encompass any increase in expression and/or activity of such biomarkers or fragments thereof that promotes, activates, stimulates, enhances, or results in modulated brown fat cell induction, thermogenesis, or energy expenditure.

### 1. Prophylactic Method (not claimed)

In one aspect, the disclosure provides a method for preventing in a subject, a disease or condition associated with an aberrant expression or activity of one or more biomarkers of the disclosure, including one or more biomarkers listed in Table 1 and the Examples or a fragment thereof, by administering to the subject an agent which modulates biomarker expression or at least one activity of the biomarker. Subjects at risk for a disease or disorder which is caused or contributed to by aberrant biomarker expression or activity can be identified by, for example, any or a combination of diagnostic or prognostic assays as described herein. Administration of a prophylactic agent can occur prior to the manifestation of symptoms characteristic of the biomarker expression or activity aberrancy, such that a disease or disorder is prevented or, alternatively, delayed in its progression.

### 2. Therapeutic Method (not claimed)

Another aspect of the disclosure pertains to methods of modulating the expression or activity or interaction with natural binding partner(s) of one or more biomarkers of the disclosure, including one or more biomarkers listed in Table 1 and the Examples or fragments thereof, for therapeutic purposes. The biomarkers of the disclosure have been demonstrated to correlate with metabolic disorders. Accordingly, the activity and/or expression of the biomarker, as well as the interaction between one or more biomarkers or a fragment thereof and its natural binding partner(s) or a fragment(s) thereof can be modulated in order to modulate the immune response.

As used herein, the term "agent" and "therapeutic agent" is defined broadly as anything that cells from a subject having a metabolic disorder may be exposed to in a therapeutic protocol.

Modulatory methods of the disclosure involve contacting a cell with one or more biomarkers of the disclosure, including one or more biomarkers of the disclosure, including one or more biomarkers listed in Table 1 and the Examples or a fragment thereof or agent that modulates one or more of the activities of biomarker activity associated with the cell. In some embodiments, the biomarkers are or encode secreted molecules such that contacting a cell with one or more biomarkers of the disclosure or agent that modulates one or more of the activities of biomarker activity is unnecessary and contact with a bodily fluid *(e.g.,* blood, serum, lung pleural fluid, etc.) is sufficient. An agent that modulates biomarker activity can be an agent as described herein, such as a nucleic acid or a polypeptide, a naturally-occurring binding partner of the biomarker, an antibody against the biomarker, a combination of antibodies against the biomarker and antibodies against other immune related targets, one or more biomarkers agonist or antagonist, a peptidomimetic of one or more biomarkers agonist or antagonist, one or more biomarkers peptidomimetic, other small molecule, or small RNA directed against or a mimic of one or more biomarkers nucleic acid gene expression product.

An agent that modulates the expression of one or more biomarkers of the disclosure, including one or more biomarkers of the disclosure, including one or more biomarkers listed in Table 1 and the Examples or a fragment thereof is, *e.g.,* an antisense nucleic acid molecule, RNAi molecule, shRNA, mature miRNA, pre-miRNA, pri-miRNA, miRNA*, anti-miRNA, or a miRNA binding site, or a variant thereof, or other small RNA molecule, triplex oligonucleotide, ribozyme, or recombinant vector for expression of one or more biomarkers polypeptide. For example, an oligonucleotide complementary to the area around one or more biomarkers polypeptide translation initiation site can be synthesized. One or more antisense oligonucleotides can be added to cell media, typically at 200 µg/ml, or administered to a patient to prevent the synthesis of one or more biomarkers polypeptide. The antisense oligonucleotide is taken up by cells and hybridizes to one or more biomarkers mRNA to prevent translation. Alternatively, an oligonucleotide which binds double-stranded DNA to form a triplex construct to prevent DNA unwinding and transcription can be used. As a result of either, synthesis of biomarker polypeptide is blocked. When biomarker expression is modulated, preferably, such modulation occurs by a means other than by knocking out the biomarker gene.

Agents which modulate expression, by virtue of the fact that they control the amount of biomarker in a cell, also modulate the total amount of biomarker activity in a cell.

In one embodiment, the agent stimulates one or more activities of one or more biomarkers of the disclosure, including one or more biomarkers listed in Table 1 and the Examples or a fragment thereof. Examples of such stimulatory agents include active biomarker polypeptide or a fragment thereof and a nucleic acid molecule encoding the biomarker or a fragment thereof that has been introduced into the cell *(e.g.,* cDNA, mRNA, shRNAs, siRNAs, small RNAs, mature miRNA, pre-miRNA, pri-miRNA, miRNA*, anti-miRNA, or a miRNA binding site, or a variant thereof, or other functionally equivalent molecule known to a skilled artisan). In another embodiment, the agent inhibits one or more biomarker activities. In one embodiment, the agent inhibits or enhances the interaction of the biomarker with its natural binding partner(s). Examples of such inhibitory agents include antisense nucleic acid molecules, anti-biomarker antibodies, biomarker inhibitors, and compounds identified in the screening assays described herein.

These modulatory methods can be performed *in vitro* (*e.g.,* by contacting the cell with the agent) or, alternatively, by contacting an agent with cells *in vivo* (*e.g.,* by administering the agent to a subject). As such, the present disclosure provides methods of treating an individual afflicted with a condition or disorder that would benefit from up- or down-modulation of one or more biomarkers of the disclosure listed in Table 1 and the Examples or a fragment thereof, *e.g.,* a disorder characterized by unwanted, insufficient, or aberrant expression or activity of the biomarker or fragments thereof. In one embodiment, the method involves administering an agent (*e.g.,* an agent identified by a screening assay described herein), or combination of agents that modulates (*e.g*., upregulates or downregulates) biomarker expression or activity. In another embodiment, the method involves administering one or more biomarkers polypeptide or nucleic acid molecule as therapy to compensate for reduced, aberrant, or unwanted biomarker expression or activity.

Stimulation of biomarker activity is desirable in situations in which the biomarker is abnormally downregulated and/or in which increased biomarker activity is likely to have a beneficial effect. Likewise, inhibition of biomarker activity is desirable in situations in which biomarker is abnormally upregulated and/or in which decreased biomarker activity is likely to have a beneficial effect.

Another aspect of the disclosure (not claimed) relates to a method for inducing brown fat cell differentiation in a mammal comprising expressing a biomarker listed in Table 1 or the Examples, or fragments or orthologs thereof, in a mammal and monitoring the differentiation of brown fat cells in the mammal. Increased brown adipose tissue in the mammal will warm up the body and blood of the mammal resulting in an increased energy expenditure from the cells. The increased energy expenditure will increase the metabolic rate of the subject and may be used for the treatment and/or prevention of certain metabolic disorders. The opposite effects can be achieved by decreasing brown adipose tissue in the mammal. The induction of brown fat cells may be monitored by analyzing 1) the expression of one or more of cidea, adiponectin (adipoq), adipsin, otopetrin, type II deiodinase, cig30, ppar gamma 2, pgc1α, ucp1, elovl3, cAMP, Prdm16, cytochrome C, cox4i1, coxIII, cox5b, cox7a1, cox8b, glut4, atpase b2, cox II, atp5o, ndufb5, ap2, ndufs1, GRP109A, acylCoA-thioesterase 4, EARA1, claudin1, PEPCK, fgf21, acylCoA-thioesterase 3, and dio; 2) cellular respiration *(i.e.,* basal, total, and/or uncoupled respiration); 3) thermogenesis of adipose cells; 4) insulin sensitivity of adipose, muscle and/or hepatic cells; 5) hepatosteatosis, obesity, type II diabetes, and/or appetite; 6) insulin secretion of pancreatic beta cells; 7) cardiac function; 8) muscle hypoplasia; 9) growth and effects of obesity-associated cancer, cachexia, and anorexia; 10) differentiation, generation, and/or proliferation of adipose cells, 11) blood glucose levels, and 12) symptoms associated with a disorder.

The effectiveness of any particular therapeutic agent to treat a metabolic disorder can be monitored by comparing two or more samples obtained from a subject undergoing treatment. In general, it is preferable to obtain a first sample from the subject prior to beginning therapy and one or more samples during treatment. In such a use, a baseline of expression of cells from subjects with the metabolic disorder prior to therapy is determined and then changes in the baseline state of expression of cells from subjects with the metabolic disorder is monitored during the course of therapy. Alternatively, two or more successive samples obtained during treatment can be used without the need of a pre-treatment baseline sample. In such a use, the first sample obtained from the subject is used as a baseline for determining whether the expression of cells from subjects with the metabolic disorder is increasing or decreasing.

In addition, these modulatory agents can also be administered in combination therapy with, *e.g*., chemotherapeutic agents, hormones, antiangiogens, radiolabelled, compounds, or with surgery, cryotherapy, and/or radiotherapy. The preceding treatment methods can be administered in conjunction with other forms of conventional therapy (e.g., standard-of-care treatments for cancer well known to the skilled artisan), either consecutively with, pre- or post-conventional therapy. For example, these modulatory agents can be administered with a therapeutically effective dose of chemotherapeutic agent. In another embodiment, these modulatory agents are administered in conjunction with chemotherapy to enhance the activity and efficacy of the chemotherapeutic agent. The Physicians' Desk Reference (PDR) discloses dosages of chemotherapeutic agents that have been used in the treatment of various cancers. The dosing regimen and dosages of these aforementioned chemotherapeutic drugs that are therapeutically effective will depend on the particular cancer *(e.g.,* head, neck, and/or lung cancers), being treated, the extent of the disease and other factors familiar to the physician of skill in the art and can be determined by the physician.

In some embodiments, it is useful to downregulate immune responses. In other embodiments, it is useful to upregulate immune responses.

### IV. Pharmaceutical Compositions

In another aspect, the present disclosure provides pharmaceutically acceptable compositions which comprise a therapeutically-effective amount of an agent that modulates (e.g., increases or decreases) a biomarker listed in Table 1 or the Examples, or orthologs or fragments thereof, formulated together with one or more pharmaceutically acceptable carriers (additives) and/or diluents. As described in detail below, the pharmaceutical compositions of the present disclosure may be specially formulated for administration in solid or liquid form, including those adapted for the following: (1) oral administration, for example, drenches (aqueous or non-aqueous solutions or suspensions), tablets, boluses, powders, granules, pastes; (2) parenteral administration, for example, by subcutaneous, intramuscular or intravenous injection as, for example, a sterile solution or suspension; (3) topical application, for example, as a cream, ointment or spray applied to the skin; (4) intravaginally or intrarectally, for example, as a pessary, cream or foam; or (5) aerosol, for example, as an aqueous aerosol, liposomal preparation or solid particles containing the compound.

The phrase "therapeutically-effective amount" as used herein means that amount of an agent that modulates (*e.g*., enhances or inhibits) the expression and/or activity of a biomarker listed in Table 1 or the Examples, or orthologs or fragments thereof, in a manner that is effective for producing some desired therapeutic effect at a reasonable benefit/risk ratio.

The phrase "pharmaceutically acceptable" is employed herein to refer to those agents, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, solvent or encapsulating material, involved in carrying or transporting the subject chemical from one organ, or portion of the body, to another organ, or portion of the body. Each carrier must be "acceptable" in the sense of being compatible with the other ingredients of the formulation and not injurious to the subject. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The term "pharmaceutically-acceptable salts" refers to the relatively non-toxic, inorganic and organic acid addition salts of the agents that modulates the expression and/or activity of a biomarker of the present disclosure and/or its interaction with its receptor. These salts can be prepared in situ during the final isolation and purification of the respiration uncoupling agents, or by separately reacting a purified respiration uncoupling agent in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like (See, for example, Berge et al. (1977) "Pharmaceutical Salts", J. Pharm. Sci. 66:1-19).

In other cases, the agents useful in the methods of the present disclosure may contain one or more acidic functional groups and, thus, are capable of forming pharmaceutically-acceptable salts with pharmaceutically-acceptable bases. The term "pharmaceutically-acceptable salts" in these instances refers to the relatively non-toxic, inorganic and organic base addition salts of agents that modulates the expression and/or activity of a biomarker of the present disclosure and/or its interaction with its receptor. These salts can likewise be prepared in situ during the final isolation and purification of the respiration uncoupling agents, or by separately reacting the purified respiration uncoupling agent in its free acid form with a suitable base, such as the hydroxide, carbonate or bicarbonate of a pharmaceutically-acceptable metal cation, with ammonia, or with a pharmaceutically-acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like (see, for example, Berge *et al., supra).*

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations useful in the methods of the present disclosure include those suitable for oral, nasal, topical (including buccal and sublingual), rectal, vaginal, aerosol and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, the particular mode of administration. The amount of active ingredient, which can be combined with a carrier material to produce a single dosage form will generally be that amount of the compound which produces a therapeutic effect. Generally, out of one hundred per cent, this amount will range from about 1 % to about 99% of active ingredient, preferably from about 5% to about 70%, most preferably from about 10% to about 30%.

Methods of preparing these formulations or compositions include the step of bringing into association an agent that modulates the expression and/or activity of a biomarker of the present disclosure and/or its interaction with its receptor, with the carrier and, optionally, one or more accessory ingredients. In general, the formulations are prepared by uniformly and intimately bringing into association a respiration uncoupling agent with liquid carriers, or finely divided solid carriers, or both, and then, if necessary, shaping the product.

Formulations suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a respiration uncoupling agent as an active ingredient. A compound may also be administered as a bolus, electuary or paste.

In solid dosage forms for oral administration (capsules, tablets, pills, dragees, powders, granules and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered peptide or peptidomimetic moistened with an inert liquid diluent.

Tablets, and other solid dosage forms, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions, which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions, which can be used include polymeric substances and waxes. The active ingredient can also be in micro-encapsulated form, if appropriate, with one or more of the above-described excipients.

Liquid dosage forms for oral administration include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the active agent may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations for rectal or vaginal administration may be presented as a suppository, which may be prepared by mixing one or more respiration uncoupling agents with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active agent.

Formulations which are suitable for vaginal administration also include pessaries, tampons, creams, gels, pastes, foams or spray formulations containing such carriers as are known in the art to be appropriate.

Dosage forms for the topical or transdermal administration of an agent that modulates the expression and/or activity of a biomarker of the present disclosure and/or its interaction with its receptor include powders, sprays, ointments, pastes, creams, lotions, gels, solutions, patches and inhalants. The active component may be mixed under sterile conditions with a pharmaceutically-acceptable carrier, and with any preservatives, buffers, or propellants which may be required.

The ointments, pastes, creams and gels may contain, in addition to a respiration uncoupling agent, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to an agent that modulates the expression and/or activity of a biomarker of the present disclosure and/or its interaction with its receptor, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

The agent that modulates the expression and/or activity of a biomarker of the present disclosure and/or its interaction with its receptor, can be alternatively administered by aerosol. This is accomplished by preparing an aqueous aerosol, liposomal preparation or solid particles containing the compound. A nonaqueous (*e.g*., fluorocarbon propellant) suspension could be used. Sonic nebulizers are preferred because they minimize exposing the agent to shear, which can result in degradation of the compound.

Ordinarily, an aqueous aerosol is made by formulating an aqueous solution or suspension of the agent together with conventional pharmaceutically acceptable carriers and stabilizers. The carriers and stabilizers vary with the requirements of the particular compound, but typically include nonionic surfactants (Tweens, Pluronics, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

Transdermal patches have the added advantage of providing controlled delivery of a respiration uncoupling agent to the body. Such dosage forms can be made by dissolving or dispersing the agent in the proper medium. Absorption enhancers can also be used to increase the flux of the peptidomimetic across the skin. The rate of such flux can be controlled by either providing a rate controlling membrane or dispersing the peptidomimetic in a polymer matrix or gel.

Ophthalmic formulations, eye ointments, powders, solutions and the like, are also contemplated as being within the scope of this disclosure.

Pharmaceutical compositions of this disclosure suitable for parenteral administration comprise one or more respiration uncoupling agents in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the disclosure include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of an agent in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions, which are compatible with body tissue.

When agents of the present disclosure are administered as pharmaceuticals, to humans and animals, they can be given per se or as a pharmaceutical composition containing, for example, 0.1 to 99.5% (more preferably, 0.5 to 90%) of active ingredient in combination with a pharmaceutically acceptable carrier.

Actual dosage levels of the active ingredients in the pharmaceutical compositions of this disclosure may be determined by the methods of the present disclosure so as to obtain an amount of the active ingredient, which is effective to achieve the desired therapeutic response for a particular subject, composition, and mode of administration, without being toxic to the subj ect.

The nucleic acid molecules of the disclosure can be inserted into vectors and used as gene therapy vectors. Gene therapy vectors can be delivered to a subject by, for example, intravenous injection, local administration (see U.S. Pat. No. 5,328,470) or by stereotactic injection (see *e.g.,* Chen et al. (1994) Proc. Natl. Acad. Sci. USA 91:3054 3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

### V. Administration of Agents (not claimed)

The diagnostic, prognostic, prevention, and/or treatment modulating agents of the present disclosure are administered to subjects in a biologically compatible form suitable for pharmaceutical administration *in vivo,* to either enhance or suppress thermogenesis and modulate metabolic responses. By "biologically compatible form suitable for administration *in vivo"* is meant a form of the protein to be administered in which any toxic effects are outweighed by the therapeutic effects of the protein. The term "subject" is intended to include living organisms in which an immune response can be elicited, e.g., mammals. Examples of subjects include humans, dogs, cats, mice, rats, and transgenic species thereof. Administration of an agent as described herein can be in any pharmacological form including a therapeutically active amount of an agent alone or in combination with a pharmaceutically acceptable carrier.

Administration of a therapeutically active amount of the therapeutic composition of the present disclosure is defined as an amount effective, at dosages and for periods of time necessary, to achieve the desired result. For example, a therapeutically active amount of a blocking antibody may vary according to factors such as the disease state, age, sex, and weight of the individual, and the ability of peptide to elicit a desired response in the individual. Dosage regimens can be adjusted to provide the optimum therapeutic response. For example, several divided doses can be administered daily or the dose can be proportionally reduced as indicated by the exigencies of the therapeutic situation.

The agents of the disclosure described herein can be administered in a convenient manner such as by injection (subcutaneous, intravenous, etc.), oral administration, inhalation, transdermal application, or rectal administration. Depending on the route of administration, the active compound can be coated in a material to protect the compound from the action of enzymes, acids and other natural conditions which may inactivate the compound. For example, for administration of agents, by other than parenteral administration, it may be desirable to coat the agent with, or co-administer the agent with, a material to prevent its inactivation.

While daily dosing or multiple doses of PTH-related and EGF-related molecules of the present disclosure have been optimized for modulating bone-related responses (*e.g.*, increasing osteoblast number, bone volume, and bone mineral density) based on the physiological timecourse of bone-related responses, prolonged and sustained exposure of such molecules using less frequent dosing and/or formulations having increased *in vivo* half-lives are superior for treating metabolic disorders. For example, administration that is twice weekly, semi-weekly, weekly, biweekly, monthly, bimonthly, semi-annually, and the like may be optimal for achieiving the sustained exposure of such molecules for modulating metabolic disorders (*e.g*., administration less frequently than once per day).

For example, US Pat. Publs. 2005-0124537 and 2013-0116180 provide art-recognized methods of generating longer-acting effects of PTH-related molecules.

An agent can be administered to an individual in an appropriate carrier, diluent or adjuvant, co-administered with enzyme inhibitors or in an appropriate carrier such as liposomes. Pharmaceutically acceptable diluents include saline and aqueous buffer solutions. Adjuvant is used in its broadest sense and includes any immune stimulating compound such as interferon. Adjuvants contemplated herein include resorcinols, non-ionic surfactants such as polyoxyethylene oleyl ether and n-hexadecyl polyethylene ether. Enzyme inhibitors include pancreatic trypsin inhibitor, diisopropylfluorophosphate (DEEP) and trasylol. Liposomes include water-in-oil-in-water emulsions as well as conventional liposomes (Sterna et al. (1984) J. Neuroimmunol. 7:27*).*

The agent may also be administered parenterally or intraperitoneally. Dispersions can also be prepared in glycerol, liquid polyethylene glycols, and mixtures thereof, and in oils. Under ordinary conditions of storage and use, these preparations may contain a preservative to prevent the growth of microorganisms.

Pharmaceutical compositions of agents suitable for injectable use include sterile aqueous solutions (where water soluble) or dispersions and sterile powders for the extemporaneous preparation of sterile injectable solutions or dispersion. In all cases the composition will preferably be sterile and must be fluid to the extent that easy syringeability exists. It will preferably be stable under the conditions of manufacture and storage and preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it is preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating an agent of the disclosure (*e.g.,* an antibody, peptide, fusion protein or small molecule) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the agent plus any additional desired ingredient from a previously sterile-filtered solution thereof.

When the agent is suitably protected, as described above, the protein can be orally administered, for example, with an inert diluent or an assimilable edible carrier. As used herein "pharmaceutically acceptable carrier" includes any and all solvents, dispersion media, coatings, antibacterial and antifungal agents, isotonic and absorption delaying agents, and the like. The use of such media and agents for pharmaceutically active substances is well known in the art. Except insofar as any conventional media or agent is incompatible with the active compound, use thereof in the therapeutic compositions is contemplated. Supplementary active compounds can also be incorporated into the compositions.

It is especially advantageous to formulate parenteral compositions in dosage unit form for ease of administration and uniformity of dosage. "Dosage unit form", as used herein, refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the disclosure are dictated by, and directly dependent on, (a) the unique characteristics of the active compound and the particular therapeutic effect to be achieved, and (b) the limitations inherent in the art of compounding such an active compound for the treatment of sensitivity in individuals.

In one embodiment, an agent of the disclosure is an antibody. As defined herein, a therapeutically effective amount of antibody (*i.e.,* an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The skilled artisan will appreciate that certain factors may influence the dosage required to effectively treat a subject, including but not limited to the severity of the disease or disorder, previous treatments, the general health and/or age of the subject, and other diseases present. Moreover, treatment of a subject with a therapeutically effective amount of an antibody can include a single treatment or, preferably, can include a series of treatments. In a preferred example, a subject is treated with antibody in the range of between about 0.1 to 20 mg/kg body weight, one time per week for between about 1 to 10 weeks, preferably between 2 to 8 weeks, more preferably between about 3 to 7 weeks, and even more preferably for about 4, 5, or 6 weeks. It will also be appreciated that the effective dosage of antibody used for treatment may increase or decrease over the course of a particular treatment. Changes in dosage may result from the results of diagnostic assays.

The agents of the present disclosure described herein can also be administered using nanoparticle-based composition and delivery methods well known to the skilled artisan. For example, nanoparticle-based delivery for improved nucleic acid (*e.g.,* small RNAs) therapeutics are well known in the art (Expert Opinion on Biological Therapy 7:1811-1822).

In addition, any agent of the present disclosure can be administered in combination therapy with another agent useful for treating a metabolic disorder, *e.g.,* a respiration uncoupling agent, weight loss drug, insulin, and the like.

### Exemplification

This invention is further illustrated by the following examples, which should not be construed as limiting.

### Example 1: Materials and Methods for Examples 2-5

### A. Animals

All animal experiments were approved by Institutional Animal Care and Use Committee of the Beth Israel Deaconess Medical Center. In all studies, C57BL/6 mice were used. Lean mice were obtained from Charles River Laboratories and diet-induced obese mice were from Jackson Laboratory, maintained in 12 hour light/dark cycles (6am-6pm) at 24°C and fed standard irradiated rodent chow diet except for diet-induced obese mice which were fed high fat diet (60% fat diet). All mice used in injection studies were sacrificed between 4-6pm.

### B. White adipocyte differentiation and Stromal-vascular (SV) culture

Inguinal SV fractions were obtained from 30-35 days-old male mice by the following procedure. Inguinal fat tissue was dissected, washed with PBS, minced and digested for 45 min at 37°C in PBS containing 10 mM CaCl, 2.4 U/mL dispase II (Roche), and 1.5 U/mL collagenase D (Roche). Digested tissue was filtered through a 100-µm cell strainer and centrifuged at 600g for 5 min to pellet the SV cells which were then resuspended in SV culture medium (DMEM/F12 [1:1; Invitrogen] plus glutamax, pen/strep, and 10% FBS), filtered through a 40-µm cell strainer, centrifugation as above, resuspended in SV culture medium and plated. SV cells were grown to confluency for adipocyte differentiation which was induced by the adipogenic cocktail containing 1 µM dexamethasone, 5 µg/mL insulin, 0.5 µM isobutylmethylxanthine (DMI), and 1 µM rosiglitazone in SV culture medium. 2 days after induction, cells were maintained in SV culture medium containing 5 µg/mL insulin and 1 µM rosiglitazone. Starting at day 6, cells were maintained in SV culture medium only and treated with various molecules for 4-24 hours and harvested at day 8.

### C. Brown adipocyte differentiation and SV culture

Interscapular brown fat pads were dissected from newborn mice (postnatal days 2-4), minced, and digested for 45 min at 37°C in an isolation buffer containing 123 mM NaCl, 5 mM KCl, 1.3 mM CaC12, 5.0 mM glucose, 100 mM HEPES, 4% BSA, 1.5 mg/mL collagenase B (Roche). Digested tissue was filtered through a 100-µm cell strainer, centrifuged at 600g for 10 min. Pelleted SV cells were resuspended in complete SV culture medium (DMEM/F12 [1:1; Invitrogen] plus glutamax, pen/strep, and 10% FBS) and plated. SV cells were grown to confluency for adipocyte differentiation which was induced by the adipogenic cocktail containing 5 µM dexamethasone, 0.02 µM insulin, 0.5 µM isobutylmethylxanthine, 1 nM T3, 125 µM indomethacin, and 1 µM rosiglitazone in SV culture medium. 2 days after induction, cells were maintained in SV culture medium containing 0.02 µM insulin, 1 nM T3 and 1 µM rosiglitazone. Starting at day 6, cells were maintained in SV culture medium only and treated with various molecules for 4-24 hours and harvested at day 8.

### D. LLC cell culturing and conditioned media preparation

LLC cells were maintained in DMEM (Invitrogen) with 10% FBS and pen/strep. For conditioned media collection, cells were plated at 1/3 confluency. The following day, medium is changed to FreeStyle expression medium (Invitrogen) which was collected 24 hours later. For concentration of expression medium, LLC conditioned media was filtered using a 3 kilodalton (Kd) cut-off filter (Millipore). Adipocytes were maintained in SV culture media (DMEM/F12 [1:1; Invitrogen] plus glutamax, pen/strep, and 10% FBS) and conditioned media treatment experiments were performed 7 days post-differentiation. When adipocytes were treated with conditioned media for 24 hours, the treatment media were composed of 50% SV culturing medium and 50% LLC condioned media. When conditioned medium was filtered and concentrated, adipocytes were exposed to 90% SV culturing medium and 10% concentrated LLC conditioned media.

### E. Gene expression analysis (RT-qPCR)

RNA was extracted from cultured cells or frozen tissue samples using TRIzol (Invitrogen), purified with Qiagen RNAeasy minicolums and reverse transcribed using High Capacity cDNA Reverse Transcription kit (Applied Biosystems). Resulting cDNA was analyzed by RT-qPCR. Briefly, 25 ng of cDNA and 150 nmol of each primer were mixed with SYBR® GreenER™ PCR Master Mix (Invitrogen). Reactions were performed in 384-well format using an ABI PRISM® 7900HT instrument (Applied Biosystems). Relative mRNA levels were calculated using the comparative CT method normalized to cyclophilin.

### F. Western Blotting

Cells were homogenized in a lysis buffer containing 50 mM Tris (pH 7.4), 500 mM NaCl, 1% NP40, 20% glycerol, 5 mM EDTA and 1 mM PMSF, supplemented with protease and phosphatase inhibitor cocktails (Roche). The homogenates were centrifuges at 13,000 rpm for 10 min and the supernatants were used as whole cell lysates. Protein concentration was determined by Bio-Rad Bradford assay and 50 µg of proteins were used in each SDS-PAGE run. PVDF membrane was blotted with UCP1 antibody (Abcam) in TBS containing 0.05% Tween and 5% BSA. For secondary antibody incubation, TBS-T containing 5% milk was used. ECL western blotting substrates from Pierce were used for visualization of the results.

### G. Statistical analysis

Values are expressed as mean ± SEM. Significant differences between two groups were evaluated using two-tailed, unpaired t test.

In addition, molecules useful for thermogenesis-related assays are well-known in the art and a non-limiting, exemplary list is provided in Table 2 below.

**Table 2: Useful Markers For Thermogenesis-Related Assays**

| Gene Symbol | Gene Name | GenBank Gene Accession Number | GenBank Protein Accession Number | Gene ID |
|---|---|---|---|---|
| adipsin | complement factor D | *e.g*., NM_013459.2 and NM_001928.2 | *e.g.,* NP_038487.1 and NP_001919.2 | *e.g.,* 11537 and 1675 |
| fatty acid | fatty acid | *e.g.,* NM_007643.3 and NM_000072.3 and | *e.g.,* NP_031669.2 and NP_000063.2 | *e.g.,* 12491 and 948 |
| transporter cd36 | transporter/cd36 | NM_001001547.2 and NM_001001548.2 and NM_001127443.1 and NM_001127444.1 | and NP_001001547.1 and NP_001001548.1 and NP_001120915.1 and NP_001120916.1 | |
| adiponectin | adiponectin | *e.g.,* NM_009605.4 and NM_004797.2 | *e.g.*, NP_0033735.3 and NP_004788.1 | *e.g.,* 11450 and 9370 |
| UCP-1 | uncoupling protein 1 | *e.g.,* NM_009463.3 and NM_021833.4 | *e.g.,* NP_ 033489.1 and NP_ 068605.1 | *e.g.,* 22227 and 7350 |
| cidea | cell death-inducing DFFA-like effector a | *e.g.*, NM_007702.2 and NM_001279.3 and NM_ 198289.2 | *e.g.,* NP_031728.1 and NP_ 001270.1 and NP_ 938031.1 | *e.g.,* 12683 and 1149 |
| PGCla | Peroxisome porliferative activated receptor, gamma, coactivator 1 alpha | *e.g.,* NM_008904.2 and NM_013261.3 | *e.g.,* NP_032930.1 and NP_ 037393.1 | *e.g.,* 19017 and 10891 |
| Elovl3 | elongation of very long chain fatty acids (FEN1Ele2, SUR4/Elo3, yeast)-like 3 | *e.g.,* NM_007703.2 and NM_152310.1 | *e.g.,* NP_031729.1 and NP_ 689523.1 | *e.g.,* 12686 and 83401 |
| C/EBPbeta | CCAAT/enhancer binding protein beta | *e.g.,* NM_009883.3 and NM_005194.2 | *e.g.,* NP_034013.1 and NP_005185.2 | *e.g.,* 12608 and 1051 |
| Cox7al | cyotchrome c oxidase subunit VIIa polypeptide 1 | *e.g.,* NM_009944.3 and NM_001864.2 | *e.g.,* NP_034074.1. and NP_ 001855.1 | *e.g.,* 12865 and 1346 |
| Otopetrin | Otopetrin 1 | *e.g.,* NM_172709.3 and NM_177998.1 | *e.g.,* NP_766297.2 and NP_ 819056.1 | *e.g.,* 21906 and 133060 |
| Type II deiodinase | Deiodinase, iodothyronine, type II | *e.g.,* NM_010050.2 and NM_000793.4 and NM_001007023.2 and NM_013989.3 | *e.g.,* NP_034180.1 and NP_000784.2 and NP_001007024.1 and NP_054644.1 | *e.g.,* 13371 and 1734 |
| cytochrome C | cytochrome c | *e.g.,* NM_009989.2 and NM_ 018947.4 | *e.g.,* NP_ 034119.1 and NP 061820.1 | *e.g.,* 13067 and 54205 |
| cox4il | cytochrome c oxidase subunit IV isoform 1 | *e.g.,* NM_009941.2 and NM_001861.2 | *e.g.*, NP_034071.1 and NP_001852.1 | *e.g.,* 12857 and 1327 |
| coxIII | mitochondrially encoded cytochrome c oxidase III | *e.g.,* NC_005089.1 and ENST00000362079 | *e.g.,* NP_ 904334.1 and ENSP00000354982 | *e.g.,* 17705 and 4514 |
| cox5b | cytochrome c oxidase subunit Vb | *e.g.,* NM_009942.2 and NM_001862.2 | *e.g.,* NP_034072.2 and NP_ 001853.2 | *e.g.,* 12859and 1329 |
| cox8b | cytochrome c oxidase subunit 8B, mitochondrial precursor | *e.g.,* NM_007751.3 | *e.g.,* NP_031777.1 | *e.g.,* 12869 and 404544 |
| glut4 | solute carrier family 2 (facilitated glucose transporter), member 4 | *e.g.,* NM_009204.2 and NM_001042.2 | *e.g.,* NP_033230.2 and NP_001033.1 | *e.g.,* 20528 and 6517 |
| atpase b2 | ATPase, H+ transportying, lysosomal 56/58kDa, VI subunit B2 | *e.g.,* NM_057213.2 and NM_001693.3 | *e.g.,* NP_ 476561.1 and NP_001684.2 | *e.g.,* 117596 and 526 |
| coxII | mitochondrially encoded cytochrome c oxidase II | e.g., NC_005089.1 and ENST00000361739 | *e.g.,* NP_904331 and ENSP00000354876 | *e.g.,* 17709 and 4513 |
| atp5o | ATP synthase, H+ transporting, mitochondrial F1 complex, O subunit | *e.g.,* NM_138597.2 and NM_001697.2 | *e.g.*, NP_613063.1 and NP_001688.1 | *e.g.,* 28080 and 539 |
| ndufb5 | NADH dehydrogenase (ubiquinone) 1 beta subcomplex, 5, 16kDa | *e.g.*, NM_025316.2 and NM_002492.2 | *e.g.,* NP_079592.2 and NP_ 002483.1 | *e.g.,* 66046 and 4711 |
| Rarres2 | retinoic acid receptor responder (tazarotene induced) 2 | *e.g.,* NM_027852.2 and NM_002889.3 | *e.g.,* NP_082128.1 and NP_ 002880.1 | *e.g.,* 71660 and 5919 |
| Car3 | carbonic anhydrase 3 | *e.g.,* NM_007606.3 and NM_005181.3 | *e.g.,* NP_031632.2 and NP_005172.1 | *e.g.,* 12350 and 761 |
| Peg10 | paternally expressed 10 | *e.g.*, NM_001040611.1 and NM_001040152.1 and NM_001172437.1 and NM_001172438.1 and NM_015068.3 | *e.g.*, NP_001035701.1 and NP_001035242.1 and NP_001165908.1 and NP_001165909.1 and NP_055883.2 | *e.g.,* 170676 and 23089 |
| Cidec | Cidec cell death-inducing DFFA-like effector c | *e.g.,* NM_178373.3 and NM_022094.2 | *e.g.,* NP_ 848460.1 and NP_071377.2 | *e.g.,* 14311 and 63924 |
| Cd24a | CD24a antigen | *e.g.,* NM_009846.2 and NM_013230.2 | *e.g.,* NP_033976.1 and NP_037362.1 | *e.g.,* 12484 and 100133941 |
| Nrld2 | nuclear receptor subfamily 1, group D, member 2 | *e.g.,* NM_011584.4 and NM_ 001145425.1 and NM_ 005126.4 | *e.g.,* NP_035714.3 and NP_001138897.1 and NP 005117.3 | *e.g.,* 353187 and 9975 |
| Ddxl7 | DEAD (Asp-Glu-Ala-Asp) box polypeptide 17 | *e.g.,* NM_001040187.1 and NM_001098504.1 and NM_001098505.1 and NM_ 006386.4 and NM_030881.3 | *e.g.,* NP_001035277.1 and NP_001091974.1 and NP_001091975.1 and NP_006377.2 and NP_112020.1 | *e.g.,* 67040 and 10521 |
| Aplp2 | amyloid beta (A4) precursor-like protein 2 | *e.g.,* NM_001102455.1 and NM_ 001142276.1 and NM_ 001142277.1 and NM_ 001142278.1 and NM_ 001642.2 | *e.g.*, NP_001095925.1 and NP_001135748.1 and NP_001135749.1 and NP_001135750.1 and NP 001633.1 | *e.g.,* 11804 and 334 |
| Nr3cl | nuclear receptor subfamily 3, group C, member 1 | *e.g.,* NM_008173.3 and NM_000176.2 and NM_001018074.1 and NM_001018075.1 and NM_001018076.1 and NM_001018077.1 and NM_001020825.1 and NM_001024094.1 | *e.g.,* NP_032199.3 and NP_000167.1 and NP_001018084.1 and NP_001018085.1 and NP_001018086.1 and NP_001018087.1 and NP_001018661.1 and NP_001019265.1 | *e.g.,* 14815 and 2908 |
| Rybp | RING1 and YY1 binding protein | *e.g.,* NM_019743.3 and NM_012234.4 | *e.g.,* NP_062717.2 and NP_036366.3 | *e.g.,* 56353 and 23429 |
| Txnip | thioredoxin interacting protein | *e.g.*, NM_001009935.2 and NM_006472.3 | *e.g.*, NP_ 001009935.1 and NP_006463.3 | *e.g.,* 56338 and 10628 |
| Cig30 | Elongation of very long chain fatty acids-like 3 | *e.g.*, NM_ 152310.1 and NM_007703.1¹ | *e.g.*, NP_689523.1 and NP_ 031729.1¹ | *e.g.,* 83401 and 12686 |
| Ppar gamma 2 | Peroxisome proliferator -activated receptor gamma 2 | *e.g.,* NM_015869.4 and NM_011146.2¹ | *e.g.*, NP_056953 and NP_035276.1¹ | *e.g.,* 5468 and 19016 |
| Prdml6 | PR domain containing 16 protein | *e.g.,* NM_022114.3 and NM_199454.2 and NM_027504.3 | *e.g.,* NP_071397.3 and NP_955533.2 and NP_081780.3 | *e.g.,* 63976 and 70673 |
| Ap2 | Fatty acid binding protein 4 | *e.g.,* NM_001442.2 and NM_ 024406.1 | *e.g.*, NP_001433.1 and NP_077717.1 | *e.g.,* 2167 and 11770 |
| Ndufs2 | NADH dehydrogenase (ubiquinone) Fe-S protein 2, 49kDa (NADH-coenzyme Q reductase | *e.g.*, NM_001166159.1 and NM_004550.4 and NM_153064.4 | *e.g.*, NP_001159631.1 and NP_004541.1 and NP_694704.1 | *e.g.,* 4720 and 226646 |
| Grp109A | Hydroxycarboxylic acid receptor 2 | *e.g.,* NM_ 177551 and NM_030701.3 | *e.g.,* NP_808219 and NP_109626.1 | *e.g.*, 338442 and 80885 |
| AcylCoA-thioesterase 4 | Acyl-coenzyme A thioesterase 4 | *e.g.,* NM_152331 and NM_134247.3 | *e.g.,* NP_689544 and NP_599008.3 | *e.g.,* 122970 and 171282 |
| Claudin1 | Claudin1 | *e.g.*, NM_021101.4 and NM 016674.4 | *e.g.*, NP_066924.1 and NP 057883.1 | *e.g.,* 9076 and 12737 |
| PEPCK | Phosphoenolpyruvate carboxy kinase (mitochondrial) | *e.g.,* NM_001018073.1 and NM_004563.2 and NM_028994.2 | *e.g.*, NP_001018083.1 and NP_004554.2 and NP_083270.1 | *e.g.,* 5106 and 74551 |
| Fgf21 | Fibroblast growth factor 21 | *e.g.*, NM_019113 and NM_020013.4 | *e.g.,* NP_ 061986 and NP_064397.1 | *e.g.,* 26291 and 56636 |
| AcyCoA-thioesterase 3 | Acyl-coenzyme A thioesterase 4 | *e.g.*, NM_001037161.1 and NM_134246.3 | *e.g.*, NP_001032238.1 and NP_599007.1 | *e.g.*, 641371 and 171281 |
| Dio2 | Type II iodothyronine deiodinase | *e.g.,* NM_00793.5 and NM_010050.2 | *e.g.*, NP_000784.2 and NP_034180.1 | *e.g.,* 1734 and 13371 |

### Example 2: Cancer cachexia is associated with induction of thermogenic gene expression in various tissue (comparative)

Inoculation of mice with Lewis Lung Carcinoma (LLC) cells results in fat mass loss (Figure 1). Cancer cachexia resulting in such mice is associated with induction of thermogenic gene expression in various tissues, such as inguinal white adipose tissue (WAT) (Figure 2A), epididymal WAT (Figure 2B), and interscapular brown adipose tissue (BAT) (Figure 2B). Ucp1 and Dio2 were upregulated in the first two weeks only for inguinal WAT (Figure 2A) because there was not much fat left in this depot in the last two weeks of the analyses.

Since LLC cells injected into mice induce thermogenic gene expression in white adipose tissues, it was next determined whether LLC cells can induce thermogenic gene expression in cell culture. Primary white inguinal adipocytes were treated with conditioned media of LLC cells for 24 hours and LLC cell products induced thermogenic gene expression (Figure 3A). LLC conditioned media filtered to only contain macromolecules larger than 3Kd similarly showed induction of thermogenic gene expression, indicating that the thermogenic induction activity from the LLC conditioned media is a protein that is bigger than 3 Kd (Figure 3B). Moreover, LLC cell clones varied in their ability to induce thermogenic gene expression in primary white inguinal adipocytes (Figures 4A-4B).

### Example 3: PTHrP/PTH induce thermogenic gene expression and increase uncoupled respiration and maximal respiration potential of primary white and brown adipocyte (comparative)

LLC clones L2, L3, L5, L28, L18, L19, L23, and L24 were used to isolated RNA and perform microarray analyses in order to identify genes that have higher expression in more thermogenic clones and are predicted to be secreted factors (Figure 5). The secreted proteins were purchased from vendors (Bachem and R&D Systems) and their thermogenic activity was tested on primary white adipocytes (Figure 6). Amino acid residues 1-34 of PTHrP (*i.e.,* PTHrP peptide), as well as the EGF proteins, BTC, HBEGF and EREG, were determined to be the most potent proteins for inducing thermogenic gene expression, such as that of Ucp1 and Dio2 (Figure 6).

PTHrP/PTH receptor (PTHR1) is highly expressed in adipose tissues (Figure 7). The N-terminal regions of PTHrp (*e.g.,* residues 1-34) and PTH (*e.g.,* residues 1-34) are powerful inducers of thermogenic gene expression (Figures 8A-8C), uncoupled respiration (Figure 8D), and maximal respiration potential (Figure 8D) in primary white adipocytes. Similar results were observed with primary brown adipocytes (Figures 9A-9B).

In addition, PTHrP neutralization blocks thermogenic activity in primary white adipocytes (Figures 10A-10B).

### Example 4: PTHrP/PTH induce thermogenic gene expression, weight loss, fat loss, and improved glucose metabolism in vivo (comparative)

Lean mice (7-9-weeks-old) housed at room temperature and treated with a single injection of 1 mg/kg PTHrP (1-34) showed increased thermogenic gene expression in inguinal white adipose tissue (Figure 11A), epididymal white adipose tissue (Figure 11C), and brown adipose tissue (Figure 11D). In addition, such mice housed at a thermoneutral temperature of 30°C also showed increased thermogenic gene expression in inguinal white adipose tissue (Figure 11B). Moreover, the thermogenic effects were significantly increased in inguinal white adipose tissue (Figure 11E), epididymal white adipose tissue (Figure 11F), and brown adipose tissue (Figure 11G) when the single injection was increased to daily injections of 1 mg/kg PTHrP (1-34) over a course of five days.

In addition to the effects of PTHrP (1-34) on thermogenic gene expression *in vivo,* diet-induced obese mice injected with 1 mg/kg PTHrP (1-34) daily for 10 days displayed amelioration of a number of indicators of metabolic disorders. Weight loss was observed by day 3 and the mice lost up to 10% body weight in 10 days (Figure 12A). The weight loss was accompanied by significant fat loss (*e.g.,* about a 20% reduction in total fat mass shown by MRI scan) (Figure 12B) and reduced fat mass (Figure 12C). Finally, pTHrP (1-34) treatment improved glucose metabolism, as demonstrated by lowered blood glucose levels (Figure 12D).

In some embodiments, fusion proteins, such as PTHrP-Fc fusion proteins, can be used to increase the pharmacological half-life and bioavailability of the agents described herein. For example, Figure 13 shows that PTHrP-Fc fusion proteins maintain the ability to increase thermogenic gene expression and increase pharmacological half-life.

### Example 5: EGF proteins, including EREG, HB-EGF, BTC, and TGFA induce thermogenic gene expression and inhibiting the EGF receptor blocks the induction

Various EGF proteins are powerful inducers of thermogenic gene expression (Figure 14A). Such induced thermogenic gene expression can be blocked using an EGF receptor inhibitor. For example, EGF receptor inhibitor AST-1306 was used to inhibit BTC, EREG and HBEGF activity on primary white adipocytes (Figure 14B). However, it was observed that AST-1306 does not block the thermogenic inductive effect of LLC cell conditioned media activity on primary white adipocytes (Figure 14C).

### Example 6: Antibody-based neutralization of PTHrP blocks browning caused by tumors and spares body weight and muscle mass of cachexia (comparative)

*In vivo* administration of neutralizing anti-PTHrP antibodies to mice afflicted with tumors resulted in a blockade of browning and a reduction in body weight and muscle mass normally associated with cachexia as compared to control animals (Figures 15A-15F). Specifically, 5 million Lewis lung carcinoma (LLC) cells per mouse were injected subcutaneously over the flank. The control group received the vehicle (PBS) only. Mice received intraperitoneal injections of 10 mg/kg body weight of an anti-PTHrP(1-34) antibody (Bachem; catalog no: T-4512) or normal rabbit IgG (R&D Systems; catalog no: AB-105-C) antibodies at days 6, 9, 12 and 15 and were sacrificed at day 16. Plasma was collected with EDTA tubes for PTHrP(1-34) ELISA assay. mRNA levels in epididymal WAT and interscapular BAT were measured by RT-qPCR.

### Example 7: Materials and methods for Example 8

### A. Reagents

All recombinant proteins used were purchased from R&D Systems, except STC1 (BioVendor) and tumour-necrosis factor-α (TNF-α) (Sigma). Synthetic PTHrP and PTH peptides, the PTHrP(1-34) enzyme-linked immunosorbent assay (ELISA) (S-1227) and the anti-PTHrP(1-34) (T-4512) antibody were purchased from Bachem. Normal rabbit IgG (Santa Cruz Biotechnology, sc-2027; and R&DSystems, AB-105-C) was used as a control for the anti-PTHrP antibody. All other antibodies were purchased from Cell Signaling Technology, including antibodies specific for total HSL (#4107), phospho-HSL (#4126), total CREB (#9197), phospho-CREB (#9198), phospho-PKA substrate (#9624), phospho-Akt (#4060) and phospho-ERK1/2 (#9101), except anti-UCPl (Abcam, ab10983) and anti-tubulin (Santa Cruz Biotechnology, sc-9935) antibody. Noradrenaline and AST-1306 were purchased from Sigma and Tocris, respectively.

### B. Animals

All animal experiments were approved by the Institutional Animal Care and Use Committee of the Beth Israel Deaconess Medical Center. Mice (*Mus musculus*) were maintained in 12 h light:dark cycles (6 a.m.-6 p.m.) at 22°C and fed a standard irradiated rodent chow diet. *Prdm16*-floxed mice (±adiponectin-Cre) were maintained on a pure C57BL/6 background. All other animals were lean C57BL/6 mice obtained from Charles River Laboratories. Six-to-ten-week-old male mice were used in all animal experiments. Sample size, determined empirically via performing preliminary experiments, was chosen to be at least four to ensure that adequate statistical power was achieved. Mice were divided into treatment groups randomly while satisfying the criteria that the average body weight in each group would be about the same. Treatment groups were clearly marked to avoid mistakes (no blinding). LLC cells (5 X 10⁶ per mouse) were injected subcutaneously over the flank. Non-tumour-bearing control mice received vehicle (PBS) only. Mice received intraperitoneal injections of IgG and anti-PTHrP antibody and subcutaneous injections of the PTHrP peptide. Non-tumour-bearing mice received IgG or vehicle (PBS). All mice were killed in the late light cycle (3 p.m.-6 p.m.). Mice were housed individually in all tumour inoculation experiments and in groups in other experiments. Plasma was collected into EDTA-containing tubes for the PTHrP(1-34) ELISA assay. Peptide-free rat serum (Bachem) was used as the standard diluent to correct for the background reading of mouse plasma samples. Heparin-containing tubes were used to collect plasma for calcium measurements, which were performed with aVITROS analyser. Whole-body energy metabolism was evaluated using aComprehensive LaboratoryAnimalMonitoring System (CLAMS, Columbus Instruments). Carbon dioxide and oxygen data were collected every 32 min for each mouse and were normalized to total bodyweight. Data on activity, heat generation and food intake were measured at more frequent intervals. For haematoxylin and eosin staining, adipose tissues were fixed in 4% formaldehyde, embedded in paraffin and cut into 6-µm sections on slides.

### C. Primary white adipocyte culture

Inguinal stroma-vascular fractionswere obtained from 30-35-day-old male mice using the following procedure: Inguinal fat tissuewas dissected, washed with PBS, minced and digested for 45 min at 37°C in PBS containing 10 mM CaCl₂, 2.4 Uml⁻¹ dispase II (Roche) and 1.5 U ml⁻¹ collagenaseD (Roche). Digested tissue was filtered through a 100-µm cell strainer and centrifuged at 600g for 5 min to pellet the stroma-vascular cells. The pellets were then resuspended in adipocyte culture medium (DMEM/F12 (1:1, Invitrogen) plus GlutaMAX, penicillin and streptomycin, and 10% FBS), filtered through a 40-µm cell strainer, centrifuged as above, resuspended in adipocyte culture medium and plated. The stroma-vascular cells were grown to confluence for adipocyte differentiation, which was induced by an adipogenic cocktail containing 1µM dexamethasone, 5 µg ml⁻¹ insulin, 0.5 µM isobutylmethylxanthine (DMI) and 1 µM rosiglitazone in adipocyte culture medium. Two days after induction, cells were maintained in adipocyte culture medium containing 5 µg ml⁻¹ insulin and 1 µM rosiglitazone. Starting on day 6, cells were maintained in adipocyte culture medium only and treated with various molecules for 2-24 h and harvested on day 8.

### D. Primary brown adipocyte culture

Interscapular brown fat pads were dissected fromnewborn mice (postnatal days 2-4), minced and digested for 45 min at 37°C in an isolation buffer containing 123 mM NaCl, 5 mM KCl, 1.3 mM CaCl₂, 5.0 mM glucose, 100 mM HEPES, 4% BSAand 1.5 mg ml⁻¹ collagenase B (Roche). Digested tissuewas filtered through a 100-µm cell strainer and centrifuged at 600*g* for 10 min. Pelleted stroma-vascular cells were resuspended in adipocyte culture medium (described above) and plated. Stroma-vascular cells were grown to confluence for adipocyte differentiation, which was induced by an adipogenic cocktail containing 5 µM dexamethasone, 0.02 µM insulin, 0.5 µM isobutylmethylxanthine, 1 nM T3, 125 µM indomethacin and 1 µM rosiglitazone in adipocyte culture medium. Two days after induction, cells weremaintained in adipocyte culture medium containing 0.02 µM insulin, 1 nM T3 and 1 µM rosiglitazone. Starting on day 4, cells were maintained in adipocyte culture medium only, treated with various molecules for 2-24 h and harvested on day 8.

### E. LLC cell culture and conditioned medium preparation

LLC cells were maintained in DMEM (Invitrogen) with 10% FBS, and penicillin and streptomycin. For conditioned medium collection, cells were plated at a 1:3 confluence. The following day, the medium was changed to the FreeStyle expression medium (Invitrogen) and was then collected 24 h later. For fractionation, the LLC-cell-conditioned medium was filtered using a 3-kDa cut-off filter (Millipore). Adipocytes were treated with conditioned medium for 24 h starting 7 days after the differentiation protocol. When adipocytes were treated with conditioned medium, the treatment medium was composed of 50% fresh adipocyte culture medium and 50% LLC-cell-conditioned medium. When conditioned medium was filtered and concentrated, adipocytes were exposed to 90% adipocyte culture medium and 10% concentrated LLC-cell conditioned medium. For the analysis of global gene expression, LLC clones were cultured in FreeStyle expression medium for 24 h. RNAwas extracted using TRIzol (Invitrogen) and purified with QIAGEN RNeasy minicolumns. GeneChip Mouse Genome 430 2.0 arrays (Affymetrix) were used to generate expression profile data, which were analysed using dChip software. The gene expression data are available through the Gene Expression omnibus, which is available on the world wide web at the ncbi.nlm.nih.gov/geo/ website under the accession number, GSE57797.

### F. Gene expression analysis (qRT-PCR)

RNA was extracted from cultured cells or frozen tissue samples using TRIzol, purified with QIAGEN RNeasy minicolumns and reverse transcribed using a High-Capacity cDNA Reverse Transcription kit (Applied Biosystems). The resultant cDNA was analysed by qRT-PCR. Briefly, 25 ng cDNAand 150 nmol of each primer weremixed with SYBR GreenER qPCR SuperMix (Invitrogen). Reactions were performed in the 384-well format using an ABI PRISM 7900HT instrument (Applied Biosystems). Relative mRNA levels were calculated using the comparative CT method and normalized to cyclophilin mRNA. The following primers were used: cyclophilin F: 5'-GGAGATGGCACAGGAGGAA-3', R: 5'-GCCCGTAGTGCTTCAGCTT-3'; Ucp1 F: 5'-AAGCTGTGCGATGTCCATGT-3', R: 5'-AAGCCACAAACCCTTTGAAAA-3'; Dio2 F: 5'-GTCCGCAAATGACCCCTTT-3', R: 5'-CCCACCCACTCTCTGACTTTC-3'; Pgcla F: 5'-AGACAAATGTGCTTCGAAAAAGAA-3', R: 5'-GAAGAGATAAAGTTGTTGGTTTGGC-3'; Ap2: F:5'-AGTGAAAACTTCGATGATTACATGAA-3', R:5'-GCCT GCCACTTTCCTTGTG-3'; Pparg F: 5'-CAAGAATACCAAAGTGCGATCAA-3', R: 5'-GAGCTGGGTCTTTTCAGAATAATAAG-3'; Pgclb F: 5'-GAGGGCTCCGG CACTTC-3', R: 5'-CGTACTTGCTTTTCCCAGATGA-3'; Prdm16 F: 5'-GCACGG TGAAGCCATTCATATG-3', R: 5'-TCGGCGTGCATCCGCTTGTG-3'; Cidea F: 5'-GGTTCAAGGCCGTGTTAAGG-3', R: 5'-CGTCATCTGTGCAGCATAGG-3'; Glut1 F: 5'-CGCCCCCCAGAAGGTTAT-3', R: 5'-CGATGCGGTGGTTCCAT-3'; Glut4 F: 5'-TTGGCTCCCTTCAGTTTGG-3', R: 5'-CTACCCAGCCACGTTGCAT-3';VdrF: 5'-GGCTTCCACTTCAACGCTATG-3', R: 5'-ATGCTCCGCCTGAAGAAAC-3'; Acox1 F: 5'-GCCCAACTGTGACTTCCATTAA-3', R: 5'-GTAGCACTCCCCTCGA GTGAT-3'; Acsl1 F: 5'-GATCTGGTGGAACGAGGCAA-3',R: 5'-CTTCGGGTTCT GGAGGCTTG-3'; Atgl F: 5'-CAGCACATTTATCCCGGTGTAC-3', R: 5'-AAATGCC GCCATCCACATAG-3'; Hsl F: 5'-GCTGGAGGAGTGTTTTTTTGC-3', R: 5'-AGTT GAACCAAGCAGGTCACA-3'; Pth1r F: 5'-TCTGCAATGGTGAGGTGCAG-3', R: 5'-GCTACTCCCACTTCGTGCTT-3'; Igf1 F: 5'-TACTTCAACAAGCCCACAGGC-3', R: 5'-ATAGAGCGGGCTGCTTTTGT-3'; Mstn F: 5'-AGAAGATGGGCTGAATCCCTT T-3', R: 5'-ATCGCAGTCAAGCCCAAAGT-3'; Fbxo32 F: 5'-TCAGAGAGGCAGATT CGCAA-3', R: 5'-GGGTGACCCCATACTGCTCT-3'; Trim63 F: 5'-TCCTGATGGAA ACGCTATGGAG-3', R: 5'-ATTCGCAGCCTGGAAGATGT-3'.

### G. Western blotting

Cells werehomogenized in a lysis buffer containing 50 mMTris (pH 7.4), 500 mM NaCl, 1% NP40, 20% glycerol, 5 mM EDTA and 1 mM phenylmethylsulphonyl fluoride (PMSF) supplemented with protease and phosphatase inhibitor cocktails (Roche). The homogenates were centrifuged at 13,000 r.p.m. for 10min, and the supernatants were used as whole-cell lysates. Protein concentration was determined by Bio-Rad protein assay, and 50 mg protein lysatewas separated in each SDS-PAGE lane. The separated proteins were transferred to a polyvinylidene difluoride (PVDF)membrane,which was blotted with antibodies in TBS containing 0.05% Tween (TBS-T) and 5% BSA. For secondary antibody incubation, TBS-T containing 5% milk was used. Enhanced chemiluminescence (ECL) western blotting substrates (Pierce) were used for visualization of the results.

### H. Respiration assays

The cellular oxygen consumption rate (OCR) of primary adipocytes was determined using an XF24 Extracellular Flux Analyzer (Seahorse Bioscience). Primary adipocytes were cultured and differentiated in XF24 microplates and treated with noradrenaline, PTHrP or PTH at 7 days post differentiation. Twenty-four hours later, adipocyte culture medium was changed to DMEM lacking NaHCO₃ but containing 10 mM glucose, 1 mM pyruvate and the treatment chemicals. The uncoupled and maximal OCRs were determined using 1 µM oligomycin and 1 µM FCCP (carbonyl cyanide p-trifluoromethoxyphenylhydrazone), respectively. Complex-I-dependent respiration was inhibited with 3 µM rotenone. *Ex vivo* tissue respiration was measured using a Clark electrode (Strathkelvin Instruments) as described in Cohen et al. (2014) Cell 156:304-316. Freshly isolated tissueswere briefly minced and transferred to the electrode chamber containing a respiration buffer consisting of 1 X DPBS (Dulbecco's phosphate-buffered saline), 2% BSA, 25 mM glucose and 1 mM pyruvate. Oxygen consumption values were normalized to tissue weight.

### I. Grip strength

Forelimb grip strength was assessed on the same day as killing (White et al. (2013) Mol. Cell. Endocrinol. 365:174-18623). Each mouse was allowed to grab a bar attached to a force transducer as the mouse was pulled by the tail horizontally away from the bar (DFX II Series force gauge; Chatillon). The results from five repetitions with a 30-s pause between each were averaged to determine the grip strength of each mouse.

### J. In situ muscle force measurements

*In situ* force was measured as described in Ruas et al. (2012) Cell 151:1319-1331. Briefly, mice were anaesthetized with a subcutaneous injection of a cocktail of ketamine, xylazine and acepromazine (1.4 ml per kg body weight). The distal tendon of the gastrocnemius muscle was cut just before the insertion at the ankle. The tendon was tied with a 4.0 nylon suture and attached to an isometric transducer. The sciatic nerve was exposed with an incision in the hamstring region. The sciatic nerve was then cut as close to the hip bone as possible and dissected from the surrounding fascia. The exposed sciatic nerve was then laid over two electrodes with a small piece of parafilm placed under the suspended nerve to prevent contact with other tissues. The nerve was kept moist by periodic treatment with mineral oil. The nerve was stimulated using a supramaximal square-wave pulse of 0.1 ms duration. Measurements were made at the length at which the maximal tension was obtained during the twitch (Lₒ). Data were recorded for maximal (Pₒ; stimulation frequency of 75-150 Hz) isometric force.

### K. Primary myoblast culture

Primarymyoblasts were isolatedfrommale mice <14 days old and cultured in Ham's F-10 nutrient mixture (Irvine Scientific) containing 20% FBS (Gemini Bio-Products) supplemented with 2.5 ng ml⁻¹ basic fibroblast growth factor (bFGF) (Promega), penicillin G (200 U ml⁻¹) and streptomycin (200 mg ml⁻¹). Myoblasts were then transferred to differentiation DMEM with 5% horse serum until mature myotube formation (∼48-72 h) and then treated with PTHrP or TNFα. The diameters of individual myotubes were measured using ImageJ software.

### L. Human study

This was a further analysis of a study designed to investigate the metabolic, nutritional and functional profile of a cohort of patients recently diagnosed with advanced non-small-cell lung cancer (stages IIIB or IV) or colorectal cancer (stage IV) (*n* = 547) (Lieffers et al. (2009) Am. J. Clin. Nutr. 89:1173-1179; Mourtzakis et al. (2008) Appl. Physiol. Nutr. Metab. 33:997-1006; Prado et al. (2012) Can. J. Diet. Pract. Res. 73:e298-e303). The inclusion criteria and measures have been described in Lieffers et al. (2009) Am. J. Clin. Nutr. 89:1173-1179; Mourtzakis et al. (2008) Appl. Physiol. Nutr. Metab. 33:997-1006; and Prado et al. (2012) Can. J. Diet. Pract. Res. 73:e298-e303. The patients were recruited from the Cross Cancer Institute, a cancer treatment centre serving Edmonton and Northern Alberta, Canada. The study was approved by the Alberta Cancer Board Research Ethics Board, and all participants provided written informed consent. Patients with advanced lung or colorectal cancer were included in the study because of their high susceptibility to weight loss and cachexia associated with cancer. All participants were under the direct care of a medical oncologist and received the standard chemotherapy for their disease and stage. None of the patients was receiving medications with the intent of managing cachexia (for example, corticosteroids, progestational agents or cannabinoids).

Participants were asked to fast for 12 h and to refrain from strenuous exercise and alcohol for 24 h before assessments, which included body composition measurement, REE measurement and a blood sample assessment. The PTHrP(1-34) ELISA assay was used to determine the serum PTHrP level. Serum samples collected from normal subjects were used to set the baseline for measuring the blood PTHrP level. Serum PTHrP measurements were performed in a blinded fashion. The REE measurement was made by indirect calorimetry (VMax29N, SensorMedics) as described in Lieffers et al. (2009) Am. J. Clin. Nutr. 89:1173-1179). Participants rested for 30 min, after which a canopy was placed over their head and shoulders for 30 min to analyse oxygen consumption and carbon dioxide production. Breath samples were measured until a steady state was reached for 15 min. The Weir equation was used to calculate the REE. On the same morning as the REE assessment, all participants also underwent a dual-energy X-ray absorptiometry (DXA) scan (Lunar Prodigy High Speed Digital Fan Beam X-Ray-Based Densitometer with enCORE 9.20 software, GE Healthcare) to measure whole-body fat mass and fat-free mass (Mourtzakis et al. (2008) Appl. Physiol. Nutr. Metab. 33:997-1006). Blood samples drawn for C-reactive protein assessment were taken and analysed in the hospital clinical laboratory. Three-day diet records (detailing intake for three consecutive days, including one weekend day) were used to assess total energy intake and meal patterns. Records were reviewed for accuracy and completeness. Three-day dietary records have been shown to provide accurate mean estimates of group dietary intake in patients with advanced cancer (Prado et al. (2012) Can. J. Diet. Pract. Res. 73:e298-e303).

### M. Statistical analysis

For every experiment, sample size was determined empirically (preliminary experiments were performed) to ensure that the desired statistical power could be achieved. The values are expressed as mean ± s.e.m. The error bars (s.e.m.) shown for all results were derived from biological replicates, not technical replicates. Significant differences between two groups were evaluated using a two-tailed, unpaired *t*-test, which was found to be appropriate for the statistics, as the sample groups displayed a normal distribution and comparable variance.

### Example 8: Tumour-derived PTH-related proteins trigger adipose tissue browning and cancer cachexia (comparative)

Cachexia is a wasting disorder that accompanies many chronic diseases such as cancer and congestive heart failure. The hallmarks of cachexia are weight loss with atrophy of fat and skeletal muscle (Fearon et al. (2012) Cell Metab. 16:153-166). Importantly, cachexia is more than just anorexia (Fearon et al. (2012) Cell Metab. 16:153-166; Ovesen et al. (1993) J. Clin. Oncol. 11:2043-2049; Tisdale (2009) Physiol. Rev. 89:381-410). Cachectic patients may ingest less food, but they are also in a state of negative energy balance that cannot be corrected by nutritional supplementation (Ovesen et al. (1993) J. Clin. Oncol. 11:2043-2049; Tisdale (2009) Physiol. Rev. 89:381-410). As a negative risk factor for cancer survival, cachexia causes frailty in patients and often prevents them from undergoing further therapies. While cachexia can lessen with the shrinkage of tumours, there are currently few effective therapies for cancer cachexia (Penna (2010) Exp. Opin. Biol. Ther. 10:1241-1250; Fearon et al. (2013) Nat. Rev. Clin. Oncol. 10:90-99).

The molecular basis of cachexia is poorly understood. Elevated cytokine levels have been observed in cachectic patients, but anti-cytokine therapies are ineffective (Penna (2010) Exp. Opin. Biol. Ther. 10:1241-1250; Fearon et al. (2013) Nat. Rev. Clin. Oncol. 10:90-99). The activation of brown fat has been described in rodent models of cachexia and in certain cachectic patients (Bianchi et al. (1989) Horm. Metab. Res. 21:640-641; Bing et al. (2000) Cancer Res. 60:2405-2410; Brooks et al. (1981) Biosci. Rep. 1:509-517; Roe et al. (1996) Metabolism 45:645-651; Shellock et al. (1986) J. Canc. Res. Clin. Oncol. 111:82-85; Tsoli et al. (2012) Cancer Res. 72:4372-4382). Brown fat dissipates chemical energy in the form of heat and, hence, could be involved in the negative energy balance. How tumours induce thermogenesis in brown fat cells and how this might relate to the wasting of fat and skeletal muscle is unknown. In addition, white fat depots contain pockets of uncoupling protein 1 (UCP1)-expressing multilocular cells, called beige (or brite) cells, that can be stimulated on exposure to cold or other stimuli via a process termed browning. Recent studies have characterized beige cells at a molecular level (Wu et al. (2012) Cell 150:366-376) and have demonstrated a requirement for the transcriptional coregulator PRDM16 in the browning process (Cohen et al. (2014) Cell 156:304-316). To understand the molecular basis of how tumours stimulate brown or beige cells, a murine model of lung cancer that is accompanied by cachexia was used: Lewis lung carcinoma (LLC).

LLC cells readily form tumours and lead to cachexia in syngeneic C57BL/6 mice. LLC-tumour-bearing mice demonstrated hypermetabolism: their rate of oxygen consumption was significantly higher than that of non-tumour-bearing mice (Figure 16A). However, this increase was not due to increased physical activity or reduced food intake (Figures 17A-17B). The lower carbon dioxide production relative to oxygen consumption (that is, the lowered respiratory exchange ratio) of LLC-tumour-bearing mice indicated that fat was the preferred source of fuel (Figures 17C-17D). Additionally, more heat production was observed in the cachectic mice (Figure 17E). All tumour-bearing mice lost weight over 3 weeks (Figure 16B), including wasting of adipose tissues and skeletal muscle (Figure 16C). Inguinal white adipose tissue (iWAT), a form of subcutaneous fat, wasted faster than epididymal fat (eWAT), a visceral white fat depot. In response to tumour growth, both white fat depots (eWAT and iWAT) and interscapular brown adipose tissue (iBAT) exhibited higher expression of genes involved in thermogenesis: Ucp1, Dio2 and Pgcla (Figures 16D-16F). An increase in the expression of certain other genes involved in energy metabolism, including glucose transporters and b-oxidation and lipolytic enzymes (Das et al. (2011) Science 333:233-238), was also observed (Figures 16D-16F0). LLC tumours also induced the skeletal muscle expression of atrophy-associated genes (Figure 16G), including the genes encoding myostatin (Mstn), atrogin 1 (also known as Fbxo32) and MuRF1 (also known as Trim63).

To examine the contribution of adipose tissue browning to cachexia, fat-specific Prdm16-deficient mice, which have a dramatically reduced thermogenic potential and are resistant to browning, were studied (Cohen et al. (2014) Cell 156:304-316). LLC-tumour-induced adipose tissue wasting was significantly inhibited in Prdm16-knockout mice, without a change in tumour size or the loss of muscle mass (Figures 18A-18B). The cachexia-associated induction of Ucp1 and Dio2 expression was impaired in the knockout mice, while the increase in expression of the other genes involved in energy metabolism was unaffected (Figures 18C-18F). These results indicate a crucial role for the beige fat thermogenesis pathway in adipose tissue wasting.

It was next examined whether LLC-tumour-derived factors may induce thermogenic gene expression in fat tissues. The treatment of primary white adipose cells with LLC-cell-conditioned medium stimulated the expression of Ucp1 and Dio2 (Figure 19A). The LLC-cell-conditioned was then filtered through a 3-kDa cut-off membrane and the response of primary adipocytes to each fraction was tested (Figure 19B). All of the thermogenic activity was retained in the filter-concentrated fraction, leaving none in the flow-through. This finding indicates that the thermogenesis-inducing activity most probably originates from a macromolecule.

To identify thermogenic factors, a combination of cell cloning and gene expression profiling was used. Single-cell clones were first established from the heterogeneous LLC cells. Thirty subclones were generated and were used to produce conditioned media, the activity of which was then tested on primary adipocytes. A range of Ucp1-inducing activity by conditioned media from these subclones was observed (Figure 19C), and eight clones were chosen that demonstrated either more or less thermogenic activity than the parent cells (Figure 20A). The global gene expression profiles of the 8 subclones were analyzed using microarrays and a list of secreted proteins for which the corresponding messenger RNA levels were higher in the more thermogenic subclones were generated (Fig 19D). The effects of these secreted proteins on primary adipocytes were tested and it was found that several members of the epidermal growth factor (EGF) family (Figure 20B), including betacellulin (BTC), heparin-binding EGF like growth factor (HBEGF) and epiregulin (EREG), as well as PTHrP (encoded by Pthlh), induced Ucp1 expression (Figure 19E).

To determine the contribution of BTC, EREG and HBEGF to the Ucp1-inducing activity found in LLC-cell-conditioned medium, an inhibitor of EGF receptor (EGFR) signaling was used. Since the three EGF family proteins signal through the receptors EGFR and ERBB4, the effects of AST-1306, a specific protein kinase inhibitor that targets both receptors, on primary adipocytes were tested. AST-1306 completely blocked Ucp1 mRNA induction when fat cells were treated in combination with BTC, EREG or HBEGF (Figure 20C). By contrast, the browning activity present in LLC-cell-conditioned medium was largely unaffected by AST-1306 treatment, indicating that these EGF family proteins do not have a major quantitative role in this process (Figure 21A).

PTHrP is processed into at least three peptide products. Primary white and brown adipocytes were treated with these peptides and PTH(1-34) (a PTH-derived peptide containing amino acid residues 1 to 34), which has sequence and structural similarity to PTHrP(1-34). Both PTH(1-34) and PTHrP(1-34), which signal through the same receptor (the PTH and PTHrP receptor (PTHR)), stimulated thermogenic gene expression (Figures 21B and 20D). For simplicity, hereafter, the 1-34 peptides is referred to as PTHrP and PTH. Time-course and dose-response analysis of PTHrP treatment revealed that this peptide potently induces Ucp1 and Dio2 mRNA (by up to 200- and 20-fold, respectively), comparable to noradrenaline, the classic thermogenic catecholamine produced by the sympathetic nervous system (Figures 21C and 22)). It was next investigated how PTHrP affects UCP1 protein levels and uncoupled respiration. Primary white and brown adipocytes were treated with noradrenaline, PTHrP or PTH. Remarkably, all three factors comparably increased UCP1 protein levels and significantly raised oxygen consumption, including basal, uncoupled and maximal respiration (Figures 23A-23C. These results show that the PTH and PTHrP pathway can be a robust regulator of cellular respiration.

PTHR is a G-protein-coupled receptor that activates the cyclic-AMP dependent protein kinase (PKA) pathway (Vilardaga et al. (2011) Cell. Mol. Life Sci. 68:1-13). Noradrenaline also signals through β-adrenergic receptors that are G-protein-coupled receptors and induce PKA signalling, thereby promoting thermogenic gene expression. The treatment of primary white and brown adipocytes with noradrenaline, PTHrP or PTH stimulated the phosphorylation of the PKA substrates CREB and HSL. A selective PKA inhibitor, H89, blocked PKA signalling, which also completely inhibited transcriptional regulation by all three factors (noradrenaline, PTHrP and PTH) (Figures 23D-23G). These results indicate that both PTHrP and PTH require the PKA pathway to mediate their effects on Ucp1 and Dio2 transcription and therefore have a signalling mechanism in common with the β-adrenergic pathway.

Next, a neutralizing antibody specific for PTHrP was used to investigate whether PTHrP is involved in LLC-cell-induced thermogenic gene expression. This antibody was capable of completely inhibiting PTHrP activity when added to adipocytes (Figure 21D). Importantly, when adipocytes were treated with LLC-cell-conditioned medium in combination with the anti-PTHrP antibody, Ucp1 and Dio2 induction was almost completely blocked (Figure 21E). These data indicate that PTHrP is a major component of the LLC-cell-secreted products that cause browning of cultured adipocytes.

PTHR (which is encoded by Pthlr) is known to be highly expressed in the kidneys and bone (Vilardaga et al. (2011) Cell. Mol. Life Sci. 68:1-13). Notably, rather high Pthlr mRNA expression was also observed in adipose and muscle tissues (Figure 24A). PTHrP-dependent transcriptional regulation *in vivo* was next investigated. PTHrP potently promoted the expression of thermogenic genes and certain other genes of energy metabolism (Figures 24B-24E), a pattern that is notably similar to the browning of adipose tissues by LLC tumours.

The role of PTHrP in LLC-tumour-induced fat browning and wasting was then investigated. Up to 500 pg ml⁻¹ PTHrP was detected in the plasma of tumour-bearing mice (Figure 25A). Mice were next injected with the neutralizing antibody specific for PTHrP or control IgG. Significantly, anti-PTHrP-treated mice did not experience significant weight loss, while the IgG-treated group displayed evident cachexia (Figure 25B). The anti-PTHrP treatment blocked both adipose tissue and skeletal muscle wasting, without changing the average tumour mass (Figures 25C-25D). Histological examination of the adipose tissues showed that anti-PTHrP treatment prevented the shrinkage of fat droplets (Figure 25E). This neutralization of PTHrP also blocked thermogenic gene expression in eWAT, iBAT (Figures 25F-25G) and iWAT (Figure 26), indicating that thermogenesis has a causal role in fat wasting. Treatment with anti-PTHrP antibody also lowered the oxygen consumption of the cachectic mice, increased their physical activity and reduced their heat production (Figures 25H and 27). A Clark electrode was used to test *ex vivo* oxygen consumption of fat and skeletal muscle tissues. Notwithstanding the limited power of this technique to measure and represent actual tissue respiration, modest increases in the fat tissue respiration of the cachectic mice were demonstrated. These increases were suppressed by the anti-PTHrP treatment (Figure 28). While additional, off-target, effects of the anti-PTHrP antibody cannot be ruled out, these data reveal a major role for PTHrP in LLC-tumour-induced adipose tissue thermogenesis and hypermetabolism.

Neutralization of PTHrP also reduced the LLC-tumour-induced muscle wasting (Figure 25D), the atrophy of muscle fibres (Figure 29A) and the atrophy-associated gene expression (Figure 30A). This effect was further investigated by treating mice with anti-PTHrP antibody until the time point at which severe cachexia was observed in the controls. The anti-PTHrP treatment preserved muscle mass to a significant extent (Figures 30B-30C). This preservation was also reflected in muscle function, as shown by improved grip strength and *in situ* muscle contraction (Figures 30D-30E). While it was possible that PTHrP-associated hypercalcaemia may have confounded the effects here, hypercalcaemia in the LLC model was not observed, and neutralization of PTHrP did not affect the circulating calcium levels (Figures 29B-29C).

PTHrP injection into healthy mice failed to affect the expression of muscle-atrophy-associated genes (Figures 29D-29E). Furthermore, PTHrP treatment of primary myotubes did not change the expression of the muscle-atrophy-associated genes or myotube diameter (Figures 29F-29G), indicating that PTHrP does not directly induce skeletal muscle atrophy. This result was further investigated by injecting PTHrP into tumour-bearing mice. Remarkably, PTHrP administration exacerbated cachexia-associated weight loss without changing tumour mass (Figures 29H-29J). PTHrP treatment led to more severe skeletal muscle wasting and poorer muscle function and significantly promoted the expression of muscle-atrophy-associated genes in the cachectic mice (Figures 30F-30H). These findings indicate that PTHrP is required for the loss of muscle mass and function in the LLC model. However, PTHrP probably does not act alone and is likely to collaborate with other tumour-derived factors.

Lastly, PTHrP expression was investigated in a cohort of patients diagnosed with metastatic non-small-cell lung cancer or colorectal cancer, whose body composition and metabolic rate were studied in the context of a clinical investigation of the aetiology of cancer cachexia (Lieffers et al. (2009) Am. J. Clin. Nutr. 89:1173-1179; Mourtzakis et al. (2008) Appl. Physiol. Nutr. Metab. 33:997-1006; Prado et al. (2012) Can. J. Diet. Pract. Res. 73:e298-e303). Seventeen out of 47 patients displayed detectable levels of serum PTHrP (mean ± s.d., 205 ± 155 pg ml⁻¹) with no medical history of hypercalcaemia. These patients had a significantly lower lean body mass (LBM) and a significantly higher resting energy expenditure (REE) per kg LBM than 30 patients lacking detectable levels of PTHrP (Figures 30I-30J). The reduced amount of lean tissue in the PTHrP-positive patients was not associated with a difference in dietary intake (PTHrP-positive, 2,364 ± 750 kcal day⁻¹ (mean ± s.d.); PTHrP-negative, 2,285 ± 487 kcal day⁻¹; P = 0.66) or a difference in systemic inflammation as determined by the serum C-reactive protein levels (PTHrP-positive, 5.7 ± 4.7mg l⁻¹ (mean ± s.d.); PTHrP negative, 12.6 ± 27mg l⁻¹; P = 0.32). The data are shown in Table 3 below.

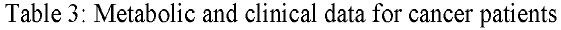

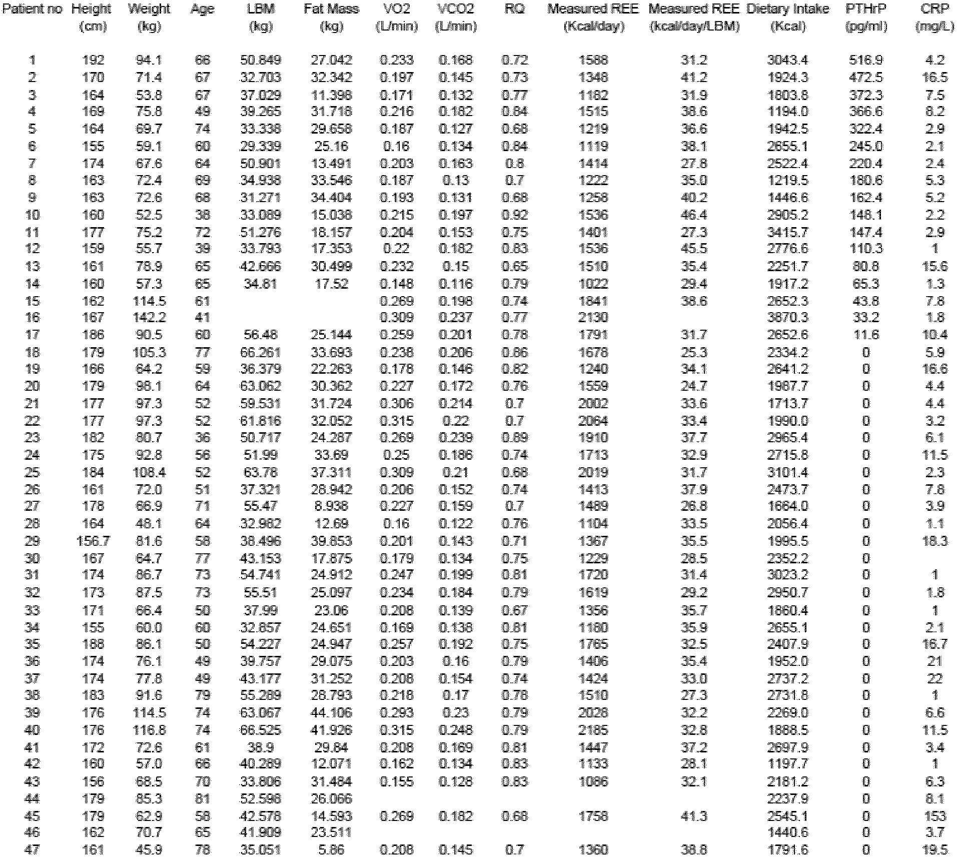

These results indicate an association between higher PTHrP concentrations and a greater degree of lean tissue wasting and elevated energy expenditure within this patient cohort .of patients.

PTHrP is secreted by many tumours and is often involved in the hypercalcaemia that accompanies certain cancers (Mundy and Edwards (2008) J. Am. Soc. Nephrol. 19:672-675). PTHrP has previously been linked to the cachexia associated with hypercalcaemia of malignancy (Iguchi et al. (2006) J. Bone Miner. Metab. 24:16-19; Iguchi et al. (2001) Int. J. Cancer 94:24-27; Sato et al. (1993) J. Bone Miner. Res. 8:849-860). However, its direct roles in the muscle wasting process and the browning of adipose tissues have not been reported. Taken together, the data described herein show that PTHrP is the major LLC-tumour-derived factor stimulating adipose tissue thermogenic gene expression and hypermetabolism. Neutralization of PTHrP blocks both the browning and the wasting of fat depots and much of the muscle wasting and weakness in this model of cachexia. These data indicate a model whereby PTHrP functions alone to drive the thermogenic/browning program in adipose tissues but is likely to collaborate with other tumour-derived molecules to bring about the profound muscle wasting and weakness observed in the LLC model (Figure 30K). The identity of the collaborating factors is unknown. The browning driven by PTHrP seems not to be obligatorily linked to the muscle wasting process. The evaluation of 47 patients with cancer identified a PTHrP-positive subset, which showed features of reduced LBM and increased REE. Further prospective studies on cachexia-prone patients study these parameters with serial tissue biopsies to determine whether there is a specific association between PTHrP, adipose tissue browning and metabolic rate in clinical cancer cachexia. It is believed that PTHrP is involved in cachexia in certain patients and that it should be possible to identify those with elevated PTHrP levels. A humanized antibody specific for PTHrP is believed to have anti-cachectic effects like those shown herein. It is also believed that elevated PTHrP or PTH levels has roles in the cachexia that is associated with certain other diseases, such as congestive heart failure and chronic kidney diseases.

## Claims

1. An *in vitro* method of modulating a metabolic response comprising contacting an adipose cell with transforming growth factor alpha (TGFA) to increase thermogenesis by the adipose cell.

2. The method of claim 1, wherein the TGFA is a polypeptide comprising an amino acid sequence having at least 80% identity to SEQ ID NOs: 2, 4, or 6.

3. The method of claim 1 or 2, wherein the TGFA is a nucleic acid molecule encoding a TGFA polypeptide and having a nucleic acid sequence at least 80% identity to SEQ ID NOs: 1, 3, or 5.

4. The method of any one of claims 1-3, wherein the TGFA polypeptide lacks a signal peptide.

5. The method of any one of claims 1-4, wherein the TGFA polypeptide further comprises a heterologous polypeptide.

6. The method of any one of claims 1-5,wherein the heterologous polypeptide is selected from the group consisting of a signal peptide, a peptide tag, a dimerization domain, an oligomerization domain, an agent that promotes plasma solubility, an agent that increases *in vivo* protein half-life, an antibody or fragment thereof, and an Fc domain.

7. The method of any one of claims 1-6,
further comprising the use of an additional agent that increases the metabolic response of the adipose cell.

## Patentansprüche

1. *In vitro*-Verfahren zur Modulierung einer metabolischen Reaktion, umfassend das Inkontaktbringen einer Fettzelle mit transformierendem Wachstumsfaktor alpha (TGFA), um die Thermogenese durch die Fettzelle zu erhöhen.

2. Verfahren nach Anspruch 1, wobei der TGFA ein Polypeptid ist, das eine Aminosäuresequenz mit mindestens 80 % Übereinstimmung mit SEQ ID NO: 2, 4 oder 6 aufweist.

3. Verfahren nach Anspruch 1 oder 2, wobei der TGFA ein Nukleinsäuremolekül ist, das für ein TGFA-Polypeptid kodiert und eine Nukleinsäuresequenz mit mindestens 80 % Übereinstimmung mit SEQ ID NO: 1, 3 oder 5 aufweist.

4. Verfahren nach einem der Ansprüche 1 bis 3, wobei dem TGFA-Polypeptid ein Signalpeptid fehlt.

5. Verfahren nach einem der Ansprüche 1 bis 4, wobei das TGFA-Polypeptid ferner ein heterologes Polypeptid umfasst.

6. Verfahren nach einem der Ansprüche 1 bis 5, wobei das heterologe Polypeptid ausgewählt ist aus der Gruppe bestehend aus einem Signalpeptid, einem Peptid-Tag, einer Dimerisierungsdomäne, einer Oligomerisierungsdomäne, einem Mittel, das die Plasmalöslichkeit fördert, einem Mittel, das die *in vivo*-Halbwertszeit von Proteinen erhöht, einem Antikörper oder einem Fragment davon und einer Fc-Domäne.

7. Verfahren nach einem der Ansprüche 1 bis 6,
ferner umfassend die Verwendung eines zusätzlichen Mittels, das die metabolische Reaktion der Fettzelle verstärkt.

## Revendications

1. Méthode *in vitro* de modulation d'une réponse métabolique comprenant la mise en contact d'une cellule adipeuse avec le facteur de croissance transformant alpha (TGFA) pour augmenter la thermogénèse par la cellule adipeuse.

2. Méthode selon la revendication 1, dans laquelle le TGFA est un polypeptide comprenant une séquence d'acides aminés présentant au moins 80% d'identité avec les SEQ ID NO : 2, 4, ou 6.

3. Méthode selon la revendication 1 ou 2, dans laquelle le TGFA est une molécule d'acide nucléique codant pour un polypeptide TGFA et ayant une séquence d'acide nucléique présentant au moins 80% d'identité avec les SEQ ID NO : 1, 3, ou 5.

4. Méthode selon l'une quelconque des revendications 1 à 3, dans laquelle le polypeptide TGFA manque un peptide signal.

5. Méthode selon l'une quelconque des revendications 1 à 4, dans laquelle le polypeptide TGFA comprend en outre un polypeptide hétérologue.

6. Méthode selon l'une quelconque des revendications 1 à 5, dans laquelle le polypeptide hétérologue est choisi dans le groupe constitué par un peptide signal, un marqueur peptidique, un domaine de dimérisation, un domaine d'oligomérisation, un agent qui favorise la solubilité plasmatique, un agent qui augmente la demi-vie des protéines *in vivo,* un anticorps ou un fragment de celui-ci, et un domaine Fc.

7. Méthode selon l'une quelconque des revendications 1 à 6,
comprenant en outre l'utilisation d'un agent supplémentaire qui augmente la réponse métabolique de la cellule adipeuse.
